(19) Europäisches Patentamt
European Patent Office
Office européen des brevets

(11) Veröffentlichungsnummer: **0 021 176**
**B1**

(12) # EUROPÄISCHE PATENTSCHRIFT

(45) Veröffentlichungstag der Patentschrift: **18.01.84**

(21) Anmeldenummer: **80103135.2**

(22) Anmeldetag: **06.06.80**

(51) Int. Cl.³: **C 07 D 501/20**
//C07D239/46, C07D407/12, C07D409/12, A61K31/545

(54) **Cephalosporine, Verfahren zu ihrer Herstellung und diese Verbindungen enthaltende Arzneimittel.**

(30) Priorität: **21.06.79 DE 2924948**

(43) Veröffentlichungstag der Anmeldung:
**07.01.81 Patentblatt 81/1**

(45) Bekanntmachung des Hinweises auf die Patenterteilung:
**18.01.84 Patentblatt 84/3**

(84) Benannte Vertragsstaaten:
**AT BE CH DE FR GB IT LI LU NL SE**

(56) Entgegenhaltungen:
**EP - A - 0 006 532**
**DE - A - 2 650 826**
**DE - A - 2 710 979**

(73) Patentinhaber: **Dr. Karl Thomae GmbH**
**Postfach 1755**
**D-7950 Biberach (Riss) (DE)**

(72) Erfinder: **Wetzel, Bernd, Dr. Dipl.-Chem.**
**Kapellenweg 21**
**D-7950 Biberach 1 (DE)**
Erfinder: **Woitun, Eberhard, Dr. Dipl.-Chem.**
**Stecherweg 17**
**D-7950 Biberach 1 (DE)**
Erfinder: **Maier, Roland, Dr. Dipl.-Chem.**
**Bodelschwinghstrasse 39**
**D-7950 Biberach 1 (DE)**
Erfinder: **Reuter, Wolfgang, Dr. Dipl.-Chem.**
**Nelkenweg 10**
**D-7951 Laupertshausen (DE)**
Erfinder: **Lechner, Uwe, Dr.**
**Panoramastrasse 12**
**D-7951 Ummendrof (DE)**
Erfinder: **Goeth, Hanns, Dr.**
**Oberer Bühl 6**
**D-7950 Biberach 1 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 021 176 B1

Cephalosporine, Verfahren zu ihrer Herstellung und diese Verbindungen enthaltende Arzneimittel

Die Erfindung betrifft neue Cephalosporine der allgemeinen Formel I

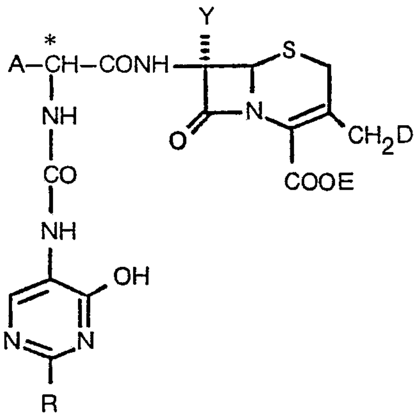

(I)

Verfahren zur Herstellung dieser Verbindungen und diese Verbindungen enthaltende Arzneimittel.

In der allgemeinen Formel I bedeuten:

A die Phenyl-, 4-Hydroxyphenyl-, 3- oder 3-Thienyl-, 2-Furyl- oder 3,4-Dihydroxyphenylgruppe,

Y ein Wasserstoffatom oder die Methoxygruppe,

D ein Wasserstoffatom, die Acetoxy-, Aminocarbonyloxy-, Pyridinium- oder Aminocarbonylpyridiniumgruppe oder die Gruppe —S-Het, in der Het die 1-Methyl-tetrazol-5-yl-, Tetrazol-5-yl-, 3-Methyl-1,2,4-thiadiazol-5-yl-, 1,2,4-Thiadiazol-5-yl-, 1,3,4-Thiadiazol-5-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder 1,2,4-Triazol-3-yl-Gruppe bedeutet,

R ein Wasserstoffatom, den Cyclopropylrest, die Hydroxygruppe, die Methoxygruppe, eine Gruppe der allgemeinen Formel

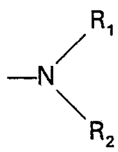

in der $R_1$ und $R_2$ gleich oder voneinander verschieden sein können und Wasserstoffatome, aliphatische, verzweigte oder unverzweigte Kohlenwasserstoffreste, die eventuell eine oder zwei Doppelbindungen oder eine Dreifachbindung und 1 bis 4 Kohlenstoffatome enthalten können, Cycloalkylreste mit 3 bis 6 Kohlenstoffatomen, durch einen Cycloalkylrest substituierte Methylgruppen mit 3 bis 6 Kohlenstoffatomen im Cycloalkylrest darstellen,

R bedeutet aber auch eine Gruppe der allgemeinen Formel —NH—Z—X, in der

Z eine geradkettige oder verzweigte Alkylgruppe mit 1 bis 3 Kohlenstoffatomen oder eine durch den Substituenten X substituierte Cycloalkylgruppe mit 5 oder 6 Kohlenstoffatomen bedeutet und

X die Hydroxygruppe, die Aminocarbonyl-, die Aminosulfonylgruppe oder die Aminocarbonylaminogruppe oder eine Gruppe der allgemeinen Formeln

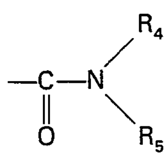 , $—NHCOR_5$, 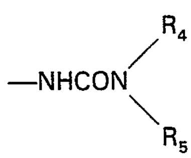 , $—NHSO_2R_4$,

$—OR_4$, $—OCOR_5$ $—SR_4$, $—SO—R_4$ und $—SO_2—R_4$

sein kann, wobei in diesen Formeln

$R_4$ eine geradkettige oder verzweigte Alkylgruppe mit 1 bis 3 Kohlenstoffatomen und $R_5$ Wasserstoff oder eine Alkylgruppe mit 1 bis 3 Kohlenstoffatomen darstellen,

R bedeutet weiter eine Gruppe der allgemeinen Formel

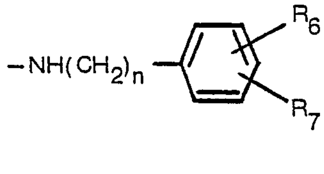

in der n null oder 1 ist und $R_6$ und $R_7$, die gleich oder voneinander verschieden sein können, Wasserstoff, Halogen, Alkylamino-, Dialkylamino, Hydroxy-, Alkoxy-, aliphatische Acylamino-, Aminocarbonylamino-, Nitro-, Alkylsulfonylamino-, Alkylcarbonyl-, Aminocarbonyl-, Alkylsulfinyl-, Alkylsulfonyl-, Aminosulfonyl- oder Alkyl- und Dialkylaminosulfonylgruppen bedeuten, wobei jeder Alkylrest in den genannten Gruppen 1 bis 3 Kohlenstoffatome enthalten kann,

E ein Wasserstoffatom oder eine in vitro oder in vivo leicht spaltbare Schutzgruppe. Als Carboxylschutzgruppen kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können.

Beispiele für in vitro leicht spaltbare Schutzgruppen sind z.B. die benzyl-, Diphenylmethyl-, Trityl-, t-Butyl-, die 2,2,2-Trichloräthyl- oder die Trimethylsilylgruppe.

Beispiele für in vivo leicht spaltbare Schutzgruppen sind z.B. Alkanoyloxyalkylgruppen, wie z.B. die Acetoxymethyl-, Propionyloxymethyl-, 2-Acetoxy-äthyl- oder Pivaloyloxy- methylgruppe oder die Phthalidyl- oder Indanylgruppe. Bedeutet E Wasserstoff, so fallen unter den Anspruch auch pharmazeutisch verträgliche Salze, wie z.B. Alkali- oder Erdalkalisalze, z.B. Natrium-, Kalium-, Magnesium- oder Calciumsalze, Ammoniumsalze, organische Aminsalze, z.B. mit Triäthylamin oder Dicyclohexylamin.

Steht für D Pyridinium oder Aminocarbonylpyridinium, dann haben die Verbindungen der Erfindung die allgemeine Formel Ia

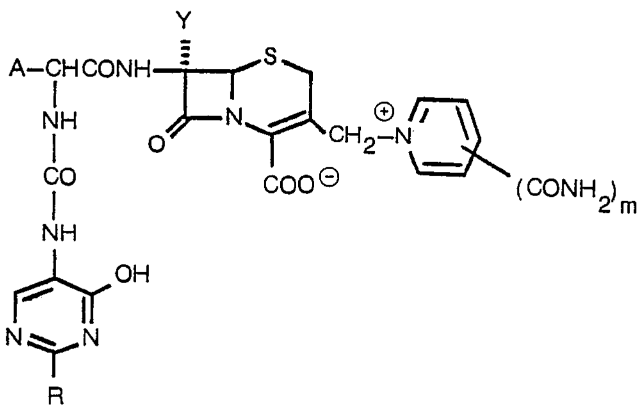

(Ia)

in der m die Zahl 0 oder 1 darstellt.

Bevorzugt sind diejenigen Verbindungen der allgemeinen Formel I, worin

A die p-Hydroxyphenyl- oder 2-Thienylgruppe,

Y ein Wasserstoffatom oder die Methoxygruppe,

D die Acetoxy oder Pyridiniumgruppe oder eine Gruppe der allgemeinen Formel —SHet bedeuten, worin Het für die 2-Methyl-1,3,4-thiadiazol-5-yl-, 2-Methylamino-1,3,4-thiadiazol-5-yl-, 2-Acetylamino-1,3,4-thiadiazol-5-yl-, oder 1-Methyl-tetrazol-5-ylgruppe steht und R die folgenden Bedeutungen besitzt:

die Cyclopropylgruppe, die n-Propylamino-, 4-Hydroxy-cyclohexylamino- oder 3-Hydroxy-n-propylaminogruppe oder eine Gruppe der allgemeinen Formel

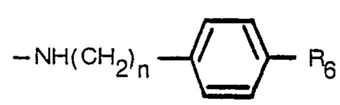

in der n null oder 1 ist und $R_6$ ein Wasserstoffatom, die Hydroxy-, Aminocarbonyl-, Aminosulfonyl- oder Methylaminosulfonylgruppe darstellt, und E ein Wasserstoffatom oder eine wie in Anspruch 1 erwähnte Estergruppe bedeutet.

Bekannt sind Lactame (DE—OS 27 20 979) und Cephenderivate (DE—OS 26 50 826), die an der Ureidogruppe eine substituierte Pyridylgruppe besitzen; besonders beschrieben wurde die D-7-[$\alpha$-(N'-4-Hydroxy-3-pyridyl-ureido)-$\alpha$-phenyl-acetamido]-cephalosporansäure bzw. ihr Natriumsalz. Es wurde überraschenderweise gefunden, daß durch die Einführung einer substituierten Pyrimidinylgruppe anstelle der substituierten Pyridylgruppe Verbindungen entstehen, die eine wesentlich bessere Hemmwirkung insbesondere gegen eine ganze Reihe gramnegativer Keime zeigen.

Die Cephalosporinverbindungen der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formen I oder I' überwiegt:

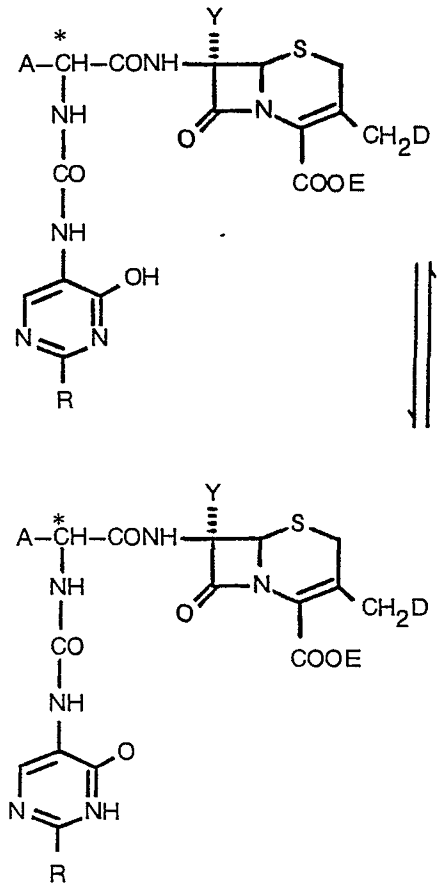

(I)

(I')

Es versteht sich von selbt, daß die eingangs angegebenen Verbindungen der allgemeinen Formel I immer beide Tautomeren umfassen.

Die Verbindungen der allgemeinen Formel I können bezüglich des Chiralitätszentrums C* in den beiden möglichen R- und S-Konfigurationen, jedoch auch als ein Gemisch dieser beiden Konfigurationen, vorliegen. Besonders bevorzugt sind solche Verbindungen, für welche die D=R-Konfiguration zutrifft.

Wenn das Endprodukt in der D,L-Form anfällt, gelingt es, die reinen D- und L-Diastereomeren durch präparative Flüssigkeitschromatographie (HPLC) herzustellen.

Die Verbindungen der allgemeinen Formel I können wie folgt hergestellt werden:

1. Zur Herstellung von Verbindungen der allgemeinen Formel I, in der D die angegebene Bedeutung mit Ausnahme der einer Pyridinium- oder Aminocarbonylpyridiniumgruppe besitzt, durch Umsetzung einer Verbindung der allgemeinen Formel II

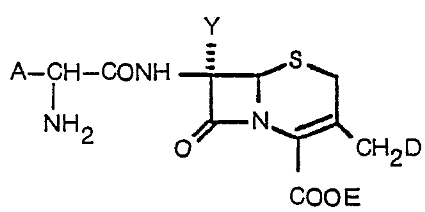

(II)

in der A, Y und E die oben angegebene Bedeutung und D die oben angegebene Bedeutung mit Ausnahme der einer Pyridinium- oder Aminocarbonylpyridiniumgruppe besitzen, mit einem Pyrimidinderivat der allgemeinen Formel III

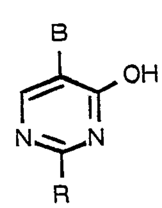

(III)

in der R wie oben definiert ist und B die Gruppe =NCO oder ein reaktives Derivat der Gruppe —NHCOOH bedeutet, wie z.B. die Gruppen —NHCOCl, —NHCOBr oder

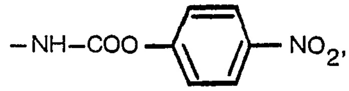

wobei die Gruppen =NCO und —NHCOCl besonders bevorzugt sind. Es können auch Gemische von solchen Pyrimidinderivaten der allgemeinen Formel III verwendet werden, in der B teils die eine und teils die andere der vorstehend genannten Bedeutungen besitzt, z.B. die Gruppen =NCO und —NHCOCl gleichzeitig nebeneinander.

Bedeutet E ein Wasserstoffatom, so können die Ausgangsprodukte der allgemeinen Formel II in Form ihrer anorganischen oder organischen Salze, z.B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin läßt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäureäthylester, aromatischen Lösungsmitteln, z.B. Benzol ausführen, wobei es zweckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Bedeutet E die Trimethylsilylgruppe, d.h. verwendet man als Ausgangsprodukte für das erfindungsgemäße Verfahren die Silylderivate der Verbindungen der allgemeinen Formel II (z.B. Mono- oder zweckmäßigerweise die an der Amino- und Carboxylgruppe silylierten Di-trimethylsilylderivate) und setzt sie mit Verbindungen der allgemeinen Formel III um, so arbeitet man im allgemeinen zweckmäßigerweise in wasser- und hydroxyl-gruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Bedeutet E eine der anderen oben genannten in vitro oder in vivo leicht abspaltbaren Schutzgruppen, z.B. die Diphenylmethylgruppe oder die Pivaloyloxymethylgruppe, so empfiehlt es sich ebenfalls in einem aprotischen Lösungsmittel zu arbeiten, z.B. in absolutem Methylenchlorid, Chloroform, Tetrahydrofuran oder Dimethylformamid.

Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calciumhydroxid, Calciumoxid, Natrium- und Kaliumcarbonat, Natrium- und Kaliumhydrogencarbonat, Äthylamin, Methyl-äthylamin, Triäthylamin, Hydroxyäthylamin, Anilin, Dimethylanilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris-(hydroxymethyl)-amino-methan-Puffer.

Die Reaktionstemperaturen können in einem größeren Bereich variiert werden. Im allgemeinen arbeitet man zwischen —20 und +50°C, vorzugsweise zwischen 0 und +20°C.

Die Reaktionspartner der allgemeinen Formeln II und III können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2) Zur Herstellung von Verbindungen der allgemeinen Formel I, in der D die oben angegebenen

Bedeutungen mit Ausnahme einer Pyridinium- oder Aminocarbonylpyridiniumgruppe besitzt, durch Umsetzung von Ureidocarbonsäuren der allgemeinen Formel IV,

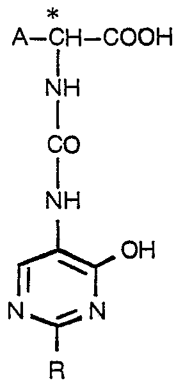

(IV)

in der A und R die oben angegebenen Bedeutungen besitzen, ihren Salzen oder reaktiven Derivaten, mit 7-Amino-cephalosporansäurederivaten der allgemeinen Formel V

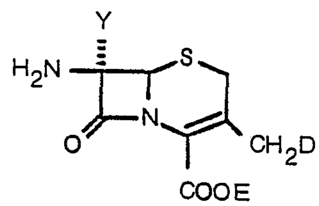

in der E und Y wie oben definiert sind, und D die oben angegebenen Bedeutungen mit Ausnahme der einer Pyridinium- oder Aminocarbonylpyridiniumgruppe besitzt.

Als reaktive Derivate der Ureidocarbonsäuren der allgemeinen Formel IV kommen beispielsweise deren Säureanhydride wie z.B. die, die sich von Chlorameisensäureestern, z.B. Chlorameisensäureäthyl- oder-isobutylester, ableiten, oder deren reaktive Ester, wie der p-Nitrophenylester oder der N-hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der β-Lactamchemie bekannt sind, verwendet werden.

Die 7-Aminocephalosporansäurederivate werden vorteilhafterweise in Form eines in vitro oder in vivo leicht spaltbaren Derivates eingesetzt. Hierfür kommen z.B. die Verbindungen der allgemeinen Formel V in Frage, bei denen E die eingangs gegebene Bedeutung mit Ausnahme der eines Wasserstoffatoms besitzt; besonders bevorzugte Derivate sind der Diphenylmethylester, der t-Butylester, der Trimethylsilylester oder das N,O-Bis-trimethylsilylderivat.

Beispielsweise werden die Ureidocarbonsäure, ihre Salze oder ihre reaktiven Derivate mit den 7-Aminocephalosporansäurederivaten in einem Lösungsmittel bei Temperaturen zwischen −40 und +40°C, gegebenenfalls in Gegenwart einer Base, umgesetzt. Wird z.B. ein Anhydrid der Ureidocarbonsäure, beispielsweise das Anhydrid mit dem Äthylchloroformiat, eingesetzt, so wird die Reaktion unter Kühlung, beispielsweise bei −10 bis +10°C in Gegenwart eines tertiären Amins, wie Triäthylamin oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel V um, so wird die Reaktion vorzugsweise bei 0 bis 20°C in Anwesenheit einer Base, wie z.B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel V erfolgt vorteilhafterweise in Gegenwart eines Kondensationsmittels, z.B. in Gegenwart von N,N'-Dicyclohexylcarbodiimid.

3) Die Verbindungen der allgemeinen Formel I, in der D entweder die Gruppe-SHet mit den oben für Het angegebenen Bedeutungen oder die Pyridinium- oder Aminocarbonylpyridiniumgruppe bedeutet, und E für Wasserstoff steht, können durch Umsetzung einer Verbindung der allgemeinen Formel VI

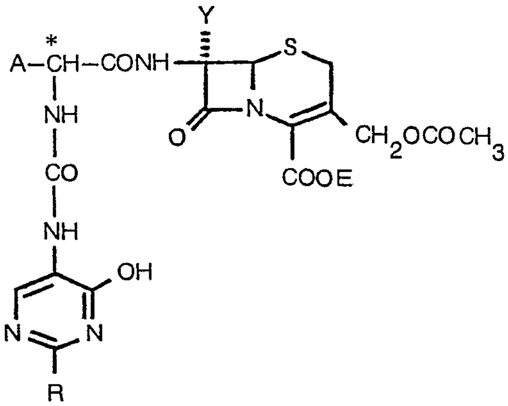

(VI)

in der A, R und Y die oben angegebenen Bedeutungen haben und E Wasserstoff bedeutet, entweder mit einer Verbindung der allgemeinen Formel VII

Het—S—M (VII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin hergestellt werden. Man erhält damit eine Verbindung der allgemeinen Formel I, in der D die Bedeutungen einer -SHet, Pyridinium- oder 4-Aminocarbonylpyridiniumgruppe und E die eines Wasserstoffatoms besitzt. Dazu wird z.B. eine Verbindung der Formel VI mit beispielsweise 5-Methyl-2-mercapto-1,3,4-thiadiazol in einem Lösungsmittel, z.B. Wasser, Methanol, Äthanol, Aceton, Methyläthylketon, Tetrahydrofuran, Acetonnitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser oder dergleiche, verwendet: In diesem Fall wird der pH-Wert der Reaktionslösung vorteilhafterweise auf 2—10 und insbesondere auf 4—8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalweise wird die Umsetzung bei einer Temperatur im Bereich von 0 bis 100°C während einer Zeitdauer von einigen Stunden durchgeführt.

4) Eine Verbindung der allgemeinen Formel I, in der Y die Methoxygruppe darstellt, kann dadurch erhalten werden, daß man eine Verbindung der allgemeinen Formel I, in der Y ein Wasserstoffatom bedeutet, in Anwesenheit von Methanol mit einem Alkalimetallethylat der allgemeinen Formel $M^{\oplus}OCH_3^{\ominus}$, worin $M^{\oplus}$ ein Alkalimetallion bedeutet, und dann mit einem Halogenierungsmittel umsetzt. Dazu wird ein Cephalosporin der allgemeinen Formel I, in der Y ein Wasserstoffatom darstellt, in einem inerten Lösungsmittel, z.B.

Tetrahydrofuran, Dioxan, Äthylenglykoldimethyläther, Methylenchlorid, Chloroform, Dimethylformamid oder Methanol oder in einem Gemisch von zweien dieser Lösungsmittel aufgelöst oder suspendiert. Zu der erhaltenen Lösung oder Suspension gibt man ein Alkalimetallmethylat zusammen mit Methanol. Das erhaltene Gemisch wird zur Reaktion gebracht und das Reaktionsgemisch wird dann mit einem Halogenierungsmittel umgesetzt. Bei dieser Reaktion verwendet man Methanol im Überschuß und die Menge des Alkalimetallmethylats beträgt vorzugsweise 2 bis 6 Äquivalente pro Äquivalent des verwendeten Cephalosporins. Der Ausdruck "im Überschuß" bedeutet eine Menge von mehr als einem Äquivalent pro Äquivalent des Cephalosporins. Alle Reaktionen werden bei —120 bis —10°C und vorzugsweise —100 bis —50°C durchgeführt. Eine Reaktionszeit von 5 bis 30 Min. reicht aus. Die Reaktion wird durch Ansäuern des Reaktionssystems abgebrochen.

Das bei diesem Verfahren eingesetzte Halogenierungsmittel ist allgemein als Quelle für positive Halogenatome, z.B. $Cl^{\oplus}$ oder $Br^{\oplus}$, $J^{\oplus}$ bekannt. Beispiele solcher Halogenierungsmittel sind Halogen, wie Chlor, Brom usw.; N-Halogenimide, wie N-Chlorsuccinimid und N-Bromsuccinimid; N-Halogenamide wie N-Chloracetamid und N-Bromacetamid, N-Halogensulfonamide, wie N-Chlorbenzolsulfonamid und N-Chlor-p-toluolsulfonamid; 1-Halogenbenzotriazole; 1-Halogentriazine, organische Hypohalogenite, wie tert.-Butylhypochlorit und tert.-Butylhypojodit; Halogenhydantoine, wie N,N-Dibromhydantoin. Unter diesen Halogenierungsmitteln ist tert.-Butylhypochlorit bevorzugt. Das Halogenierungsmittel wird in einer Menge eingesetzt, welche ausreicht, eine zur Menge des Cephalosporins der allgemeinen Formel VI äquivalente Menge positives Halogen abzugeben.

Geeignete Säuren für das Abbrechen der Reaktion sind solche, welche bei Zusatz zum kalten Reaktionsgemisch nicht zu einer Verfestigung des Reaktionsgemisches oder zum Gefrieren des Reaktionsgemisches zu einer schweren viskosen Masse führen. Beispiele geeigneter Säuren sind 98%-ige Ameisensäure, Eisessig, Trichloressigsäure und Methansulfonsäure. Nach dem Abbrechen der Reaktion wird das überschüssige Halogenierungsmittel durch behandlung mit einem Reduktionsmittel, z.B. Trialkylphosphit oder Natriumthiosulfat entfernt.

Die nach den Verfahren 1,2 und 4 hergestellten erfindungsgemäßen Verbindungen, in denen E für eine in vitro leicht abspaltbare Schutzgruppe steht, können nach bekannten Methoden der Cephalosporinchemie in die freien Carbonsäuren der allgemeinen Formel I, in der E ein Wasserstoffatom ist, überführt werden. So kann beispielsweise die Trimethylsilylgruppe leicht durch wäßrige Hydrolyse entfernt werden, die Benzhydrylgruppe z.B. durch hydrolytische Spaltung mit Trifluoressigsäure. Diese Eliminierung der Schutzgruppen sind literaturbekannt.

Ebenso können die Cephalosporine der allgemeinen Formel I, in der E ein Wasserstoffatom bedeutet, in die Acyloxyalkylester, worin E z.B. einen Pivaloyloxymethylrest

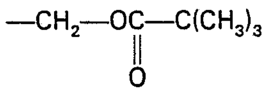

darstellt, überführt werden, indem man ein Alkalisalz der Cephalosporincarbonsäure, beispielsweise ein Natrium- oder Kaliumsalz, mit einem Pivaloyloxymethylhalogenid der Formel

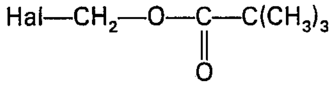

worin Hal für Chlor, Brom oder Jod steht, umsetzt. Weitere geeignete Acyloxyalkylhalogenide sind z.B. Chlormethylacetat, Brommethylpropionat oder 1-Bromäthylacetate.

Die Herstellung des Acyloxyalkylesters der allgemeinen Formel I wird vorgenommen, indem man das jeweilige Alkalisalz der Stammsäure in einem inerten Lösungsmittel mit einem leichten molaren Überschuß des Jod-, Brom- oder Chloralkylesters, wie Pivaloyloxymethyljodid, bei Raumtemperatur oder leicht erhöhter Temperatur bis zu etwa 40 bis 45°C umsetzt. Als Lösungsmittel können beispielsweise Aceton, Tetrahydrofuran, Dioxan, Dimethylformamid oder Methylenchlorid verwendet werden.

Die Indanylester der allgemeinen Formel I, worin E für einen Rest der Formel

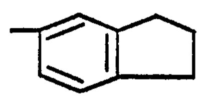

steht, können hergestellt werden, indem man 5-Indanol in einem inerten Lösungsmittel, wie Dioxan oder Tetrahydrofuran, mit der freien Säureform einer Verbindung der allgemeinen Formel I, worin E für Wasserstoff steht, in Gegenwart eines Kondensationsmittels, wie eines Diimids, beispielsweise Dicyclohexylcarbodiimid, umsetzt. Die Umsetzung wird unter Rühren bei einer Temperatur von etwa 20 bis 35°C über eine Zeitspanne von etwa 6 bis 8 Stunden vorgenommen. Zur Isolierung des Indanylesters wird das Reaktonsgemisch erst mit Wasser verdünnt, worauf man den unlöslichen Dicyclohexylharnstoff vom Reaktionsgemisch abfiltriert. Der Ester wird dann aus dem Filtrat extrahiert.

Die Indanylester lassen sich ferner auch herstellen, indem man ein aus einer Cephalosporansäure der allgemeinen Formel I und Essigsäure gebildetes Anhydrid mit 5-Indanol umsetzt.

Die Phthalidylester der allgemeinen Formel I, worin $R_3$ für die Phthalidylgruppe der Formel

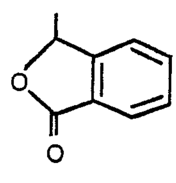

steht, können hergestellt werden, indem man Bromphthalid der Formel

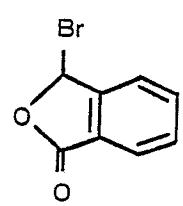

mit einem Salz einer Cephalosporansäure der allgemeinen Formel I umsetzt. Die Veresterung kann vorgenommen werden, indem man ein Gemisch aus äquimolaren Mengen des Cephalosporinsalzes, wie des Natrium- oder Kaliumsalzes, und Bromphthalid in Dimethylformamid, Dimethylacetamid, Dioxan, Tetrahydrofuran oder Gemischen hiervon langsam erwärmt.

Die Aufarbeitung der nach den genannten Verfahren erhaltenen Reaktionsgemische nach erfolgter

Umsetzung wird nach den bei $\beta$-Lactam-Anitibiotica gebräuchlichen Methoden vorgenommen; dasselbe gilt für die Isolierung und Reinigung der Endprodukte, beispielsweise für die Freisetzung der Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanot oder die Umsetzung der freien Säure mit der entsprechenden Menge Natriumbicarbonat unter pH-Kontrolle und anschließender Gefriertrocknung.

Typische Ausgangsprodukte der allgemeinen Formel II, bei denen A Phenyl, substituiertes Phenyl oder Thienyl bedeutet und D z.B. Für 1-Methyl-1H-tetrazol-5-yl oder 2-methyl-1,3,4-thiadiazol-5-yl steht, sind literaturbekannt, vgl. z.B. US—PS 3 641 021. Ausgangsprodukte der allgemeinen Formel II, bei denen A eine Furyl- oder Thienylgruppe sowie ein Wasserstoffatom oder die Methoxygruppe bedeutet, werden z.B. in J. Antibiotics 31, S. 546, bzw. S. 560 (1978) beschrieben.

Ebenso sind die Ausgangsprodukte der allgemeinen Formel V literaturbekannt. Zu solchen 7-Aminocephalosporansäuresystemen, bei denen D für ein heterocyclisches System steht, gelangt man beispielsweise, wenn man 7-Aminocephalosporansäure mit dem entsprechenden Mercaptoheterocyclus in bekannter Weise umsetzt.

Die Ausgangsstoffe der allgemeinen Formel III können beispeielsweise durch Umsetzung der entsprechenden 5-Aminopyrimidine der allgemeinen Formel VIII

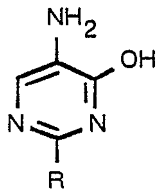

(VIII)

in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen —40 und +60°C, vorzugsweise zwischen —10 und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel VIII in einem der erwähnten Lösungsmittel schwer löslich sein, kann die Phosgenierung auch in heterogener Phase durchgeführt werden. Des weiteren können in einer besonders bevorzugten Ausführungsform die Aminopyrimidine der allgemeinen Formel IX durch Behandlung mit einem Silylierungsmittel, wie Hexamethyldisilazan, Trimethylchlorsilan/Triäthylamin, Trimethylsilyldiäthylamin oder N,O-Bis-trimethylsilylacetamid in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel III reagiert, wobei bevorzugt ohne Basenzusatz gearbeitet wird.

Je nach der Art des Lösungsmittels, der Höhe der Temperatur, der Menge und Art einer eventuell zugesetzten Base entsteht entweder überwiegend das entsprechende Isocyanat oder Carbaminsäurehalogenid oder ein Gemisch dieser beiden Verbindungen. Je nach den Bedingungen liegt das Isocyanat der allgemeinen Formel VI auch teilweise oder ganz als ein den Isocyanaten isomeres Dihydro-oxazolopyrimidin der allgemeinen Formel IIIa

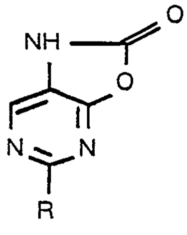

(IIIa)

oder ein je nach Art des Substituenten R ein oder mehrfach silyliertes Analoges vor.

Die durch Phosgenierung entstanden Ausgangsprodukte der allgemeinen Formel III bzw. IIIa oder deren Gemische oder silylierte Analoge sind in den obenerwähnten Lösungsmitteln im allgemeinen gut löslich und können, nach Entfernung des überschüssigen Phosgens, ohne weitere Reinigung mit den entsprechenden Cephalosporinderivaten der allgemeinen Formel II direkt umgesetzt werden.

Es ist jedoch auch möglich, das Zwischenprodukt der allgemeinen Formel IIa zu isolieren, es gegebenenfalls mit einem protischen Lösemittel, beispielsweise Wasser oder Methanol zu entsilylieren, es auf Grund seiner Löslichkeitseigenschaften zu reinigen und in der oben dargelegten Weise umzusetzen.

Synthesen für 2-substituierte 5-Amino-4-hydroxy-pyrimidine der allgemeinen Formel VIII werden in den DE—OSS 28 08 153 und 29 10 190 beschrieben.

Die Ureidocarbonsäuren der allgemeinen Formel IV lassen sich leicht durch Umsetzung der Pyrimidinderivate der allgemeinen Formel III mit Glycinderivaten der allgemeinen Formel IX

$$A{-}\overset{*}{C}H{-}COOH \qquad (IX)$$
$$\quad | $$
$$\quad NH_2$$

in der A wie oben definiert ist, erhalten. Die Umsetzung erfolgt bei Temperaturen zwischen −20 und +40°C, vorzugsweise zwischen 0 und +20°C, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Cephalosporinantibioika werden in großen Umfang bei der Behandlung von Krankheiten verwendet, die durch pathogene Bakterien bei Mensch und Tier hervorgerufen werden, und sind insbesondere bei der behandlung von Krankheiten verwendbar, die durch Bakterien hervorgerufen werden, welche gegenüber anderen Antibiotika, wie Penicillinverbindungen, resistent sind, sowie bei einer Behandlung von penicillinempfindlichen Patienten. In zahlreichen Fällen ist es erwünscht, ein Cephalosporinantibiotikum zu verwenden, das sowohl gegenüber grampositiven als auch gramnegativen Mikroorganismen eine Aktivität besitzt, und es wurden umfangreiche Untersuchungen auf die Entwicklung verschiedenartiger Typen von Cephalosporinantibiotika mit breitem Wirkungsspektrum gerichtet.

Bei diesen Untersuchungen hat es sich herausgestellt, daß es schwierig ist, Cephalosporinantibiotika zu finden, die neben einem breiten Wirkungsspektrum auch gegen verschiedenartige Stämme von Pseudomonas aeruginosa eine hohe Aktivität besitzen. Es wurde zwar versucht, entsprechend den Untersuchungen auf dem Penicillingebiet durch Acylierung von $\alpha$-Aminobenzylcephalosporinen zu Pseudomonas-wirksamen Cephalosporinen zu gelangen, doch erwiesen sich derartige Verbindungen in der Regel als zu wenig aktiv. Es besteht daher weiter ein Bedürfnis, nach neuen Cephalosporinen zu suchen, die eine gesteigerte Wirksamkeit gegen verschiedenste Stämme von Pseudomonas aeruginosa neben einem breiten Wirkungsspektrum besitzen.

Während wie erwähnt, allgemein über Acylderivate von $\alpha$-Aminobenzylderivaten intensiv geforscht wurde und noch wird, ist nur wenig über Derivate bekannt geworden, bei denen ein heterocyclisches System über eine Ureidobrücke (—NHCONH—) an das $\alpha$-Benzylkohlenstoffatom von $\alpha$-Aminobenzylcephalosporinen geknüpft ist. Lediglich in der DE—OS 2 710 979 und DE—OS 2 650 826 werden Cephalosporine beschrieben, bei denen Pyridine bzw. kondensierte Pyridine in der genannten Weise mit dem Cephalosporingerüst verknüpft sind.

Diese Verbindungen weisen jedoch eine unbefriedigende Pseudomonaswirkung auf.

Im Gegensatz dazu wurde gefunden, daß einige der erfindungsgemäßen Verbindung hinsichtlich ihrer antibiotischen Aktivität ein breites Wirkungsspektrum zusammen mit einer ungewöhnlich guten Wirksamkeit gegen Pseudomonas-Stämmen zeigen. Die hohe Aktivität erstreckt sich auch auf zahlreiche $\beta$-Lactamase bildende, gramnegative Stämme, da diese Verbindungen eine hohe Stabilität gegenüber $\beta$-Lactamasen, die von einer Reihe gramnegativer Organismen gebildet werden, besitzen. Außerdem sind die erfindungsgemäßen Wirkstoffe sehr gut verträglich, sie können daher zur Prophylaxe und Chemotherapie von lokalen und systemischen Infektionen in der Human- und Tiermedizin verwendet werden.

Als Krankheiten, die durch die erfindungsgemäßen Verbindungen verhindert bzw. geheilt werden können, seien beispielsweise solche der Atmungswege, des Rachenraumes und der Harnwege genannte; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien verwendet werden, besonders von organischen Materialien, wie Polymeren, Schmiermittel, Farben, Fasern, Leder, Papier und Holz sowie von Lebensmitteln.

Dieses wird wie bereits dargelegt dadurch ermöglicht, daß die Verbindungen der allgemeinen Formel I sowohl in vitro als auch in vivo gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Mischungen der folgenden Erreger verursacht werden:
Micrococcaceae, wie Staphylokakken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe,

Klebsiella-Bakterien, z.B. K. pneumoniae;
Proteae-Bakterien der Proteus-Gruppe z.B. Proteus vulgaris;
Salmonella-Bakterien, z.B. S. thyphimurium;
Shigella-Bakterien, z.B. Shigella dysenteriae,
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;
Parvobacteriaceae oder Brucellaceae, wie Pateurella-Bakterien;
Brucella-Bakterien, z.B. Brucella abortus;
Haemophilus-Bakterien, z.B. Haemophilus influenzae;
Bordetella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema palladum;
Leptospira-Bakterien, wie Leptospira interrogans.

Die obige Aufzählung von Eregern ist lediglich beispielhaft und keineswegs beschränkend aufzufassen.

Spezielle Beispiele für Verbindungen der allgemeinen Formel I werden in der folgenden Tabelle genannt:

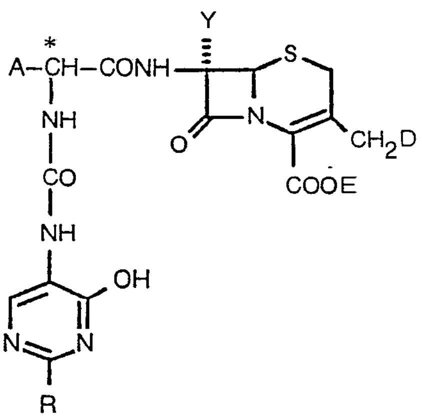

TABELLE 1

| | A | R | Y | D | E |
|---|---|---|---|---|---|
| 1 | Phenyl | H | H | H | H |
| 2 | Phenyl | $CH_3$ | H | H | H |
| 3 | Phenyl | 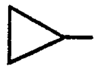 | H | H | H |
| 4 | p-HO-phenyl | 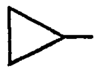 | H | H | H |
| 5 | p-HO-phenyl | 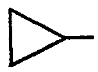 | H | $OCOCH_3$ | H |
| 6 | p-HO-phenyl | 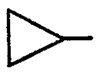 | H | 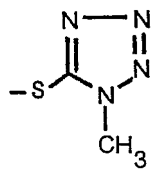 | H |
| 7 | p-HO-phenyl | 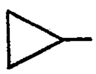 | H | 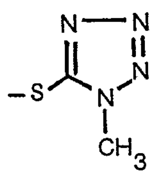 | $-CH_2OCOC(CH_3)_3$ |
| 8 | p-HO-phenyl | 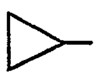 | H | 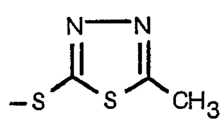 | H |
| 9 | p-HO-phenyl | 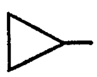 | $CH_3O-$ | 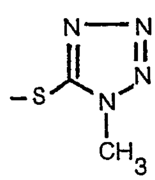 | H |
| 10 | 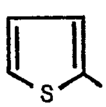 | 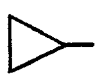 | H | 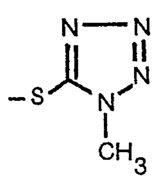 | H |

TABELLE 1 (Fortsetzung)

| | A | R | Y | D | E |
|---|---|---|---|---|---|
| 11 | 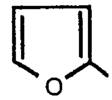 | 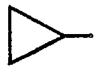 | H | 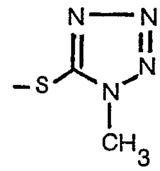 | H |
| 12 | p-HO-phenyl | HO– | H | $-OCOCH_3$ | H |
| 13 | p-HO-phenyl | $CH_3O-$ | H | 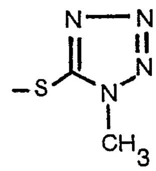 | H |
| 14 | p-HO-phenyl | $(CH_3)_2N-$ | H | 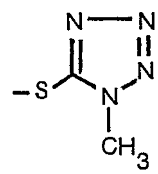 | H |
| 15 | Phenyl | $NH_2-$ | H | H | H |
| 16 | p-HO-phenyl | $-NHCH_3$ | H | $-OCOCH_3$ | H |
| 17 | p-HO-phenyl | $-NHC_2H_5$ | H | 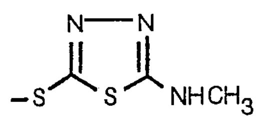 | H |
| 18 | p-HO-phenyl | $-NHC_3H_7$ | H | $-OCOCH_3$ | H |
| 19 | p-HO-phenyl | $-NHC_3H_7$ | H | 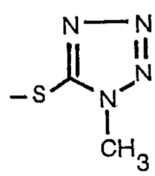 | H |
| 20 | p-HO-phenyl | $-NHC_3H_7$ | $-OCH_3$ | 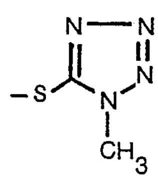 | H |
| 21 | p-HO-phenyl | $-NHC_3H_7$ | H | 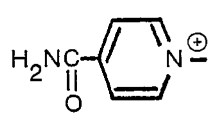 | – |
| 22 | p-HO-phenyl | $-NHCH(CH_3)_2$ | H | H | H |

TABELLE 1 (Fortsetzung)

| | A | R | Y | D | E |
|---|---|---|---|---|---|
| 23 | p-HO-phenyl | $-NHCH(CH_3)_2$ | H | $-OCOCH_3$ | H |
| 24 | p-HO-phenyl | $-NHCH(CH_3)_2$ | H | 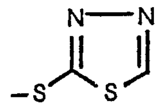 | H |
| 25 | p-HO-phenyl | $-NHCH(CH_3)_2$ | H | 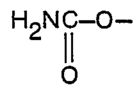 | H |
| 26 | p-HO-phenyl | $-NHCH(CH_3)_2$ | H | 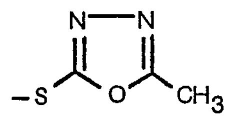 | H |
| 27 | p-HO-phenyl | $-NHCH(CH_3)_2$ | H | 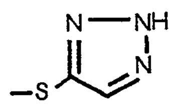 | H |
| 28 | p-HO-phenyl | $-NHCH(CH_3)_2$ | H | 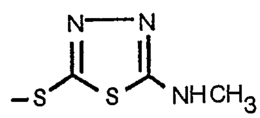 | H |
| 29 | p-HO-phenyl | $-NHCH(CH_3)_2$ | H | 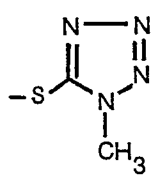 | H |
| 30 | p-HO-phenyl | $-NHCH(CH_3)_2$ | $-OCH_3$ | 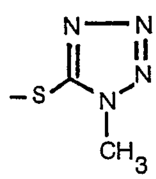 | H |
| 31 | p-HO-phenyl | $-NHCH(CH_3)_2$ | H | 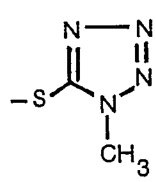 | $-CH_2OCOC(CH_3)_3$ |
| 32 | p-HO-phenyl | $-NHCH(CH_3)_2$ | H | 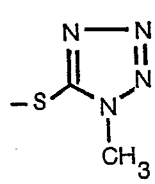 | 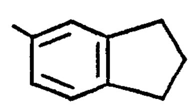 |

TABELLE 1 (Fortsetzung)

| | A | R | Y | D | E |
|---|---|---|---|---|---|
| 33 | p-HO-phenyl | $-NHCH(CH_3)_2$ | H | 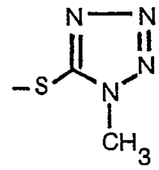 | 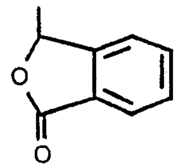 |
| 34 | 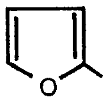 | $-NHC_3H_7$ | H | 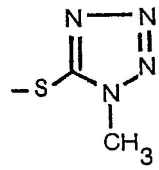 | H |
| 35 | 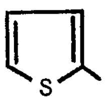 | $-NHC_3H_7$ | H | 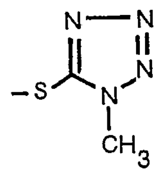 | H |
| 36 | 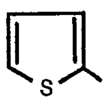 | $-NHC_3H_7$ | $-OCH_3$ | 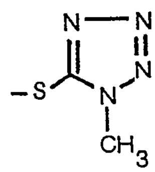 | H |
| 37 | p-HO-phenyl | $-NHCH_2CH(CH_3)_2$ | H | 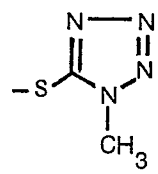 | H |
| 38 | p-HO-phenyl | $-NHCH_2CH(CH_3)_2$ | H | 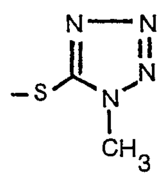 | H |
| 39 | p-HO-phenyl | $-NHCH_2CH(CH_3)_2$ | H | 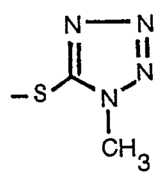 | H |
| 40 | p-HO-phenyl | $-NHC_4H_9$ | H | 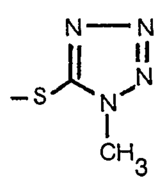 | H |

TABELLE 1 (Fortsetzung)

| | A | R | Y | D | E |
|---|---|---|---|---|---|
| 41 | 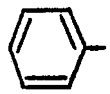 | $-NHCH_2CH=CH_2$ | H | $-OCOCH_3$ | H |
| 42 | p-HO-phenyl | $NHCH_2CH=CH_2$ | H | 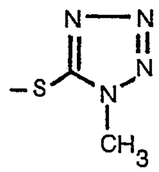 | H |
| 43 | p-HO-phenyl | $-NHCH_2CH=CH_2$ | $-OCH_3$ | 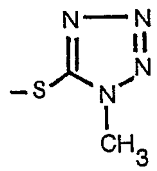 | H |
| 44 | p-HO-phenyl | $-NHCH_2CH=CH-CH_3$ | H | 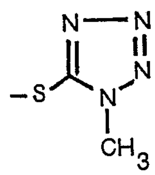 | H |
| 45 | p-HO-phenyl | $-NHCH_2CH=CH-CH_3$ | H | 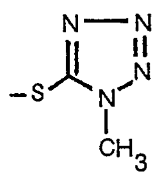 | $-CH_2OCOC(CH_3)_3$ |
| 46 | 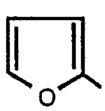 | $-NHCH_2CH=CH-CH_3$ | H | 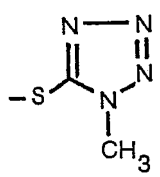 | H |
| 47 | p-HO-phenyl | $-NHCH_2CH=CH-CH_3$ | H | 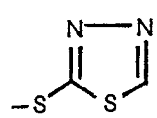 | H |
| 48 | p-HO-phenyl | $-NHCH_2CH=CH-CH_3$ | H | $-OCONH_2$ | H |
| 49 | p-HO-phenyl | $-NHCH_2C=CH_2$<br>$CH_3$ | H | 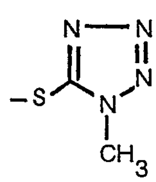 | H |

TABELLE 1 (Fortsetzung)

| | A | R | Y | D | E |
|---|---|---|---|---|---|
| 50 | 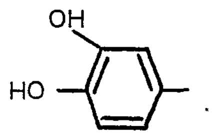 | 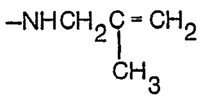 | H | 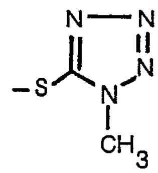 | H |
| 51 | p-HO-phenyl | $-NHC_6H_{11}$ | H | 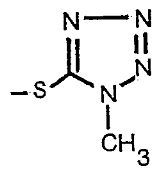 | H |
| 52 | 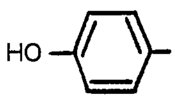 | $-NHCH_2C \equiv CH$ | H | 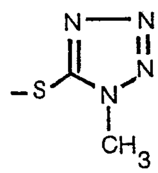 | H |
| 53 | 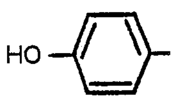 | $-NHCH_2C \equiv CH$ | H | 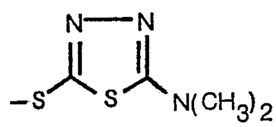 | H |
| 54 | 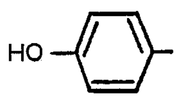 | 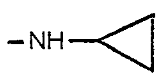 | H | 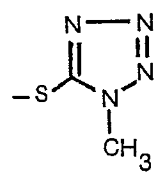 | H |
| 55 | 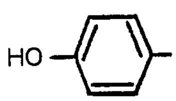 | 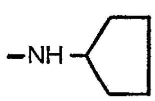 | H | 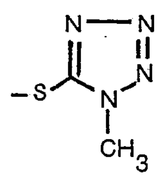 | H |
| 56 | 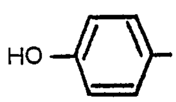 | 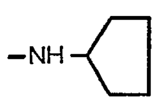 | $-OCH_3$ | 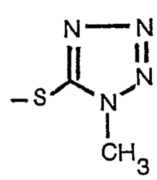 | H |
| 57 | 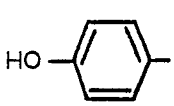 | 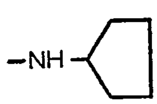 | H | 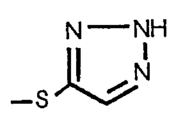 | H |

TABELLE 1 (Fortsetzung)

| | A | R | Y | D | E |
|---|---|---|---|---|---|
| 58 | 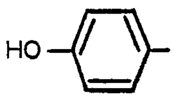 | 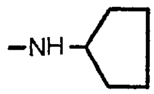 | H | 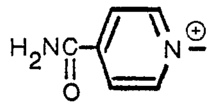 | – |
| 59 | 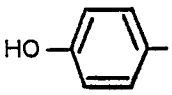 | 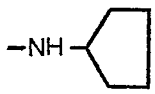 | H | 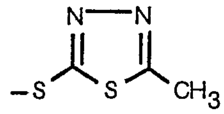 | H |
| 60 | 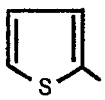 | 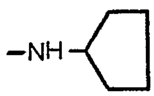 | H | 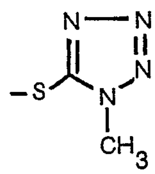 | H |
| 61 | 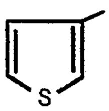 | 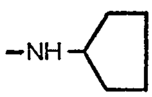 | H | 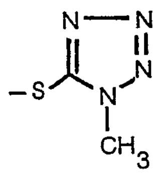 | H |
| 62 | 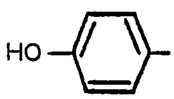 | 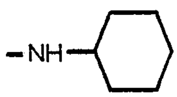 | H | 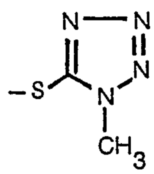 | H |
| 63 | 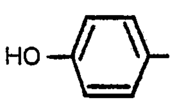 | 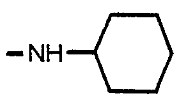 | H | –$OCOCH_3$ | H |
| 64 | 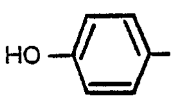 | 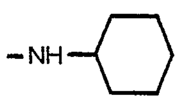 | H | 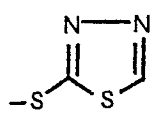 | H |
| 65 | 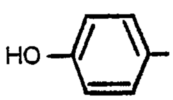 | 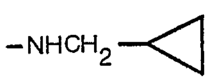 | H | 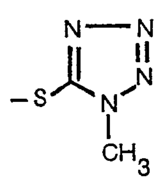 | H |
| 66 | 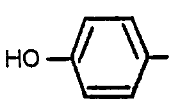 | 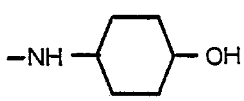 | H | H | H |

TABELLE 1 (Fortsetzung)

| | A | R | Y | D | E |
|---|---|---|---|---|---|
| 67 | 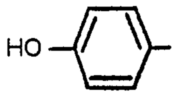 | 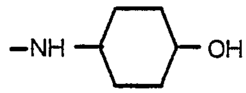 | H | $-OCOCH_3$ | H |
| 68 | 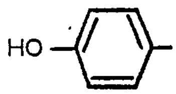 | 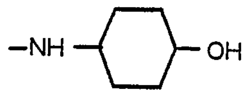 | H | 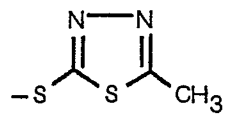 | H |
| 69 | 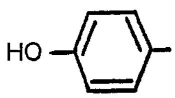 | 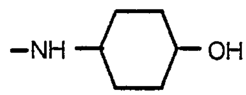 | H | 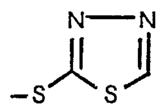 | H |
| 70 | 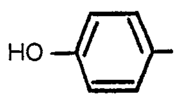 | 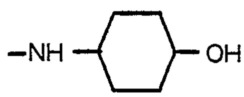 | H | 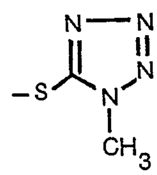 | H |
| 71 | 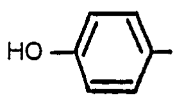 | 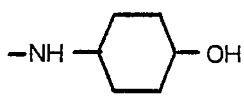 | $-OCH_3$ | 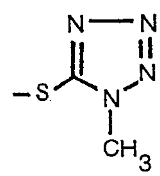 | H |
| 72 | 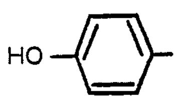 | 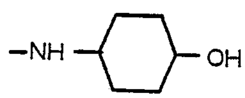 | H | 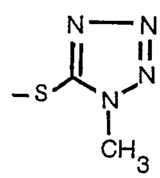 | $-CH_2O\underset{O}{\underset{\Vert}{C}}CH_3$ |
| 73 | 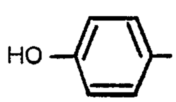 | 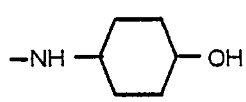 | H | 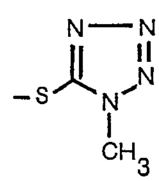 | 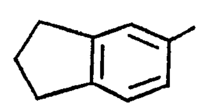 |
| 74 | 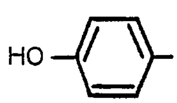 | 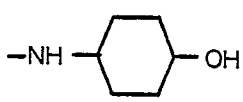 | H | $-O\underset{O}{\underset{\Vert}{C}}NH_2$ | H |
| 75 | 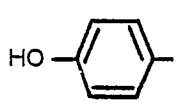 | 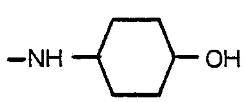 | H | 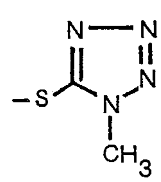 | H |

TABELLE 1 (Fortsetzung)

| | A | R | Y | D | E |
|---|---|---|---|---|---|
| 76 | 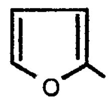 | 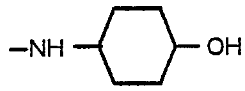 | H | 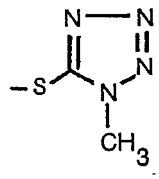 | H |
| 77 | 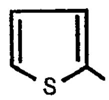 | 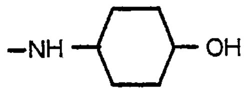 | H | 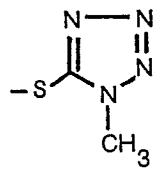 | H |
| 78 | 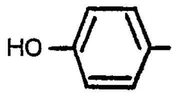 | $-NHCH_2CH_2SC_2H_5$ | H | 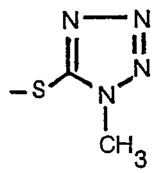 | H |
| 79 | 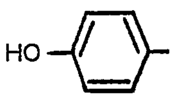 | $-NHCH_2CH_2SC_2H_5$ | H | 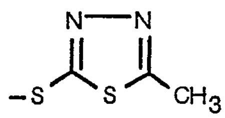 | H |
| 80 | 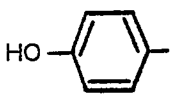 | $-NHCH_2CH_2SC_2H_5$ | H | 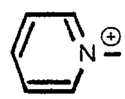 | – |
| 81 | 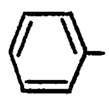 | $-NHCH_2CH_2SC_2H_5$ | H | 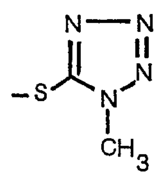 | H |
| 82 | 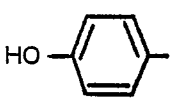 | $-NHCH_2CH_2OCH_3$ | H | 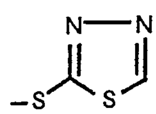 | H |
| 83 | 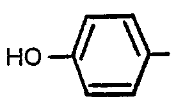 | $-NHCH_2CH_2OCH_3$ | H | 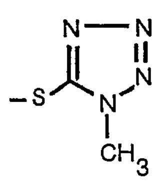 | H |

TABELLE 1 (Fortsetzung)

| | A | R | Y | D | E |
|---|---|---|---|---|---|
| 84 | 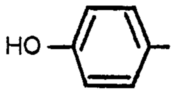 | $-NH(CH_2)_3OH$ | H | 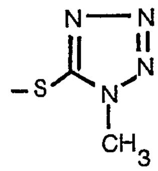 | H |
| 85 | 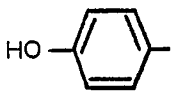 | $-NH(CH_2)_3OH$ | $-OCH_3$ | 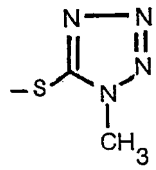 | H |
| 86 | 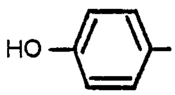 | $-NH(CH_2)_3OH$ | H | 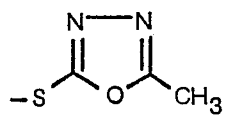 | H |
| 87 | 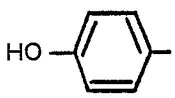 | $-NH(CH_2)_3OH$ | H | 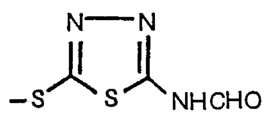 | H |
| 88 | 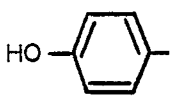 | $-NH(CH_2)_3OH$ | H | 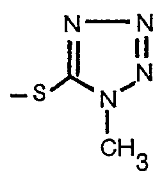 | $-CH_2OCOC(CH_3)_3$ |
| 89 | 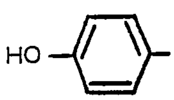 | $-NH(CH_2)_3OH$ | H | 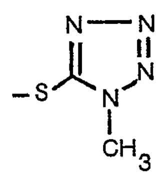 | 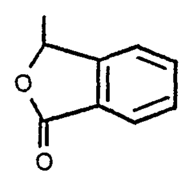 |
| 90 | 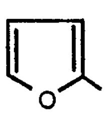 | $-NH(CH_2)_3OH$ | H | 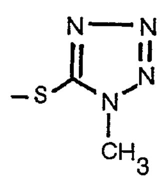 | H |
| 91 | 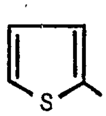 | $-NH(CH_2)_3OH$ | H | 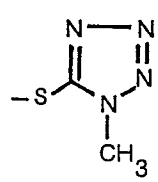 | H |

TABELLE 1 (Fortsetzung)

| | A | R | Y | D | E |
|---|---|---|---|---|---|
| 92 | 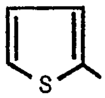 | $-NH(CH_2)_3OH$ | H | 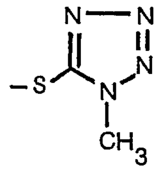 | H |
| 93 | 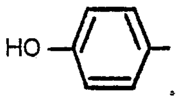 | $-NH(CH_2)_3OH$ | H | 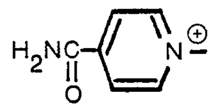 | – |
| 94 | 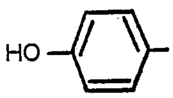 | $-NH(CH_2)_3OH$ | H | 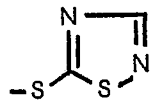 | H |
| 95 | 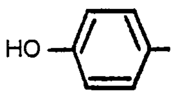 | $-NH(CH_2)_3OH$ | H | 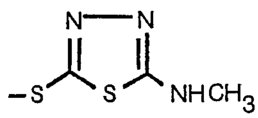 | H |
| 96 | 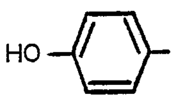 | $-NH(CH_2)_3OH$ | H | 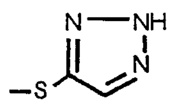 | H |
| 97 | 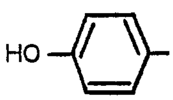 | $-NH(CH_2)_3SH$ | H | 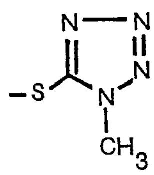 | H |
| 98 | 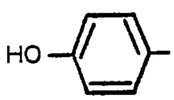 | $-NH(CH_2)_3SO_2NH_2$ | | 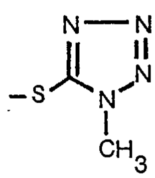 | H |
| 99 | 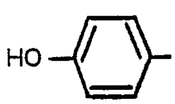 | $-NH(CH_2)_3SO_2NH_2$ | $-OCH_3$ | 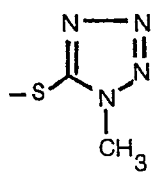 | H |
| 100 | 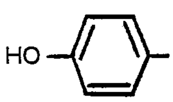 | $-NH(CH_2)_3SO_2NH_2$ | H | $OCOCH_3$ | H |

TABELLE 1 (Fortsetzung)

| | A | R | Y | D | E |
|---|---|---|---|---|---|
| 101 | 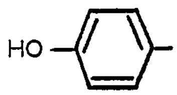 | $-NH(CH_2)_3SO_2NH_2$ | H | 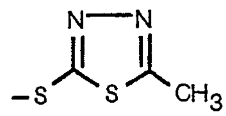 | H |
| 102 | 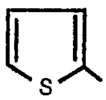 | $-NH(CH_2)_3SO_2NH_2$ | H | 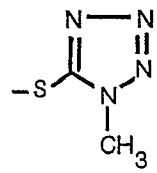 | H |
| 103 | 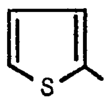 | $-NH(CH_2)_3SO_2NH_2$ | $-OCH_3$ | 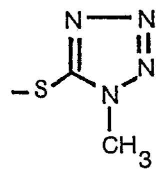 | H |
| 104 | 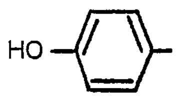 | $-NH(CH_2)_3CONH_2$ | H | 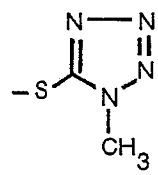 | H |
| 105 | 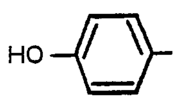 | $-NH(CH_2)_3CONH_2$ | H | $-OCOCH_3$ | H |
| 106 | 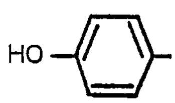 | $-NH(CH_2)_3CONH_2$ | $-OCH_3$ | 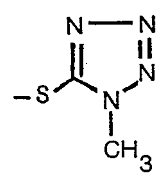 | H |
| 107 | 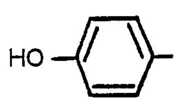 | $-NH(CH_2)_3NHCONH_2$ | H | 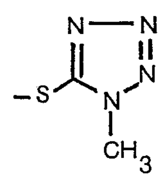 | H |
| 108 | 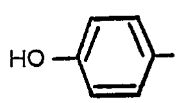 | $-NH(CH_2)_2NHCOCH_3$ | H | 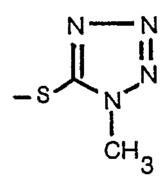 | H |
| 109 | 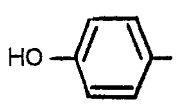 | $-NH(CH_2)_2NHCOCH_3$ | H | $-OCOCH_3$ | H |

TABELLE 1 (Fortsetzung)

| | A | R | Y | D | E |
|---|---|---|---|---|---|
| 110 | 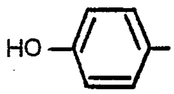 | $-NH(CH_2)_3NHSO_2CH_3$ | H | 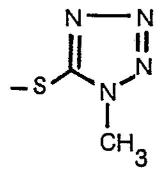 | H |
| 111 | 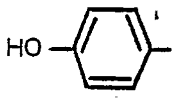 | $-NH(CH_2)_3NHSO_2CH_3$ | H | $-OCOCH_3$ | H |
| 112 | 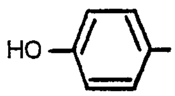 | $-NH(CH_2)_3OCOCH_3$ | H | 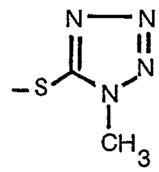 | H |
| 113 | 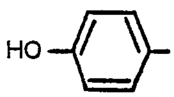 | $-NH(CH_2)_3SOCH_3$ | H | 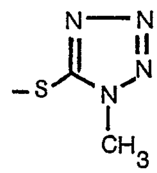 | H |
| 114 | 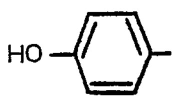 | 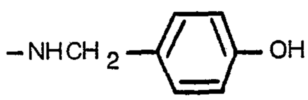 | H | H | H |
| 115 | 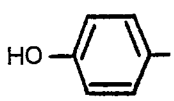 | 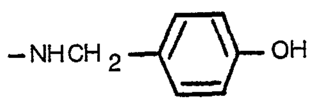 | H | $-OCOCH_3$ | H |
| 116 | 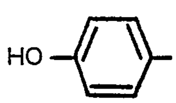 | 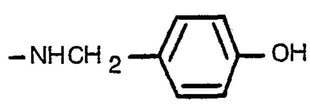 | $-OCH_3$ | 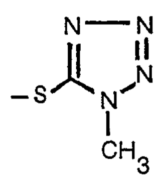 | H |
| 117 | 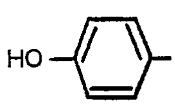 | 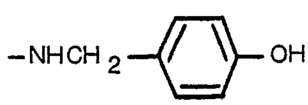 | H | 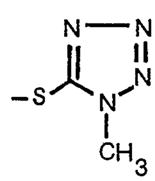 | H |
| 118 | 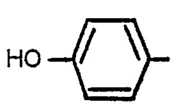 | 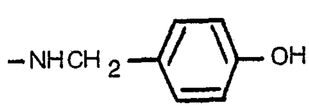 | H | 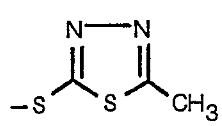 | H |

TABELLE 1 (Fortsetzung)

| | A | R | Y | D | E |
|---|---|---|---|---|---|
| 119 | 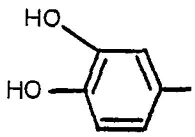 | 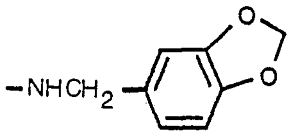 | H | 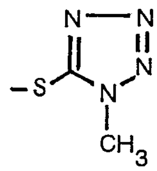 | H |
| 120 | 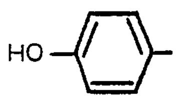 | 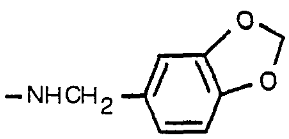 | H | 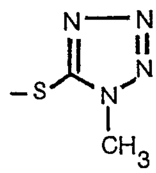 | H |
| 121 | 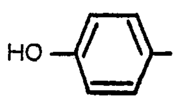 | 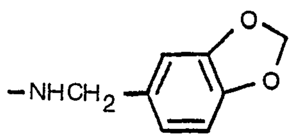 | H | $-OCOCH_3$ | H |
| 122 | 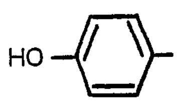 | 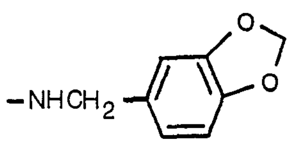 | H | 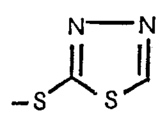 | H |
| 123 | 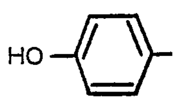 | 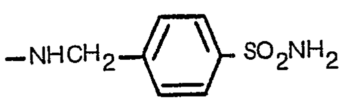 | H | 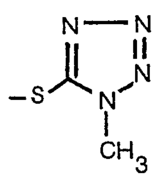 | H |
| 124 | 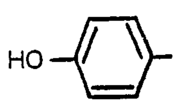 | 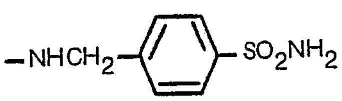 | H | $-OCOCH_3$ | H |
| 125 | 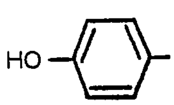 | 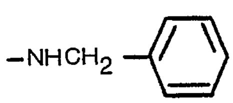 | H | $-OCOCH_3$ | H |
| 126 | 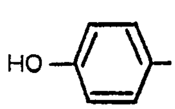 | 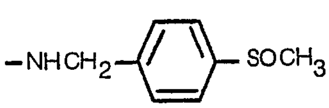 | H | 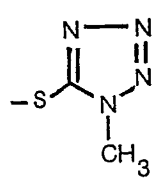 | H |

TABELLE 1 (Fortsetzung)

| | A | R | Y | D | E |
|---|---|---|---|---|---|
| 127 | 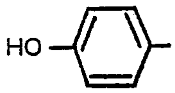 | 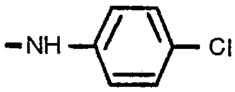 | H | H | H |
| 128 | 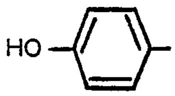 | 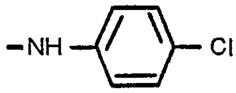 | H | $-OCOCH_3$ | H |
| 129 | 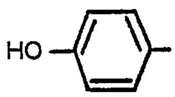 | 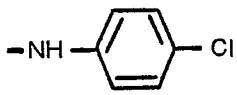 | H | 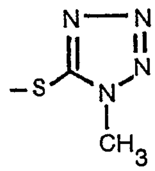 | H |
| 130 | 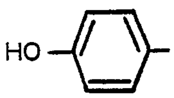 | 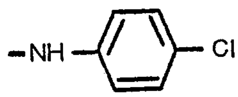 | H | 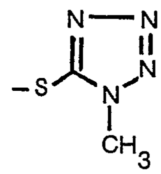 | $-CH_2OCOC(CH_3)_3$ |
| 131 | 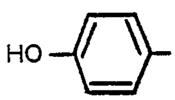 | 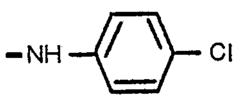 | H | 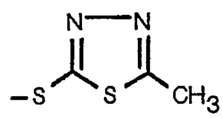 | H |
| 132 | 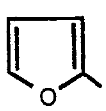 | 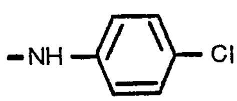 | H | 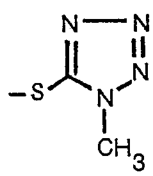 | H |
| 133 | 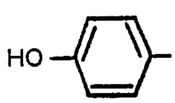 | 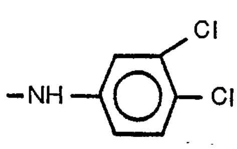 | H | 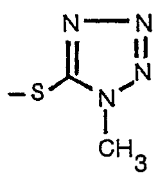 | H |
| 134 | 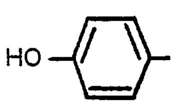 | 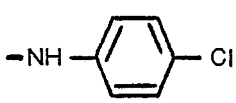 | H | 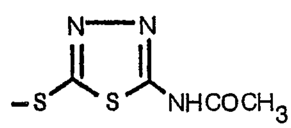 | H |

TABELLE 1 (Fortsetzung)

| | A | R | Y | D | E |
|---|---|---|---|---|---|
| 135 | 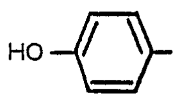 | 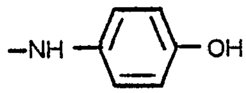 | H | 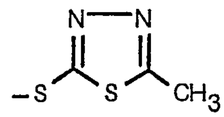 | H |
| 136 | 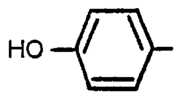 | 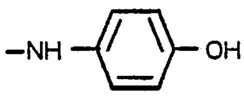 | H | 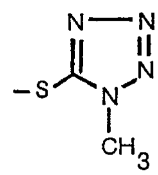 | H |
| 137 | 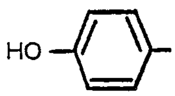 | 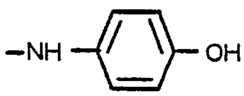 | $-OCH_3$ | 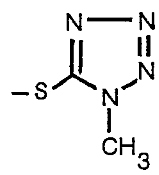 | H |
| 138 | 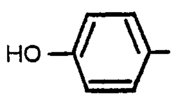 | 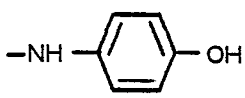 | H | 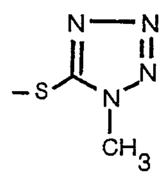 | $-CH_2OCO(CH_3)_3$ |
| 139 | 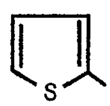 | 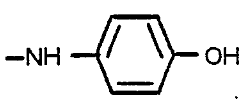 | H | 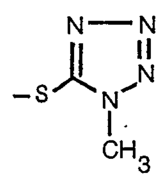 | H |
| 140 | 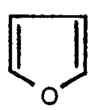 | 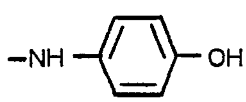 | H | 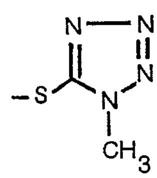 | H |
| 141 | 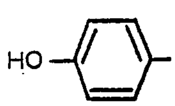 | 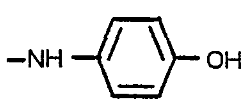 | H | $-OCONH_2$ | H |
| 142 | 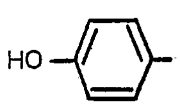 | 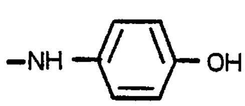 | H | 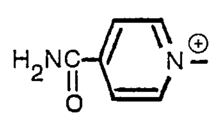 | – |

TABELLE 1 (Fortsetzung)

| | A | R | Y | D | E |
|---|---|---|---|---|---|
| 143 | 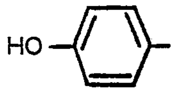 | 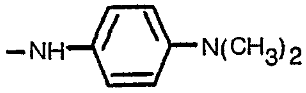 | H | 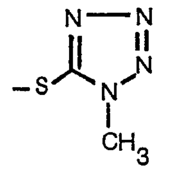 | H |
| 144 | 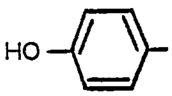 | 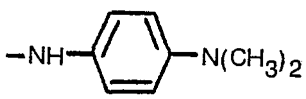 | H | 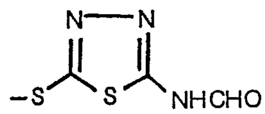 | H |
| 145 | 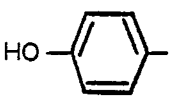 | 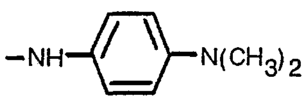 | H | $-OCOCH_3$ | H |
| 146 | 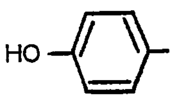 | 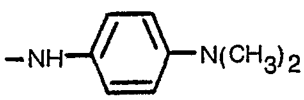 | H | 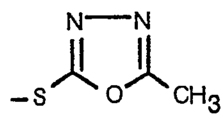 | H |
| 147 | 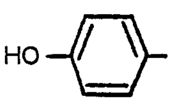 | 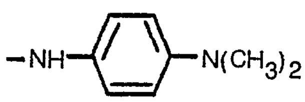 | H | 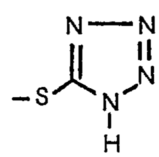 | H |
| 148 | 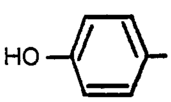 | 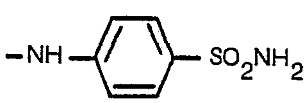 | H | H | H |
| 149 | 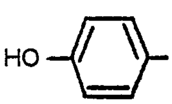 | 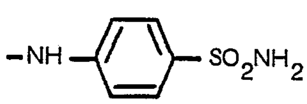 | H | $-OCOCH_3$ | H |
| 150 | 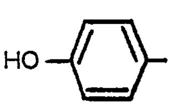 | 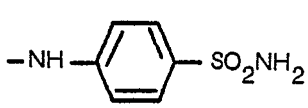 | H | 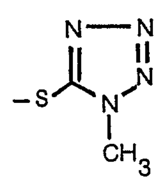 | H |
| 151 | 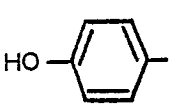 | 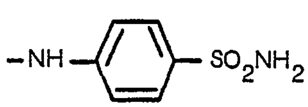 | H | 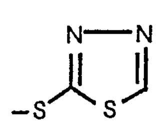 | H |

TABELLE 1 (Fortsetzung)

| | A | R | Y | D | E |
|---|---|---|---|---|---|
| 152 | 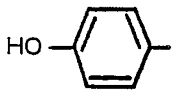 | 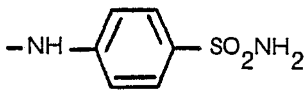 | H | 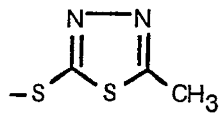 | H |
| 153 | 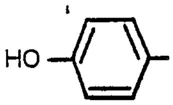 | 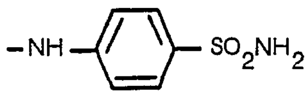 | H | $-OCONH_2$ | H |
| 154 | 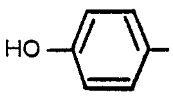 | 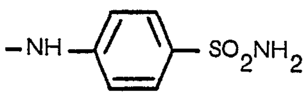 | H | 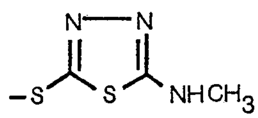 | H |
| 155 | 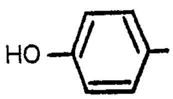 | 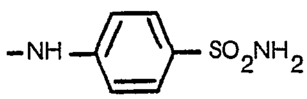 | H | 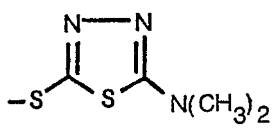 | H |
| 156 | 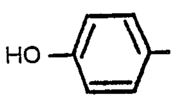 | 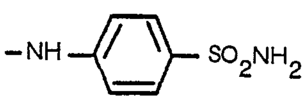 | H | 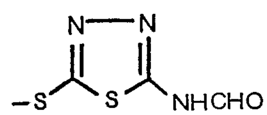 | H |
| 157 | 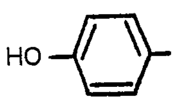 | 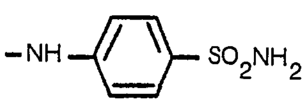 | H | 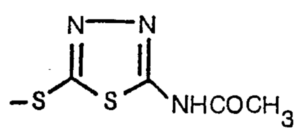 | H |
| 158 | 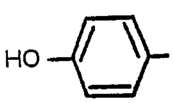 | 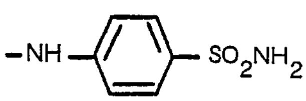 | H | 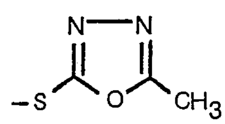 | H |
| 159 | 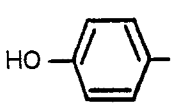 | 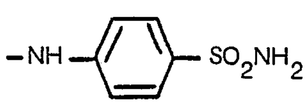 | H | 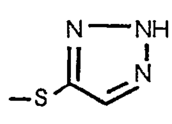 | H |
| 160 | 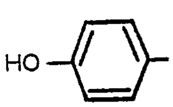 | 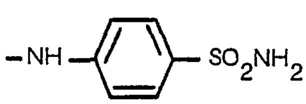 | H | 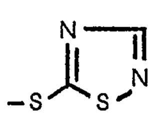 | H |

TABELLE 1 (Fortsetzung)

| | A | R | Y | D | E |
|---|---|---|---|---|---|
| 161 | 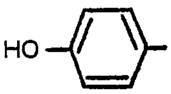 | 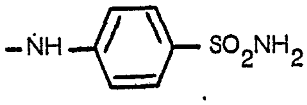 | H | 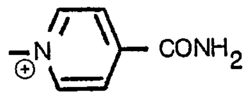 | – |
| 162 | 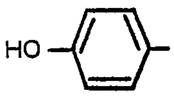 | 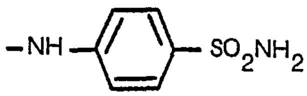 | H | 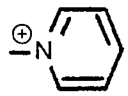 | – |
| 163 | 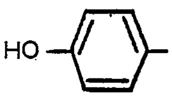 | 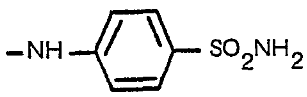 | H | 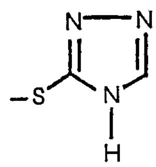 | H |
| 164 | 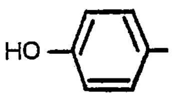 | 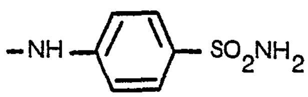 | H | 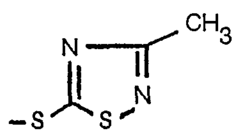 | H |
| 165 | 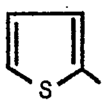 | 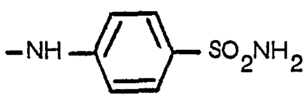 | H | $-OCOCH_3$ | H |
| 166 | 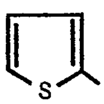 | 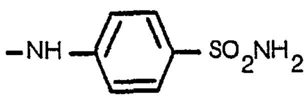 | H | 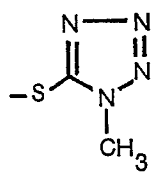 | H |
| 167 | 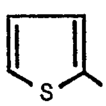 | 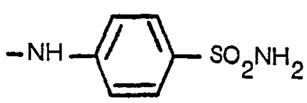 | $-OCH_3$ | 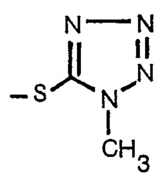 | H |
| 168 | 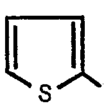 | 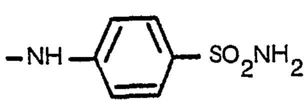 | H | $-OCONH_2$ | H |
| 169 | 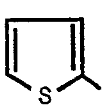 | 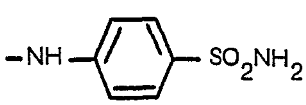 | $-OCH_3$ | $-OCONH_2$ | H |

TABELLE 1 (Fortsetzung)

| | A | R | Y | D | E |
|---|---|---|---|---|---|
| 170 | 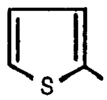 | 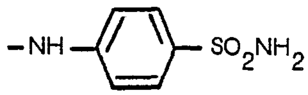 | H | 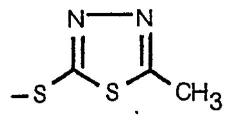 | H |
| 171 | 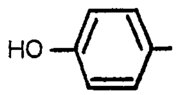 | 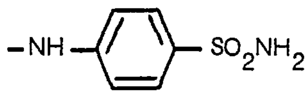 | $-OCH_3$ | $-OCOCH_3$ | H |
| 172 | 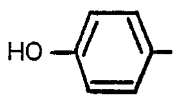 | 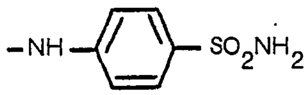 | $-OCH_3$ | $-OCONH_2$ | H |
| 173 | 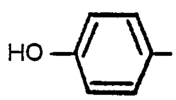 | 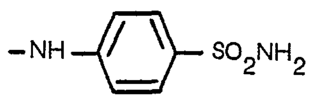 | $-OCH_3$ | 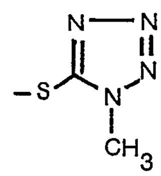 | H |
| 174 | 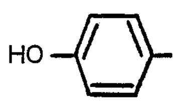 | 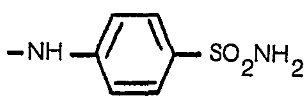 | H | 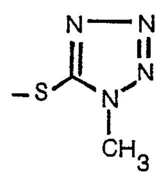 | $-CH_2OCOC(CH_3)_3$ |
| 175 | 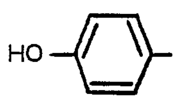 | 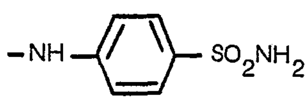 | H | 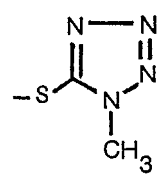 | 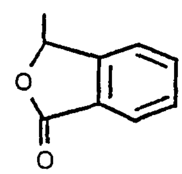 |
| 176 | 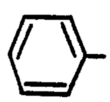 | 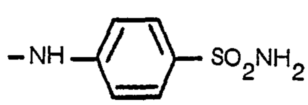 | H | 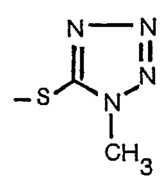 | H |
| 177 | 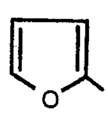 | 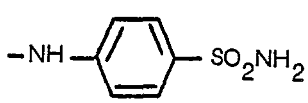 | H | 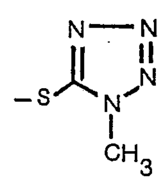 | H |
| 178 | 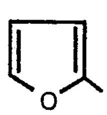 | 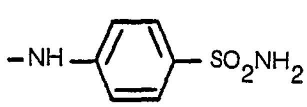 | H | $-OCOCH_3$ | H |

TABELLE 1 (Fortsetzung)

| | A | R | Y | D | E |
|---|---|---|---|---|---|
| 179 | 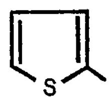 | 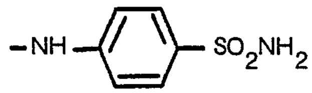 | H | 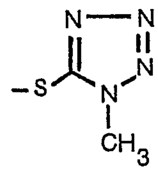 | H |
| 180 | 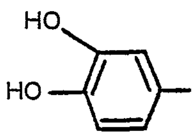 | 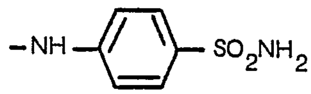 | H | 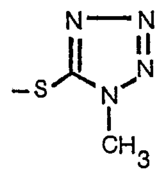 | H |
| 181 | 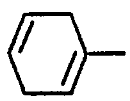 | 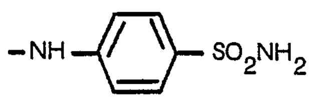 | H | 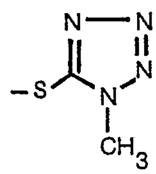 | H |
| 182 | 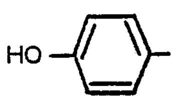 | 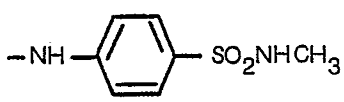 | H | 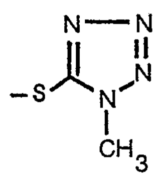 | H |
| 183 | 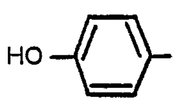 | 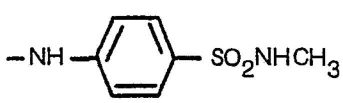 | H | $-OCOCH_3$ | H |
| 184 | 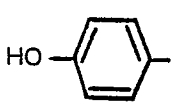 | 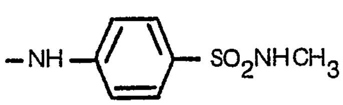 | $OCH_3$ | 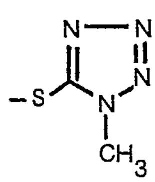 | H |
| 185 | 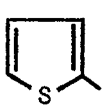 | 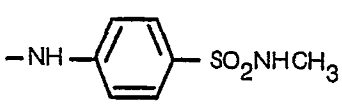 | H | 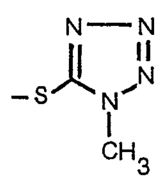 | H |
| 186 | 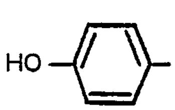 | 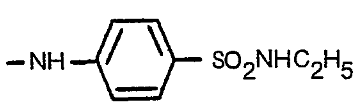 | H | 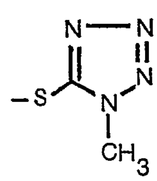 | H |

TABELLE 1 (Fortsetzung)

| | A | R | Y | D | E |
|---|---|---|---|---|---|
| 187 | 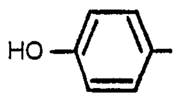 | 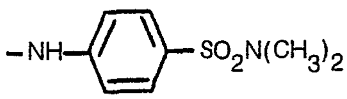 | H | 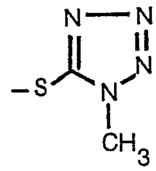 | H |
| 188 | 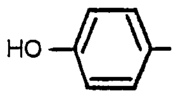 | 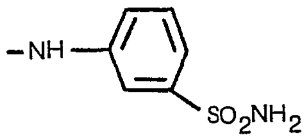 | H | 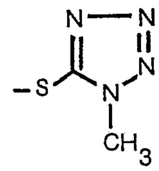 | H |
| 189 | 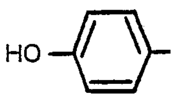 | 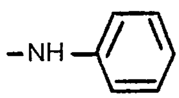 | H | $-OCOCH_3$ | H |
| 190 | 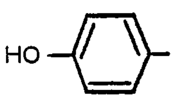 | 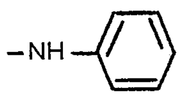 | H | 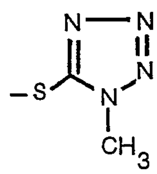 | H |
| 191 | 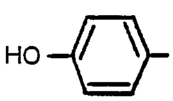 | 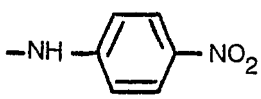 | H | 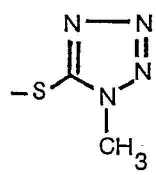 | H |
| 192 | 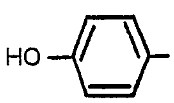 | 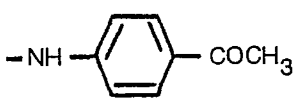 | H | 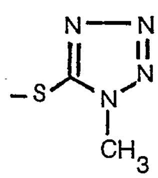 | H |
| 193 | 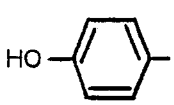 | 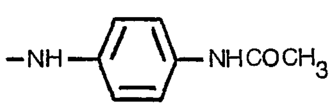 | H | $-OCOCH_3$ | H |
| 194 | 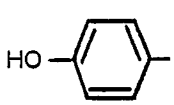 | 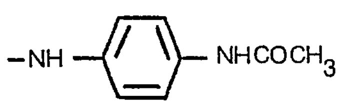 | H | 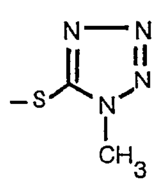 | H |

TABELLE 1 (Fortsetzung)

| | A | R | Y | D | E |
|---|---|---|---|---|---|
| 195 | 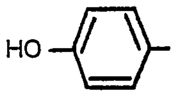 | 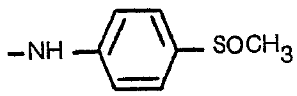 | H | 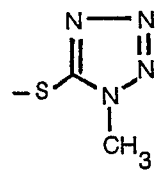 | H |
| 196 | 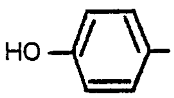 | 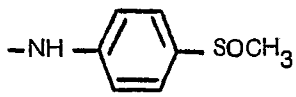 | H | $-OCOCH_3$ | H |
| 197 | 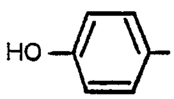 | 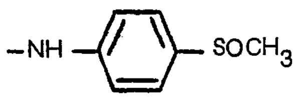 | H | 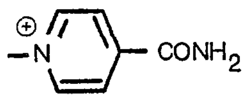 | – |
| 198 | 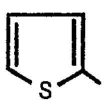 | 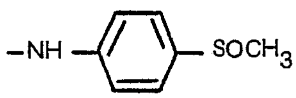 | H | 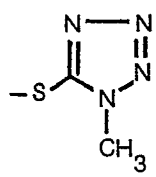 | H |
| 199 | 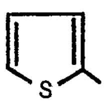 | 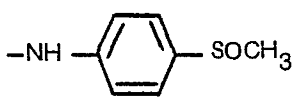 | $-OCH_3$ | 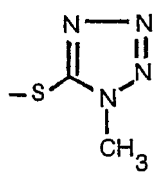 | H |
| 200 | 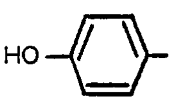 | 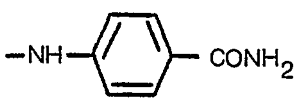 | H | 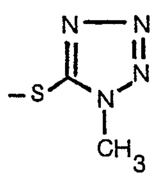 | H |
| 201 | 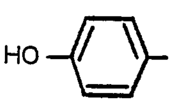 | 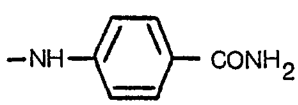 | $-OCH_3$ | 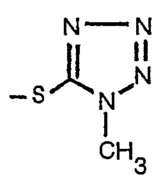 | H |
| 202 | 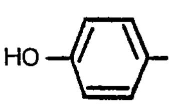 | 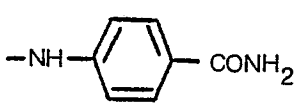 | H | $-OCOCH_3$ | H |
| 203 | 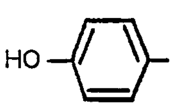 | 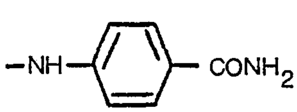 | H | 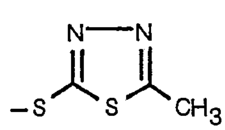 | H |

TABELLE 1 (Fortsetzung)

| | A | R | Y | D | E |
|---|---|---|---|---|---|
| 204 | 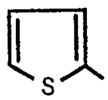 | 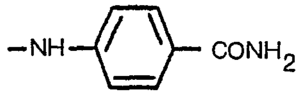 | H | 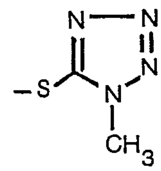 | H |
| 205 | 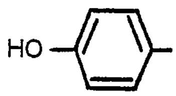 | 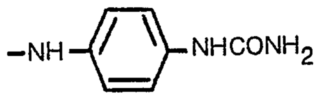 | H | 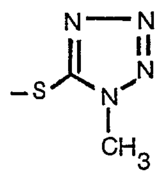 | H |
| 206 | 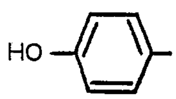 | 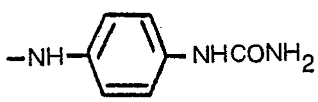 | H | $-OCOCH_3$ | H |
| 207 | 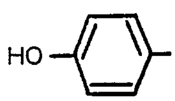 | 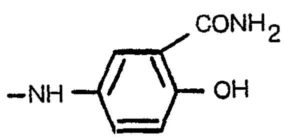 | H | 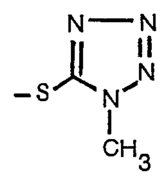 | H |
| 208 | 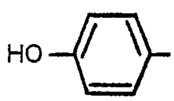 | 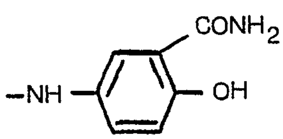 | H | $-OCOCH_3$ | H |
| 209 | 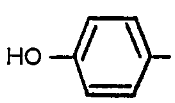 | 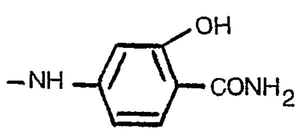 | H | $-OCOCH_3$ | H |
| 210 | 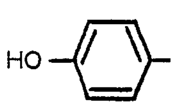 | 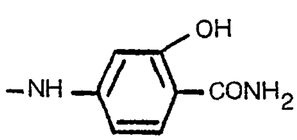 | H | 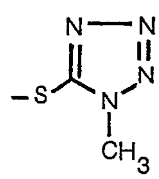 | H |
| 211 | 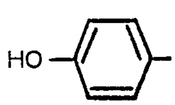 | 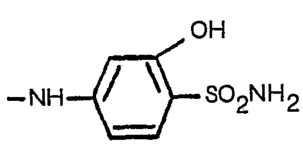 | H | 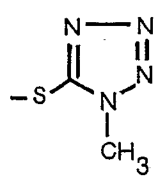 | H |
| 212 | 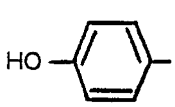 | 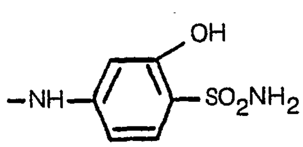 | H | $-OCOCH_3$ | H |

TABELLE 1 (Fortsetzung)

| | A | R | Y | D | E |
|---|---|---|---|---|---|
| 213 | 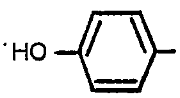 | $-NHCOCH_3$ | H | $-OCOCH_3$ | H |
| 214 | 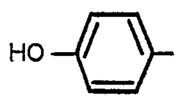 | $-NHCOCH_3$ | H | 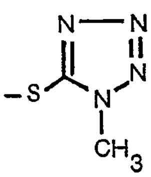 | H |
| 215 | 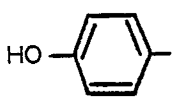 | $-NHCOCH_3$ | H | 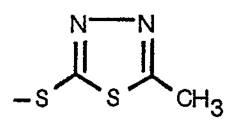 | H |
| 216 | 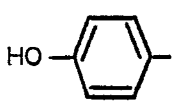 | 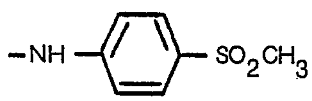 | H | 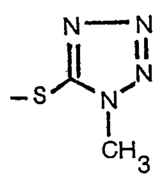 | |

Die Wirksamkeit der erfindungsgemäßen Cephalosporine läßt sich durch folgende Untersuchungen beispielhaft demonstrieren:

1. In vitro-Versuche

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:

80; 40; 20; 10; 5; 2,5; 1,25; 0,6; 0,3; 0.08 und 0,02 μg/ml. Es wurde ein Nährboden folgender Zusammenseztung benutzt: 10 g Pepton, 8 g Fleischextrakt-Oxoid, 3 g Natriumchlorid, 2 g sek. Natriumphosphat werden mit dest. Wasser auf 100 ml aufgefüllt (pH 7,2—7,4). Lediglich bei der Testung gegen Streptokokken wurde 1% Glucose hinzugefügt. Das Alter der Primärkulturen betrug etwa 20 Stunden. Die Einstellung der Keimsuspension erfolgte am Photometer (nach "Eppendorf") (Reagenzglas-Durchmesser 14 mm, Filter 546nm) anhand der Trübung einer Bariumsulfat-Vergleichssuspension, die durch eine Bariumsulfat-Aufschwemmung erzeugt wurde, welche durch die Zugabe von 3,0 ml 1%ige Bariumchloridlösung in 97 ml 1%ige Schwefelsäure entstand. Nach der Einstellung wurden Streptococcus Aronson im Verhältnis 1:15 und die übrigen Testkeime im Verhältnis 1:1500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopishc vorgenommen, wobei die jeweilige Grenzkonzentration (=niedrigste noch bakteriostatisch wirksame Konzentration) ermittelt wurde.

Als Testorganismen werden benützt:

Staphylococcus aureus SG 511, St. aureus E 88 (β-Lactamase-Träger), Escherichia coli ATCC 11 775, Pseudomonas aeruginosa Hamburgensis und Pseudomonas aeruginosa Walter, Serratia marcescens ATCC 13 880, Klebsiella pneumoniae ATCC 10 031 und 272, Proteus mirabilis Hamburgensis, Proteus rettgeri Eb. cloaceae ATCC 13 047, E. coli R+TEM (β-Lactamase-Träger).

In der folgenden Tabelle 1 sind die ermittelten minimalen Hemmkonzentrationen (MHK) für typische Vertreter der erfindungsgemäßen Verbindungen aufgeführt:

Natriumsalze von Verbindungen der allgemeinen Formel I mit den Bedeutungen von A, R, Y und D:

| A | R | Y | D | Verbindung |
|---|---|---|---|---|
| 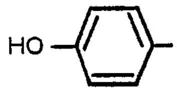 | 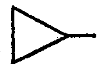 | H | 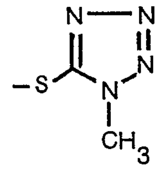 | A |
| 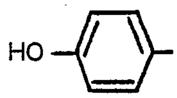 | $-NHC_3H_7$ | H | 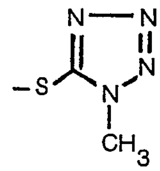 | B |
| 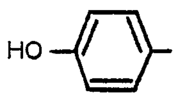 | 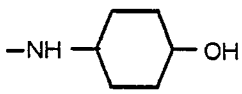 | H | 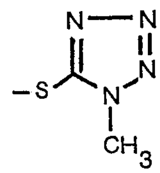 | C |
| 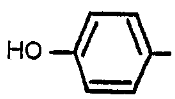 | 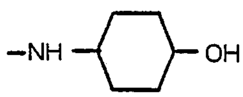 | H | 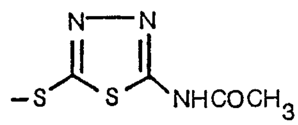 | D |
| 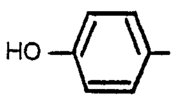 | $NH(CH_2)_3OH$ | H | 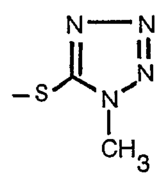 | E |
| 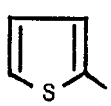 (D, L) | 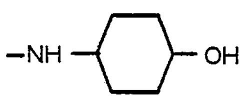 | H | 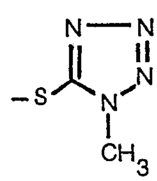 | F |
| 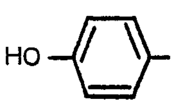 | 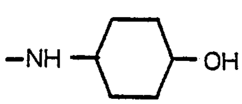 | H | 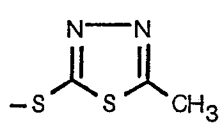 | G |
| 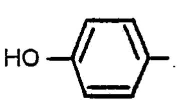 | 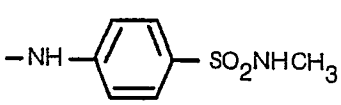 | H | $-OCOCH_3$ | H |

| A | R | Y | D | Verbindung |
|---|---|---|---|---|
| 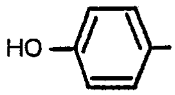 | 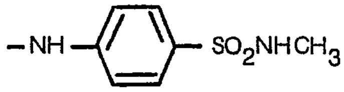 | H | 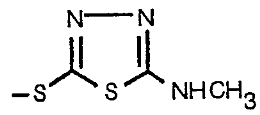 | J |
| 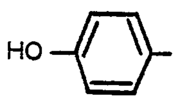 | 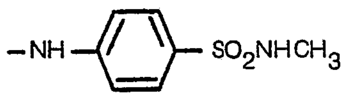 | H | 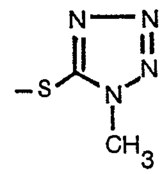 | K |
| 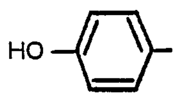 | 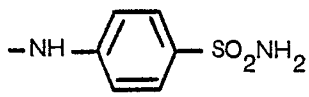 | H | 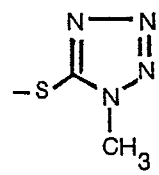 | L |
| 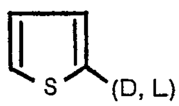 | 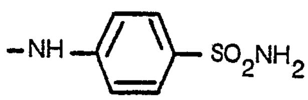 | H | 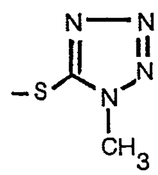 | M |
| 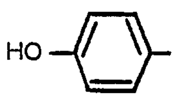 | 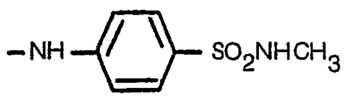 | H | 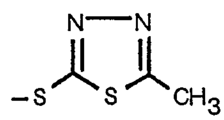 | N |

Als Vergleichsverbindungen werden zwei typische, sich im Handel befindliche Cephalosporine herangezogen.

TABELLE 1

MHK-Werte verschiedener Cephalosporine (in $\mu$g/ml)

| Verbindung | S.aureus SG 511 | S.aureus E 88 | E.coli ATCC 11775 | Pseud. aerug. Hbg. | Pseud. aerug. Walter | Klebs. pneum. ATCC 10031 | Klebs. pneum. 272 | Prot. mir. Hbg, | Prot. rettg. | Eb. cloacae | E.coli R+TEM | Serr. marcesc. ATCC 13880 |
|---|---|---|---|---|---|---|---|---|---|---|---|---|
| Cefuroxim | 1 | 16 | 8 | >100 | >100 | 2 | 4 | 0,5 | 2 | 32 | 4 | 8 |
| Cephazolin | 0,06 | 8 | 4 | >100 | >100 | 1 | 2 | 4 | >100 | >100 | 4 | >100 |
| A | 0,05 | 8 | 0,5 | 8 | 8 | 1 | 1 | 0,5 | 2 | 2 | 8 | 2 |
| B | 1 | 8 | 0,5 | 4 | 8 | 0,5 | 0,5 | 0,25 | 1 | 1 | 8 | 1 |
| C | 1 | 8 | 0,13 | 4 | 4 | 0,5 | 0,5 | 0,13 | 1 | 0,5 | 8 | 0,5 |
| D | 1 | 8 | 1 | 8 | 8 | 2 | 2 | 1 | 8 | 8 | 16 | 4 |
| E | 1 | 8 | 0,25 | 4 | 8 | 0,5 | 1 | 0,13 | 1 | 0,5 | 16 | 1 |
| F | 1 | 8 | 0,5 | 16 | 16 | 2 | 2 | 0,5 | 2 | 4 | 16 | 2 |
| G | 1 | 16 | 0,5 | 16 | 16 | 2 | 2 | 0,5 | 1 | 2 | 16 | 2 |
| H | 1 | 16 | 0,13 | 4 | 4 | 0,5 | 0,5 | 0,06 | 0,5 | 2 | 16 | 0,5 |
| J | 1 | 16 | 0,5 | 8 | 8 | 0,5 | 0,25 | 0,25 | 1 | 1 | 8 | 0,5 |
| K | 0,5 | 4 | <0,03 | 4 | 2 | 0,06 | 0,13 | 0,06 | 0,25 | 0,5 | 2 | 0,13 |
| L | 0,5 | 4 | <0,03 | 2 | 2 | 0,06 | 0,06 | <0,06 | 0,13 | 0,5 | 2 | 0,06 |
| M | 0,5 | 4 | 0,13 | 4 | 4 | 0,25 | 0,25 | 0,13 | 0,5 | 0,5 | 4 | 0,25 |
| N | 1 | 8 | 0,25 | 4 | 4 | 0,25 | 0,5 | 0,5 | 0,5 | 1 | 4 | 0,25 |

Wie aus der vorstehenden Tabelle ersichtlich wird, sind die genannten Verbindungen den Vergleichssubstanzen in ihrer Wirksamkeit gegenüber typischen gramnegativen Hospitalkeimen bei Erhalt der Wirkung gegen grampositive Keime deutlich überlegen. Hervorzuheben ist die gute Wirksamkeit gegen Pseudomonasstämme.

Die akute Toxizität wurde durch perorale und subcutane Applikation der Verbindungen der Tabelle 1 an weiße Laboratoriumsmäuse in steigenden Dosen bestimmt.

Die $LD_{50}$ ist die Dosis, nach deren Applikation 50% der Tiere innerhalb von 8 Tagen sterben. Sämtliche Substanzen zeigten bei oraler Gabe eine $LD_{50}$ von über 4 g/kg, bei subcutaner gabe eine $LD_{50}$ von über 3 g/kg, d.h. bei 3 g/kg starben keine Tiere, sie sind damit für die Praxis untoxisch.

Eine Reihe der erfindungsgemäßen Verbindungen wurde in vivo bei experimentellen Infektionen an Mäusen untersucht. Man verwendete als pathogene Bakterien E.coli ATCC 11775 und Pseudomonas aeruginosa Walter. Es wurde eine intraperitoneale Infektion mit 0,2 ml einer Bakteriensuspension (mit 5% Mucin) gesetzt. Dies entspricht etwa $1,4 \times 10^6$ Keime E.coli bzw. $1,3 \times 10^6$ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Cephalosporine subcutan zur Bestimmung der $ED_{50}$ (Dosis bei der 50% der Tiere überleben) behandelt. Bei der E.coli-Infektion wurde am ersten Tag dreimal therapiert (1,4 und 7 h post-Infektionem) und am 2. Tag 2 mal. Bei der Pseudomonas-Infektion wurde am ersten Tag 6x (1,3,5,7,9,11 h post-Infektionem) und an den darauffolgenden 2 Tagen zweimal täglich behandelt.

Der Beobachtungszeitraum betrug in beiden Fällen 7 Tage. Die Ergebnisse dieser Teste mit repräsentativen Vertretern der erfindungsgemäßen Cephalosporine sind in der Tabelle 2 dargestellt.

TABELLE 2

In vivo Aktivität bei Mäusen

a) E.coli-Infektion (s.c. Applikation):

| Verbindung | $ED_{50}$ (mg*/kg) |
|---|---|
| A | 1,6 |
| B | 1,1 |
| C | 0,4 |
| E | 0,8 |
| K | 0,3 |
| L | 0,15 |
| Cefuroxim | 37 |

*pro Dosis

b) Pseudomonas (s.c. Applikation):

| Verbindung | $ED_{50}$ (mg*/kg) |
|---|---|
| A | 15,2 |
| B | 12,4 |
| C | 4,2 |
| E | 11,2 |
| K | 3,6 |
| L | 2,1 |
| Cefuroxim | bei 200 mg/kg alle Tiere gestorben |

*pro Dosis

Auch hierbei zeigt sich wieder eine signifikante Überlegenheit der erfindungsgemäßen Substanzen gegenüber der Vergleichssubstanz.

Es ist eine weitere Aufgabe der Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10—200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern:

## Beispiel 1

*Darstellung der Ureidocarbonsäuren*

a) D-$\alpha$-(2-Cyclopropyl-4-hydroxy-5-pyrimidinyl)-ureido-p-hydroxyphenylessigsäure

1,8 g 5-Amino-2-cyclopropyl-4-hydroxy-pyrimidin (0,012 Mol) werden in 80 ml absolutem Tetrahydrofuran gelöst und mit 1,65 ml Triäthylamin versetzt. Diese Lösung wird bei 0°C zu einer Lösung von 1,20 g Phosgen in 25 ml Tetrahydrofuran getropft. Man rührt unter Eiskühlung etwa 10 Minuten nach. Anschließend wird Stickstoff durch die Lösung geblasen, um nicht umgesetztes Phosgen zu entfernen.

Eine Suspension von 2,0 g (0,012 Mol) D-$\alpha$-Amino-p-hydroxyphenylessigsäure in 50 ml Tetrahydrofuran und 20 ml Wasser wird durch Zugabe von 12 ml 1n Natronlauge unter Kühlen und Rühren in Lösung gebracht. Zu dieser Lösung wird unter Eiskühlung die oben hergestellte Suspension getropft, wobei der pH-Wert mit n-Natronlauge auf 8,0—8,5 gehalten wird. Es wird eine Stunde bei 5°C und 2 Stunden bei Raumtemperatur nachgerührt. Dann wird das Tetrahydrofuran im Vakuum entfernt und die zurückbleibende wäßrige Lösung zweimal mit Essigsäureäthylester bei pH 8,0—8,5 ausgeschüttelt. Die wäßrige Phase wird dann mit 200 ml Essigester überschichtet und unter Kühlen und Rühren mit verdünnter Salzsäure auf pH 1,5 eingestellt. Die organische Schicht wird abgetrennt und die wäßrige Phase noch einmal mit 50 ml Essigester ausgeschüttelt; die organischen Phasen werden vereinigt, über Natriumsulfat getrocknet und das Lösemittel im Vakuum entfernt.

Ausbeute: 3,0 g (73%)

IR-Spektrum: 3320 (breit), 1650, 1550 $cm^{-1}$;

NMR-Spektrum ($CDCl_3 + D_2O$) Signale bei ppm: 1,15 (m, 4H) 1,9 (m, 1H), 5,45 (s, 1H), 6,9 (d, 2H), 7,45 (d, 2H), 8,6 (s, 1H).

Nach dieser Methode wurden die Ureidocarbonsäuren der folgenden Tabelle synthetisiert:

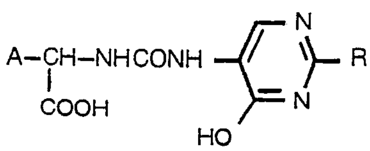

| | A | R | Ausbeute (%) | IR-Spektrum $cm^{-1}$ | NMR-Spektrum Signale bei ppm (DMSO + $CD_3OD$) |
|---|---|---|---|---|---|
| b) | 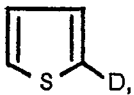 D,L | 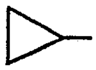 | 64,5 | 3360 (breit) 1670, 1560 | 1,10(m,4H), 1,95 (m,1H), 5,5(s,1H), 7,0(m,2H), 7,4(d,1H), 8,55(s,1H) |
| c) | 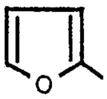 D,L | 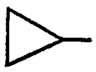 | 62 | 3350 (breit) 1650 1570 | 1,05(m,4H), 1,90 (m,1H), 5,45(s,1H), 6,4(m,2H), 7,65 (s,1H), 8,50(s,1H) |
| d) | 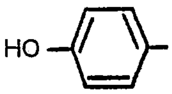 | $-NHC_3H_7$ | 71 | 3300 (breit) 1680, 1620 1530 | 0,9(t,3H), 1,5(m,2H), 3,2(m,2H), 5,05(s,1H), 6,7(d,2H), 7,15(d,2H), 8,0(s,1H) |
| e) | 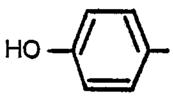 | $-NHCH(CH_3)_2$ | 66 | 3350, 1650, 1560 | 1,15(d,6H), 3,9(m,1H), 5,1(s,1H), 6,8(d,2H), 7,3(d,2H), 8,05(s,1H) |
| f) | 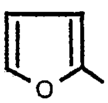 D,L | $-NHC_3H_7$ | 49 | 3350, 1670, 1570 | 1,0(t,3H), 1,6(m,2H), 3,2(m,2H), 5,4(s,1H), 6,4(m,2H), 7,6(s,1H), 8,05(s,1H) |
| g) | 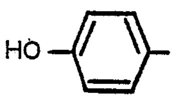 | $-NHCH_2CH{=}CH_2$ | 54 | 3350, 1665, 1575 | 3,75(breit,2H), 4,65 (breit,2H), 5,05 (s,1H), 5,4(m,1H), 6,80(2H), 7,3(2H), 8,1(1H) |
| h) | 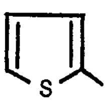 D,L | $-NHC_3H_7$ | 52 | 3370, 1675, 1550 | 0,9(t,3H), 1,55(m,2H), 3,2(m,2H), 5,5(s,1H), 7,0(m2H), 7,4(d,1H), 8,0(s,1H) |
| i) | 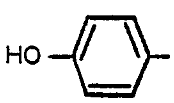 | $-N(CH_3)_2$ | 72 | 3350, 1670, 1560 | 3,0(s,6H), 5,1(s,1H), 6,75(d,2H), 7,2(d,2H), 8,0(s,1H) |
| j) | 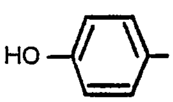 | $-NHCH_2CH(CH_3)_2$ | 63,5 | 3300, 1670, 1565 | 0,85(d,6H), 1,8(m,1H), 3,1(m,2H) 5,0(s,1H), 6,75(d,2H), 7,2(d,2H), 8,0(s,1H) |

| | A | R | Ausbeute (%) | IR-Spektrum $cm^{-1}$ | NMR-Spektrum Signale bei ppm (DMSO + $CD_3OD$) |
|---|---|---|---|---|---|
| k) | 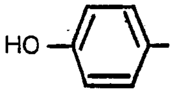 | $-NHCH_2CH=CHCH_3$ | 66 | 3370 (breit) 1680 1565 | 1,65(d,3H), 3,75(breit), 2H), 5,1(s,1H), 5,4(m, 2H), 6,8(d,2H), 7,3 (d,2H), 8,05(s,1H) |
| l) | 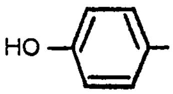 | $-NHCH_2C=CH_2$ <br> $CH_3$ | 71 | 3350, 1670, 1560 | 1,75(s,3H), 3,75(s,2H), 4,7(breit,2H), 5,1(s,1H), 6,85(2H), 7,3(2H), 8,1 (1H) |
| m) | 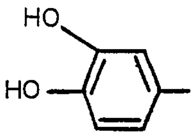 | $-NHCH_2C=CH_2$ <br> $CH_3$ | 44 | 3380, 1650, 1550 | 1,70(s,3H), 3,80(s,2H), 4,75(breit, 2H), 5,25 (s,1H), 6,9(m,2H), 7,3(1H), 8,05(1H) |
| n) | 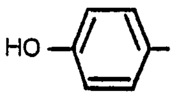 | 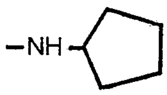 | 80 | 3800, 2950, 1660, 1520 | 1,6(m,8H), 4,1(m,1H), 5,2(s,1H), 6,85(d,2H), 7,3(d,2H), 8,1(s,1H) |
| o) | 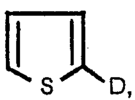 D,L | 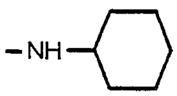 | 72 | 3350 (breit) 1660, 1540 | 1,65(m,10H), 4,05(m,1H), 5,45(s,1H), 7.0(m,2H), 7,4(d,1H), 8,05(s,1H) |
| p) | 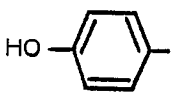 | 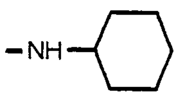 | 67,5 | 3370 (breit) 1665, 1545 | 1,70(m,10H), 4,0(m,1H), 5,1(s,1H), 6,8(d,2H), 7,35(d,2H), 8,05(s,1H) |
| q) | 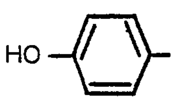 | 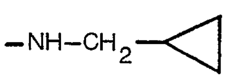 | 58 | 3350 (breit) 1670, 1610, 1540, 1520 | ($CDCl_3/CD_3OD$) 0,3(m,2H), 0,6(m,2H), 1,1(m,1H), 3,1(d,2H), 5,25(s,1H), 6,75(d,2H), 7,2(d,2H), 8,0(s,1H) |
| r) | 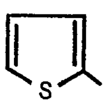 | 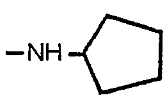 | 71 | 3360 (breit) 1665, 1660, 1540 | 1,65(m,8H), 4,05(m,1H), 5,5(s,1H), 7,0(m,2H), 7,4(d,1H), 8,05(s,1H) |
| s) | 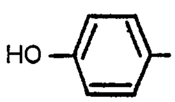 | 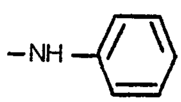 | 66,5 | 3350, 1660, 1600, 1535 | 5,15(s,1H), 6,8(d,2H), 7,35(m,7H), 8,35(s,1H) |

t) D-α-4-Hydroxy-2-(4′-hydroxycyclohexylamino-5-pyrimidinyl)-ureido-p-hydroxy-phenylessigsäure

2,24 g (0,01 Mol) 5-Amino-4-hydroxy-2-(4′-hydroxycyclohexylamino)-pyrimidin werden zusammen mit 7,5 g Trimethylsilyldiäthylamin 10 Minuten auf 80°C erhitzt. Anschließend wird im Vakuum zur Trockne eingeengt und das Festprodukt unter Stickstoff in 60 ml trockenem Tetrahydrofuran gelöst. Diese Lösung wird unter Eiskühlung zu 1,05 g Phosgen, gelöst in 50 ml Tetrahydrofuran getropft. Man rührt 5 Minuten bei Raumtemperatur nach und engt im Vakuum etwa auf das halbe Volumen ein. Die weitere Umsetzung mit 1,7 g p-Hydroxyphenylglycin erfolgt analog Beispiel 1a). Es wird jedoch wie folgt aufgearbeitet: Die wäßrige Phase wird bei pH 7,0 2 mal mit Essigester ausgeschüttelt und dann unter Eiskühlung mit 2 n Salzsäure auf pH 3,0 angesäuert. Das ausgefallene Produkt wird abgesaugt und getrocknet.

Ausbeute: 1,84 g (44%),

IR-Spektrum: 3400 (breit), 1670, 1550 $cm^{-1}$;

NMR-Spektrum ($DMSO + CD_3OD$) Signale bei ppm: 1,8 (m, 8H), 3,8 (m, 2H), 5,1 (s, 1H), 6,8 (d, 2H), 7,3 (d, 2H), 8,0 (s, 1H).

Analog dazu wurden folgende Ureidocarbonsäuren synthetisiert:

| | A | R | Ausbeute (%) | IR-Spektrum $cm^{-1}$ | NMR-Spektrum Signale bei ppm ($DMSO + CD_3OD$) |
|---|---|---|---|---|---|
| u) | 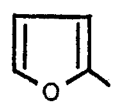 D,L | 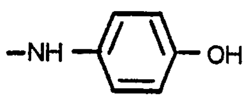 | 41 | 3380 (breit) 1660 1560 | 1,75(m,8H), 3,6–4–1 (m,1+1H) 5,5(s,1H) 6,4(m,2H), 7,65(s,1H), 8,0(s,1H) |
| v) | 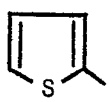 D,L | 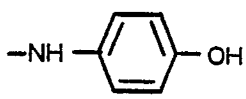 | 47 | 3360 (breit) 1670 1570 | 1,80(m,8H), 3,55–4,1 (m,1+1H), 5,45(s,1H), 7,0(m,2H), 7,4(d,1H), 8,05(s,1H) |
| w) | 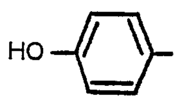 | $-NH-CH_2CH_2SC_2H_5$ | 61 | 3350 (breit) 1660, 1540 | 1,3(t,3H), 2,7(m,4H), 3,4(m,2H), 5,1(s,1H), 6,8(d,2H), 7,2(d,2H), 8,1(s,1H) |
| x) | 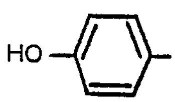 | $-NHCH_2CH_2OCH_3$ | 56 | 3320, 1670, 1550, 1515 | 3,0–3,6(4H,m), 3,5 (3H,s), 5,2(s,1H), 6,8(d,2H), 7,3(d,2H), 8,15(s,1H) |
| y) | 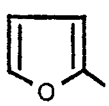 D,L | $-NH(CH_2)_3OH$ | 42 | 3350, 1660, 1550 | 1,85(m,2H), 3,3(m,2H), 3,6(m,2H), 5,4(s,1H), 6,45(m,2H), 7,6(s,1H), 8,05(s,1H) |
| z) | 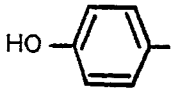 | $-NH(CH_2)_3OH$ | 51 | 3340, 1650, 1570 | 1,85(m,2H), 3,3(m,2H), 3,65(m,2H), 5,15(s,1H), 6,8(d,2H), 7,3(d,2H), 8,05(s,1H) |
| aa) | 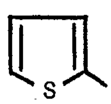 D,L | $-NH(CH_2)_3OH$ | 44 | 3320, 1640, 1530 | 1,90(m,2H), 3,3(m,2H), 3,65(m,2H), 5,5(s,1H), 7,0(m,2H), 7,4(d,1H), 8,05(s,1H) |

| | A | R | Ausbeute (%) | IR-Spektrum $cm^{-1}$ | NMR-Spektrum Signale bei ppm ($DMSO + CD_3OD$) |
|---|---|---|---|---|---|
| ab) | 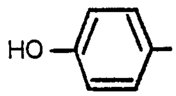 | $-NH(CH_2)_3SO_2NH_2$ | 56 | 3340, 1635, 1540 | 2,0(m,2H), 2,7(m,2H), 3,40(m,2H), 5,15(s,1H), 6,8(d,2H), 7,3(d,2H), 8,10(s,1H) |
| ac) | 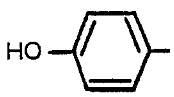 | $-NH(CH_2)_3CONH_2$ | 51,5 | 3800, 1630, 1580, 1530 | 1,9(t,2H), 2,5(m,2H), 3,45(m,2H), 5,15(s,1H), 6,8(d,2H), 7,35(d,2H), 8,10(s,1H) |
| ad) | 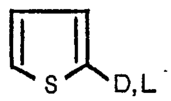 | $-NH(CH_2)_3SO_2NH_2$ | 54 | 3330, 1640, 1545 | 1,95(t,2H), 2,8(m,2H), 3,35(m,2H), 5,45(s, breit, 1H),7,05(m,2H), 7,4(m,2H), 7,4(d,1H), 8,05(s,1H) |
| ae) | 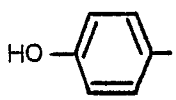 | $-NH(CH_2)_2NHCOCH_3$ | 38 | 3320, 1635, 1575, 1525 | 2,2(s,3H), 2,7(m,2H), 3,3(m,2H), 5,10(s,1H), 6,85(d,2H), 7,35(d,2H), 8,10(s,1H) |
| af) | 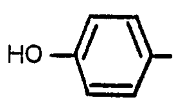 | 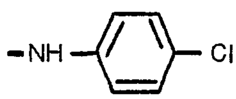 | 71 | 3330, 1645, 1540 | 5,15(s,1H), 6,8(d,2H), 7,3(m,2+2H), 7,7(d,2H), 8,3(s,1H) |
| ag) | 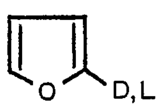 | 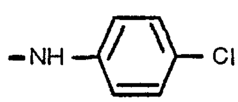 | 65 | 3350, 1640 | 5,45(s,1H), 6,40, (m,2H), 7,3(d,2H), 7,6(m,2+1H), 8,35(s,1H) |
| ah) | 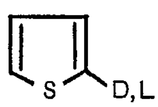 | 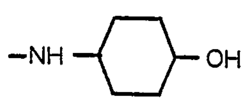 | 60 | 3320, 1660, 1570 | 5,4(s,1H), 7,0(m,2H), 7,4(d,1H), 7,6(m,1H), 8,25(s,1H) |
| aj) | 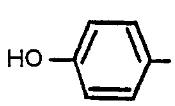 | 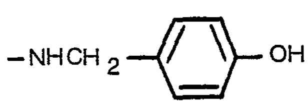 | 62 | 3340, 1650, 1540 | 4,25(breit,2H), 5,15(s,1H), 6,65(m,4H), 7,15(m,4H), 8,0(s,1H) |
| ak) | 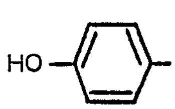 | 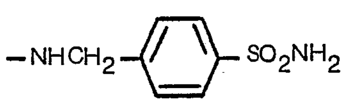 | 48 | 3300, 1645, 1535 | 4,5(breit,2H), 6,80(d,2H), 7,20(d,2H), 7,50(d,2H), 7,80(d,2H), 8,05(s,1H) |

| | A | R | Ausbeute (%) | IR-Spektrum $cm^{-1}$ | NMR-Spektrum Signale bei ppm (DMSO + $CD_3OD$) |
|---|---|---|---|---|---|
| al) | 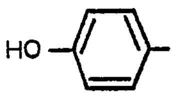 | 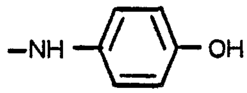 | 71,5 | 3320, 1650, 1545 | 5,15(s,1H), 6,85(d,2H), 7,5(m,4H), 8,0(d,2H), 8,30(s,1H) |
| am) | 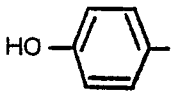 | 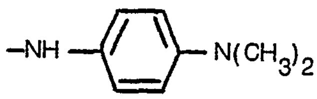 | 49 | 3350 (breit), 1645, 1540 | 3,0(d,6H), 5,15(s,1H), 6,80(d,2H), 7,5(m,4H), 7,7(d,2H), 8,25(s,1H) |
| an) | 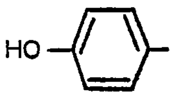 | 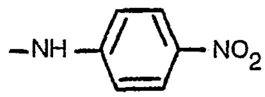 | 61 | 3300, 1640, 1545 | 3,05(d,6H), 5,10(s,1H), 6,75(d,2H), 7,35(d,2H), 7,6(d,2H), 7,95(d,2H), 8,40(s,1H) |
| ao) | 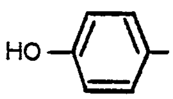 | 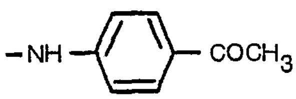 | 57 | 3320, 1650 | 2,1(s,3H), 5,15(s,1H), 6,8(d,2H), 7,40(m,4H), 7,7(d,2H), 8,25(s,1H) |
| ap) | 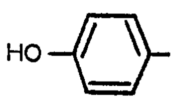 | 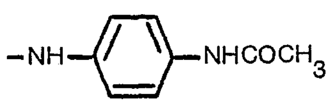 | 46 | 3350, 1640, 1610, 1540 | 2,05(s,2H), 5,15(s,1H), 6,75(d,2H), 7,3(d,2H), 7,4(d,2H), 7,7(d,2H), 8,3(s,1H) |
| aq) | 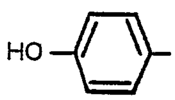 | 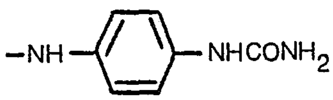 | 64 | 3340, 1650, 1600, 1550 | 5,10(s,1H), 6,70(d,2H), 7,3(d,2H), 7,35(d,2H), 7,55(d,2H), 8,2(s,1H) |
| ar) | 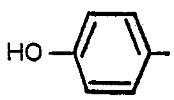 | 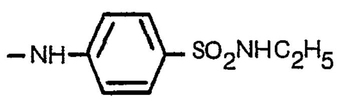 | 51 | 3330, 1645, 1545 | 1,25(t,3H), 3,55(m,2H), 5,15(s,1H), 6,75(d,2H), 7,25(d,2H), 7,8(9,4H), 8,35(s,1H) |
| as) | 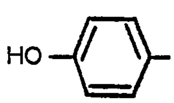 | 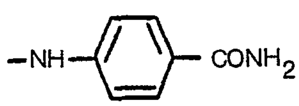 | 58 | 3350 (breit) 1650, 1600, 1550 | 5,15(s,1H), 6,75(d,2H), 7,35(d,2H), 7,8 (sich uberlegendes dd), 8,40(s,1H) |
| at) | 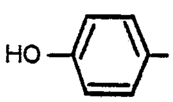 | 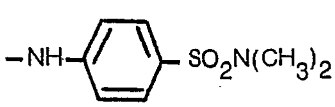 | 61 | 3320, 1650, 1530 | 2,45(6H), 5,15(s,1H), 6,75(d,2H), 7,25(d,2H), 7,75(q,4H), 8,30(s,1H) |

| | A | R | Ausbeute (%) | IR-Spektrum $cm^{-1}$ | NMR-Spektrum Signale bei ppm (DMSO + $CD_3OD$) |
|---|---|---|---|---|---|
| au) | 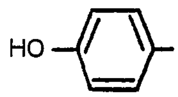 | 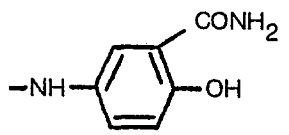 | 37 | 3340, 1655, 1610, 1535 | 5,15(s,1H), 6,85(d,1H), 7,30(d,2H), 7,55(m,1H), 8.0(d,1H), 8,1(s,1H) |
| av) | 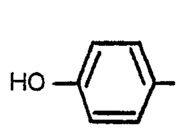 | 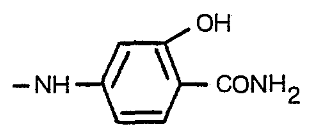 | 44 | 3360, 1655, 1605, 1535 | 5,10(s,1H), 6,75(d,2H), 7,25(m,3H), 7,7(m,2H), 8,35(s,1H) |
| aw) | 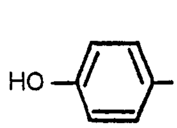 | 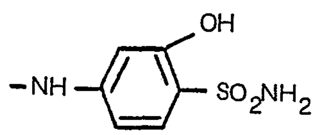 | 56,5 | 3320, 1650, 1540 | 5,15(s,1H), 6,80(d,2H), 7,30(m,3H), 8,0(m,2H), 8,35(s,1H) |
| ax) | 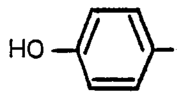 | 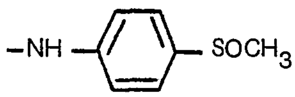 | 49,5 | 3330, 1660, 1530 | 2,7(s,3H), 5,15(s,1H), 6,75(d,2H), 7,2–8,1 (m,6H), 8,35(s,1H) |
| ay) | 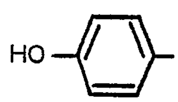 | $-NHCOCH_3$ | 48 | 3350, 1655, 1580 | 2,3(s,3H), 5,1(s,1H), 6,8(d,2H), 7,35(d,2H), 8,25(s,1H) |

az) D-$\alpha$-(2-p-Aminosulfonylanilino-4-hydroxy-5-pyrimidinyl)-ureido-p-hydroxy-phenylessigsäure

1,405 g (0,005 Mol) 5-Amino-2-p-aminosulfonylanilino-4-hydroxy-pyrimidin werden in 50 ml trockenem Tetrahydrofuran aufgeschlämmt und mit 4 g Trimethylsilyldiäthylamin bis zur vollständigen Lösung zum Rückfluß erhitzt (10—30 Minuten). Die Lösung wird im Vakuum zur Trockne eingeengt weider in 50 ml Tetrahydrofuran aufgenommen und unter Eiskühlung zu einer Lösung 530 mg Phosgen in 35 ml trockenem Tetrahydrofuran zugetropft. Man rührt 10 Minuten bei Raumtemperatur nach und engt dann im Vakuum zur Trockne ein. Das zurückbleibende Festprodukt wird unter Eiskühlung mit 30 ml Methanol versetzt, wobei Lösung eintritt. Nach kurzer Zeit fällt analysenreines 7-p-Aminosulfonylanilino-2-hydroxy-oxazolo-[5,4-d]-pyrimidin aus, das abgesaugt und getrocknet wird.

1,3 g des so isolierten Zwischenproduktes werden unter Eiskühlung portionsweise zu einer Lösung von 700 mg (4,5 mMol) p-Hydroxyphenylglycin, die mit 4,5 ml 1 n Natronlauge in 50 ml 80%igem Tetrahydrofuran hergestellt wurde, gegeben. Dabei wird der pH-Wert auf etwa 7,5 gehalten. Die weitere Umsetzung und Aufarbeitung erfolgt analog Beispiel 1t).

Ausbeute: 1,36 g (76%),

IR-Spektrum: 3300 (breit), 1640, 1530, 1155 $cm^{-1}$;

NMR-Spektrum (DMSO + $CD_3OD$) Signale bei ppm: 5,15 (s, 1H), 6,77 (d, 2H), 7,22 (d, 2H), 7,80 (q, 4H), 8,31 (s, 1H).

Analog wurden synthetisiert:

| | A | R | Ausbeute (%) | IR-Spektrum $cm^{-1}$ | NMR-Spektrum Signale bei ppm (DMSO + $CD_3OD$) |
|---|---|---|---|---|---|
| ba) | 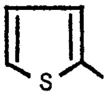 D,L | 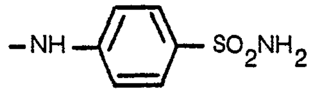 | 81,5 | 3320, 1645, 1535 | 5,50(s,1H), 7,05(m,2H), 7,45(m,1H), 7.8(q,4H), 8,35(s,1H) |
| bb) | 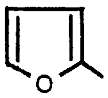 D,L | 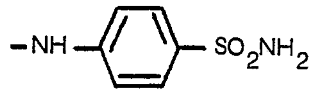 | 68 | 3300, 1650, 1540 | 5,4(s,1H), 6,45(m,2H), 7,6(s,1H), 7,8(q,4H), 8,35(s,1H) |
| bc) | 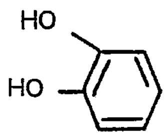 D,L | 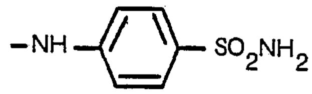 | 54 | 3320, 1655, 1545 | 5,15(s,1H), 6,75(m,2H), 7,3(m,1H), 7,8(q,4H), 8,35(s,1H) |
| bd) | 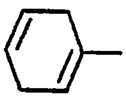 | 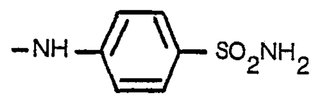 | 62 | 3340, 1660, 1545 | 2,50(m,4H), 4,90(1H), 5,30(m,3H), 7,8(q,4H), 8,35(s,1H) |
| be) | 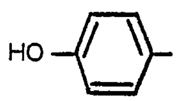 | 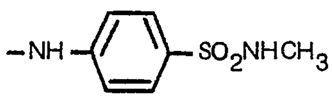 | 73 | 3330, 1655, 1550 | 2,45(s,3H), 5,15(s,1H), 6,80(d,2H), 7,30(d,2H), 7,75(q,4H), 8,35(s,1H) |
| bf) | 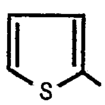 | 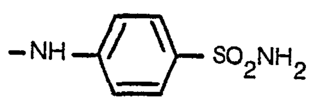 | 79 | 3300, 1660, 1555 | 2,45(s,3H), 5,50(s,1H), 7,05(m,2H), 7,40(m,1H), 7,80(q,2H), 8,35(s,1H) |
| bg) | 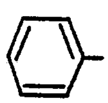 | 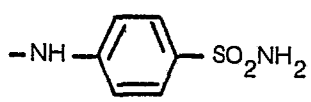 | 71 | 3300, 1650, 1550 | 5,15(s,1H), 7,45(m,5H), 7,80(q,4H), 8,35(s,1H) |

### Beispiel 2

Natrium-7-{D-α-[(cyclopropyl-4-hydroxy-5-pyrimidinyl)-ureido]-p-hydroxy-phenylacetamido}-3-acetoxymethyl-ceph-3-em-4-carboxylat

1,72 g (0,005 Mol) der nach Beispiel 1a) erhaltenen Ureidocarbonsäure werden zusammen mit 2,1 g 7-Amino-3-acetoxymethyl-ceph-3-em-4-carbonsäure-benzhydrylester (0,005 Mol) in Methylenchlorid gelöst. Zu der Lösung wird unter Eiskühlung 1,15 g (0,0055 Mol) dicyclohexylcarbodiimid gegeben und die Lösung 8 h bei 5°C gerührt. Dann wird vom entstandenen Harnstoff abfiltriert und im Vakuum zur Trockne eingeengt. Zur Entfernung geringfügiger Verunreinigungen wird über eine Kieselgelsäule chromatographiert (Eluens Methylenchlorid-Methanol 12:1)

Ausbeute an Benzhydrylester 3,2 g (82%).

Das so erhaltene Produkt wird in wenig Methylenchlorid suspendiert und unter Eiskühlung 30

Minuten mit 2 ml Anisol und 10 ml Trifluoroessigsäure gerührt. Anschließend werden zweimal 50 ml Toluol hinzugefügt und im Vakuum jeweils zur Trockne eingeengt. Das entstandene Produkt wird mit Äther versetzt und durch Absaugen isoliert. Durch Zugabe der berechneten Menge Natriumäthylhexanoat in Methanol und Zufügen von Äther wird das Natriumsalz ausgefällt, das abgesaugt und im Vakuum getrocknet wird.

Ausbeute an Natriumsalz: 2,23 g (91%),

IR-Spektrum: 1760, 1660, 1615, 1550 $cm^{-1}$;

NMR-Spektrum (DMSO + $CD_3OD$) Signale bei ppm: 1,20 (m, 4H), 1.95 (m, 1H), 2,1 (s, 3H), 3,45 (q, 2H), 4,85 (q, 2H), 4,40 (d, 1H), 5,55 (s, 1H), 5,60 (d, 1H), 6,75 (d, 2H), 7,3 (d, 2H), 8,50 (s, 1H).

Beispiel 3

Natrium-7-{D-$\alpha$-[(2-cyclopropyl-4-hydroxy-5-pyrimidinyl)-ureido]-p-hydroxy-phenylacetamido}-3-[(1-methyl-tetrazol-5-yl)-thiomethyl]-ceph-3-em-4-carboxylat

3,44 g (0,01 Mol) der Ureidocarbonsäure des Beispiels 1 a werden auf analoge Weise, wie in Beispiel 2 beschrieben, mit 4,94 g (0,01 Mol) 7-Amino-3-[(1-methyl-tetrazol-5-yl)-thiomethyl]-ceph-3-em-4-carbonsäurebenzhydrylester umgesetzt. Nach Abspaltung der Schutzgruppe erhält man 4,52 g (65%) Natriumsalz.

IR-Spektrum: 1760, 1660, 1610, 1540 $cm^{-1}$;

NMR-Spektrum (DMSO + $CD_3OD$) Signale bei ppm: 1,25 (m, 4H), 1,90 (m, 1H), 3,50 (q, 2H), 3,90 (s, 3H), 4,30 (q, 2H, teilweise verdeckt durch LM), 4,80 (d, 1H), 5,50 (s, 1H), 5,70 (d, 1H), 6,75 (d, 2H), 7,35 (d, 2H), 8,45 (s, 1H).

Beispiel 4

Natrium-7-{D-$\alpha$-[(2-cyclopropyl-4-hydroxy-5-pyrimidinyl)-ureido]-p-hydroxy-phenylacetamido}-3-[(2-methyl-1,3,4-thiadiazol-5-yl)-thiomethyl]-ceph-3-em-4-carboxylat

Ausgehend von 700 mg (0,002 Mol) der Ureidocarbonsäure des Beispiels 1 a und 1,02 g (0,002 Mol) 7-Amino-3-[(2-methylthiadiazol-5-yl)-thiomethyl]-ceph-3-em-4-carbonsäure-benzhydryl-ester. Nach Abspaltung der Schutzgruppe wurden 540 mg (39%) Natriumsalz erhalten.

IR-Spektrum: 1760, 1655, 1615, 1540 $cm^{-1}$;

NMR-Spektrum (DMSO + $CD_3OD$) Signale bei ppm: 1,0 (m, 4H), 1,9 (m, 1H), 2,7 (s, 3H), 3,50 (q, 2H), 4,45 (q, 2H), 4,90 (d, 1H), 5,50 (s, 1H), 5,65 (d, 1H), 6,75 (d, 2H), 7,3 (d, 2H), 8.45 (s, 1H).

Beispiel 5

Natrium-7-{D,L-$\alpha$-[(2-cyclopropyl-4-hydroxy-5-pyrimidinyl)-ureido]-2-thienylacetamido}-3-[(1-methyl-tetrazol-5-yl)-thiomethyl]-ceph-3-em-4-carboxylat

Dieses Cephalosporin wird erhalten, wenn man von 1,0 g (0,0029 Mol) der Ureidocarbonsäure des Beispiels 1b sowie 1,5 g (0,003 Mol) des in Beispiel 3 eingesetzten Benzhydryl-esters ausgeht und die Umsetzung analog Beispiel 2 führt. Nach Abspalten der Schutzgruppe werden 930 mg (48%) Natriumsalz erhalten.

IR-Spektrum: 1760, 1660, 1610, 1540 $cm^{-1}$;

NMR-Spektrum (DMSO + $CD_3OD$) Signale bei ppm: 1,0 (m, 4H), 1,9 (m, 1H), 3,55 (q, 2H), 3,90 (s, 3H), 4,35 (q, 2H), 4,90 (dd, 1H), 5,5 (dd, 1H), 5,75 (d, 1H), 6,9 (m 2H), 7,35 (d, 1H), 8,40 (s, 1H).

Beispiel 6

Natrium-7-{D-$\alpha$-[(2-p-aminosulfonylanilino-4-hydroxy-5-pyrimidinyl)-ureido]-p-hydroxy-phenylacetamido}-3-[(2-methyl-1,3,4-thiadiazol-5-yl)-thiomethyl]-ceph-3-em-4-carboxylat

Dieses Cephalosporin erhält man aus 950 mg (0,2 m Mol) der Ureidocarbonsäure des Beispiels 1 az) sowie 900 mg des Diphenylmethylesters der 7-Amino-3-[(2-methyl-1,3,4-thiadiazol-5-yl)-thiomethyl]-cephalosporansäure nach der Methode des Beispiels 2. Als Lösungsmittel wird ein 2:1 Gemisch von Methylenchlorid und Dimethylformamid verwendet. Nach Abspalten der Schutzgruppe erhält man 821 mg (54%) Natriumsalz.

IR-Spektrum: 1760, 1660, 1600, 1150 $cm^{-1}$;

NMR-Spektrum (DMSO + $CD_3OD$) Signale bei ppm: 2,7 (s, 3H), 3,50 (q, 2H), 4,45 (q, 2H), 4,90 (d, 1H), 5,50 (s, 1H), 5,65 (d, 1H), 6,75 (d, 2H), 7,35 (d, 2H), 7,7 (d, 2H), 8,0 (d, 2H), 8,37 (s, 1H).

Beispiel 7

Natrium-7-{D-$\alpha$-[(4-hydroxy-2-isopropylamino-5-pyrimidinyl)-ureido]-p-hydroxy-phenylacetamido}-3-[(1-methyl-tetrazol-5-yl)-thiomethyl]-ceph-3-em-4-carboxylat

Dieses Cephalosporin wird analog Beispiel 2 erhalten, indem man von 3,55 g (0,01 Mol) der Ureidocarbonsäure des Beispiels 1e sowie 4,94 g (0,01 Mol) des in Beispiel 3 eingesetzten Cephalosporinderivats ausgeht.

Ausbeute nach Abspalten der Schutzgruppe: 3,65 g Natriumsalz (51%)

IR-Spektrum: 1760, 1655, 1610, 1545 $cm^{-1}$;

NMR-Spektrum (DMSO + $CD_3OD$) Signale bei ppm: 1,15 (d, 6H), 3,55 (q, 2H), 3,90 (s, 3H + m, 1H), 4,30 (q, 2H), 4,95 (d, 1H), 5,4 (s, 1H), 5,6 (d, 1H), 6,8 (d, 2H), 7,35 (d, 2H), 8,05 (s, 1H).

Beispiel 8

Natrium-7-{D-α-[(4-hydroxy-2-isopropylamino-5-pyrimidinyl)-ureido]-p-hydroxy-phenylacetamido}-3-[(2-methyl-1,3,4-thiadiazol-5-yl)-thiomethyl]-ceph-3-em-4-carboxylat

Analog Beispiel 2, ausgehend von 500 mg (0,0014 Mol) der Ureidocarbonsäure des Beispiels 1e und 720 mg (0,0014 Mol) des Cephalosporins, das im Beispiel 4 eingesetzt wurde.

Ausbeute nach Abspalten der Benzhydrylgruppe 460 mg (46%) Natriumsalz.

IR-Spektrum: 1760, 1660, 1610, 1550 $cm^{-1}$;

NMR-Spektrum (DMSO + $CD_3OD$) Signale bei ppm: 1,15 (d, 6H) 2,75 (s, 3H), 3,55 (q, 2H), 3,95 (m, 1H), 4,25 (q, 2H teilweise durch LM verdeckt), 5,0 (d, 1H), 5,45 (s, 1H), 5,65 (d, 1H), 6,85 (d, 2H), 7,35 (d, 2H), 8,05 (s, 1H).

Beispiel 9

Natrium-7-{D,L-α-[(4-hydroxy-2-propylamino-5-pyrimidinyl)-ureido]-2-furylacetamido}-3-[(1-methyl-tetrazol-5-yl)-thiomethyl]-ceph-3-em-4-carboxylat

Analog Beispiel 2 werden 670 mg der Ureidocarbonsäure des Beispiels 1f (0,002 Mol) mit 1,0 g (0,0021 Mol) des Cephalosporin-benzhydrylesters, der auch in Beispiel 3 verwendet wurde, umgesetzt.

Nach Abspaltung der Benzhydrylschutzgruppe erhält man 680 mg (50%) Natriumsalz.

IR-Spektrum: 1760, 1655, 1615, 1545 $cm^{-1}$;

NMR-Spektrum (DMSO + $CD_3OD$) Signale bei ppm: 1,0 (t, 3H), 1,6 (q, 2H), 3,2 (t, 2H), 3,55 (q, 2H), 3,90 (s, 3H), 4,35 (q, 2H), 4,95 (dd, 1H), 5,45 (dd, 1H), 5,75 (d, 1H), 6,4 (m, 2H), 7,6 (s, 1H), 8,05 (s, 1H).

Beispiel 10

Natrium-7-{D-α-[(4-hydroxy-2-(2′-methylallylamino)-5-pyrimidinyl)-ureido]-p-hydroxy-phenylacetamido}-3-[(1-methyl-tetrazol-5-yl)-thiomethyl]-ceph-3-em-4-carboxylat

Analog Beispiel 2 ausgehend von 370 mg (0,001 Mol) der Ureidocarbonsäure des Beispiels 1l sowie 500 mg (0,001 Mol) des Benzhydrylesters, der auch in Beispiel 3 eingesetzt wurde. Nach Abspaltung der Benzhydrylschutzgruppe erhält man 385 mg Natriumsalz (53,5%),

IR-Spektrum: 1760, 1655, 1615, 1540 $cm^{-1}$;

NMR-Spektrum (DMSO + $CD_3OD$) Signale bei ppm: 1,8 (s, 3H), 3,4 (q, 2H), 3,85 (breites s, 2H), 4,35 (q, 2H), 4,85 (m, 2+1 H), 5,40 (s, 1H), 5,55 (d, 1H), 6,7 (d, 2H), 7,35 (d, 2H), 8,0 (s, 1H).

Beispiel 11

Natrium-7-{D,L-α-[(4-hydroxy-2-(4′-hydroxycyclohexylamino)-5-pyrimidinyl)-ureido]-2-thienylacetamido}-3-[(2-methyl-thiadiazol-5-yl)-thiomethyl]-ceph-3-em-4-carboxylat

Herstellung analog Beispiel 2. Man geht aus von 815 mg (0,002 Mol) der Ureidocarbonsäure des Beispiels 1v sowie 1,0 g des Benzhydrylesters der 7-Amino-3-[(2-methyl-thiadiazol-5-yl)-thiomethyl]-ceph-3-em-4-carbonsäure (0,002 Mol).

Nach Abspaltung der Schutzgruppe erhält man 640 mg Natriumsalz (42,5%).

IR-Spektrum: 1760, 1660, 1615, 1540 $cm^{-1}$;

NMR-Spektrum (DMSO + $CD_3OD$) Signale bei ppm: 1,8 (m, 8H), 2,7 (s, 3H), 3,45 (q, 2H), 3,5—4,1 (m, 1+1H), 4,40 (q, 2H), 4,95 (dd, 1H), 5,45 (dd, 1H), 5,70 (d, 1H), 6,9 (m, 2H), 7,3 (d, 1H), 8,05 (s, 1H).

Beispiel 12

Natrium-7-{D,L-α-[(4-hydroxy-2-hydroxyanilino)-5-pyrimidinyl)-ureido]-2-thienylacetamido}-3-[1-methyl-tetrazol-5-yl)-thiomethyl]-ceph-3-em-4-carboxylat

Herstellung analog Beispiel 2. Man geht aus von 400 mg (0,001 Mol) der Ureidocarbonsäure des Beispiels 1ah) sowie 500 mg des Benzhydrylesters des Beispiels 3 (0,001 Mol).

Nach Abspaltung der Schutzgruppe wurden 220 mg Natriumsalz (29,5%) erhalten.

IR-Spektrum: 1760, 1655, 1615, 1540 $cm^{-1}$;

NMR-Spektrum (DMSO + $CD_3OD$) Signale bei ppm: 3,45 (q, 2H), 3,90 (s, 3H), 4,30 (q, 2H, teilweise durch LM verdeckt), 4,95 (dd, 1H), 5,50 (dd, 1H), 5,65 (d, 1H), 7,0 (m, 2H), 7,5 (m, 5H), 8,25 (s, 1H).

Beispiel 13

Natrium-7-{D-α-[(2-acetylamino-4-hydroxy-5-pyrimidinyl)-ureido]-p-hydroxy-phenylacetamido}-3-[(2-methyl-thiadiazol-5-yl)-thiomethyl]-ceph-3-em-4-carboxylat

Synthese analog Beispiel 2, ausgehend von 1,0 g der Ureidocarbonsäure des Beispiels 1ax) (0,0028 Mol) sowie 1,53 g (0,003 Mol) 7-Amino-3-[(2-methyl-thiadiazol-5-yl)-thiomethyl]-ceph-3-em-4-carbonsäure-benzhydrylester.

Ausbeute an Natriumsalz 750 mg (36%),

IR-Spektrum: 1760, 1660, 1610, 1545 $cm^{-1}$;

NMR-Spektrum (DMSO + $CD_3OD$) Signale bei ppm: 2,3 (s, 3H), 2,75 (s, 3H), 3,45, (q, 2H), 4,45 (q, 2H), 4,95 (d, 1H), 5,40 (s, 1H), 5,60 (d, 2H), 6,85 (d, 2H), 7,35 (d, 2H), 8,05 (s, 1H).

Analog wurden weiter hergestellt:

Natrium-7-{D-α-[(2-p-chloranilino-4-hydroxy-5-pyrimidinyl)-ureido]-p-hydroxy-phenylacetamido}-3-acetoxymethyl-ceph-3-em-4-carboxylat

Natrium-7-{D-α-[(2-p-chloranilino-4-hydroxy-5-pyrimidinyl)-ureido]-p-hydroxy-phenylacetamido}-3-[(2-methyl-thiadiazol-5-yl)-thiomethyl]-ceph-3-em-4-carboxylat

Natrium-7-{D-α-[(2-cyclohexylamino-4-hydroxy-5-pyrimidinyl)-ureido]-p-hydroxy-phenylacetamido}-3-acetoxymethyl-ceph-3-em-4-carboxylat

Natrium-7-{D-α-[(4-hydroxy-2-(4'-hydroxy-cyclohexylamino)-5-pyrimidinyl)-ureido]-p-hydroxy-phenylacetamido}-3-[(2-methylthiadiazol-5-yl)-thiomethyl]-ceph-3-em-4-carboxylat

Natrium-7-{D-α-[(2-sec.-butylamino-4-hydroxy-5-pyrimidinyl)-ureido]-p-hydroxy-phenylacetamido}-3-[(1,2,4-thiadiazol-5-yl)-thiomethyl]-ceph-3-em-4-carboxylat.

## Beispiel 14

Natrium-7-{D-α-[(2-(3'-aminosulfonylpropylamino)-4-hydroxy-5-pyrimidinyl)-ureido]-p-hydroxy-phenylacetamido}-3-[(1-methyltetrazol-5-yl)-thiomethyl]-ceph-3-em-4-carboxylat

Synthese analog Beispiel 6, ausgehend von 600 mg (1,36 m Mol) der Ureidocarbonsäure des Beispiels 1 ab) sowie 680 mg des Cephalosporins des Beispiels 3 (1,36 m Mol).

Man erhält nach Abspaltung der Schutzgruppe 460 mg Natriumsalz (42%);

IR-Spektrum: 1760, 1655, 1610, 1550 $cm^{-1}$;

NMR-Spektrum (DMSO + $CD_3OD$) Signale bei ppm: 2,0 (m, 2H), 2,75 (m, 2H), 3,35 (m, 2H), 3,5 (m, 2H), 3,95 (s, 3H), 4,35 (m, 2H), 4,90 (d, 1H), 5,4 (s, 1H), 5,6 (d, 2H), 6,75 (d, 2H), 7,3 (d, 2H), 8,10 (s, 1H).

## Beispiel 15

Natrium-7-{D-α-[(2-(3'-aminosulfonylpropylamino)-4-hydroxy-5-pyrimidinyl)-ureido]-p-hydroxy-phenylacetamido}-3-acetoxymethyl-ceph-3-em-4-carboxylat

Synthese analog Beispiel 6 bzw. 14, aber ausgehend von dem Cephalosporinderivat des Beispiels 2.

Ausbeute: 47,5%,

IR-Spektrum: 1760, 1650, 1615, 1550 $cm^{-1}$;

NMR-Spektrum (DMSO + $CD_3OD$) Signale bei ppm: 2,0 (m, 2H), 2,1 (s, 3H), 2,75 (m, 2H), 3,35 (m, 2H), 3,45 (m, 2H), 4,80 (q, 2H+d, 1H), 5,45 (s, 1H), 5,60 (d, 2H), 6,75 (d, 2H), 7,3 (d, 2H), 8,10 (s, 1H).

## Beispiel 16

Natrium-7-{D-α-[(2-(3'-aminocarbonylpropylamino)-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Synthese analog Beispiel 6, ausgehend von 800 mg (1,9 m Mol) der Ureidocarbonsäure des Beispiels 1 ac) sowie 950 mg des Cephalosporinderivats des Beispiels 3.

Nach Abspaltung der Schutzgruppe erhält man 602 mg Natriumsalz (40,5%).

IR-Spektrum: 1760, 1655, 1610, 1555 $cm^{-1}$;

NMR-Spektrum (DMSO + $CD_3OD$) Signale bei ppm: 1,9 (t, 2H), 2,45 (m, 2H), 3,4—3,5 (m, 4H), 3,95 (s, 3H), 4,40 (m, 2H), 4,95 (d, 1H), 5,5 (s, 1H), 5,65 (d, 1H), 6,75 (d, 2H), 7,3 (d, 2H) 8,10 (s, 1H).

## Beispiel 17

Natrium-7-{D-α-[(2-(2'-acetylaminoäthylamino)-4-hydroxy-5-pyrimidinyl)-ureido]-p-hydroxy-phenylacetamido}-3-[(1-methyltetrazol-5-yl)-thiomethyl]-ceph-3-em-4-carboxylat

Synthese analog Beispiel 6, ausgehend von 425 mg der Ureidocarbonsäure des Beispiels 1 ae) (1,0 m Mol) sowie 500 mg des Cephalosporinderivats des Beispiels 3.

Ausbeute: 355 mg Natriumsalz (46,5%),

NMR-Spektrum (DMSO + $CD_3OD$) Signale bei ppm: 2,2 (s, 3H), 2,7 (m, 2H), 3,2—3,5 (m, 4H), 3,95 (s, 3H), 4,4 (q, 2H), 4,90 (d, 1H), 5,45 (s, 1H), 5,60 (d, 1H), 6,85 (d, 2H), 7,35 (d, 2H), 8,10 (s, 1H).

## Beispiel 18

Natrium-7-{D-α-[(2-p-aminosulfonylbenzylamino-4-hydroxy-5-pyrimidinyl)-ureido]-p-hydroxy-phenylacetamido}-3-[(1-methyltetrazol-5-yl)-thiomethyl]-ceph-3-em-4-carboxylat

Synthese analog Beispiel 6, ausgehend von 1,0 g der Ureido-carbonsäure des Beispiels 1 ak) (2,05 m Mol) und 1,1 g des Cephalosporinderivats des Beispiels 14 (2,2 m Mol).

Ausbeute: 950 mg (54,8%) Natriumsalz;

IR-Spektrum: 1760, 1655, 1615, 1550 $cm^{-1}$;

NMR-Spektrum (DMSO + $CD_3OD$) Signale bei ppm: 3,5 (m, 2H), 3,95 (s, 3H), 4,3 (m, 2H), 4,55 (s, breit, 2H), 4,9 (d, 1H), 5,4 (s, 1H), 5,6 (d, 1H), 6,75 (d, 2H), 7,3 (d, 2H), 7,5 (d, 2H), 7,85 (d, 2H), 8,1 (s, 1H).

Beispiel 19

Natrium-7-{D-α-[2-p-aminosulfonylbenzylamino-4-hydroxy-5-pyrimidinyl)-ureido]-p-hydroxy-phenylacetamido}-3-acetoxymethyl-ceph-3-em-4-carboxylat

Synthese analog Beispiel 6, ausgehend von 700 mg der Ureidocarbonsäure des Beispiels 1 ak) (1,43 m Mol) und 600 mg des Cephalosporinderivats des Beispiels 2.

Ausbeute: 535 mg (47%) Natriumsalz,

NMR-Spektrum (DMSO + $CD_3OD$) Signale bei ppm: 2,05 (s, 3H), 3,45 (q, 2H), 4,55 (s, 2H), 4,9 (m, 2+1H), 5,5 (s, 1H), 5,6 (d, 1H), 6,75 (d, 2H), 7,3 (d, 2H), 8,1 (s, 1H).

Beispiel 20

Natrium-7-{D-α-[(2-p-aminosulfonylanilino-4-hydroxy-5-pyrimidinyl)-ureido]-p-hydroxy-phenylacetamido}-3-[(1-methyltetrazol-5-yl)-thiomethyl]-ceph-3-em-4-carboxylat

1,2 g der Ureidocarbonsäure des Beispiels 1 az) (2,53 m Mol) werden in 30 ml trockenem Dimethylformamid gelöst und mit einer Lösung von 1,36 g 7-Amino-3-(1-methyl-tetrazol-5-yl)-thiomethyl-cephalosporancarbonsäure-diphenylmethylester (2,75 m Mol) in 50 ml trockenem Methylenchlorid versetzt. Unter Eiskühlung gibt man 0,57 g Dicyclohexylcarbodiimid, gelöst in 30 ml trockenem Methylenchlorid zu und rührt bei dieser Temperatur 8 Stunden. Das Methylenchlorid wird im Wasserstrahlvakuum entfernt und der Dicyclohexylharnstoff abfiltriert. Dann engt man im Hochvakuum zur Trockene ein und rührt den Rückstand zweimal mit je 40 ml Methanol und einmal mit Methylenchlorid aus. Das zurückbleibende Festprodukt wird abgesaugt. Es ist laut Dünnschicht-Chromatogramm (Laufmittel Methanol/Methylenchlorid 1:5) einheitlich.

Die Abspaltung der Diphenylmethylester-Gruppe erfolgt analog Beispiel 2. Die Herstellung des Natriumsalzes wird in Dimethylformamid mit Natriumhexanoat durchgeführt.

Ausbeute: 1,24 g (6,1%) Natriumsalz (enthält laut NMR-Spektrum etwa 1 Mol Dimethylformamid).

NMR-Spektrum (DMSO + $CD_3OD$) Signale bei ppm: 3,45 (m, 2H), 3,92 (s, 3H), 4,35 (m, 2H, teilweise durch LM verdeckt), 4,88 (d, 1H), 5,45 (s, 1H), 5,55 (d, 1H), 6,72 (d, 2H), 7,30 (d, 2H), 7,7 (d, 2H), 8,0 (d, 2H), 8,38 (s, 1H).

IR-Spektrum 1760, 1660, 1605, 1150 $cm^{-1}$;

Analog dieser Vorschrift wurden ausgehend von 7-Amino-3-(1-methyl-tetrazol-5-yl)-thiomethyl-cephalosporancarbonsäure-diphenylmethyl-ester bzw. von 7-Amino-3-acetoxymethyl-cephalosporancarbonsäure-diphenylmethylester und der entsprechenden Ureidocarbonsäure folgende Cephalosporine synthetisiert:

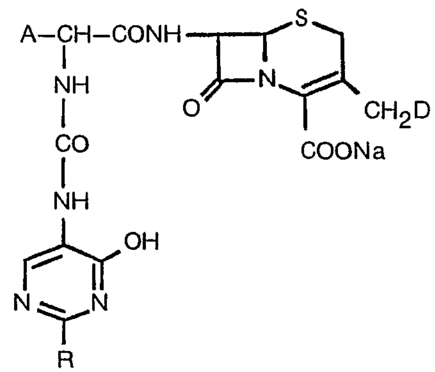

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 21 | 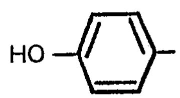 | 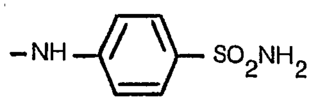 | $-OCOCH_3$ | 1 az) | 56 | 1760, 1660<br>1600, 1150 | 2,05(s,3H), 3,4(q,2H), 5,0(m,2+1H), 5,5(s,1H), 5,65(d,1H), 6,8(d,2H), 7,35(d,2H), 7,7(d,2H), 8,0(d,2H), 8,36(s,1H) |
| 22 | 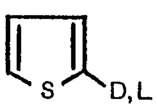 | 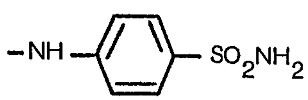 | 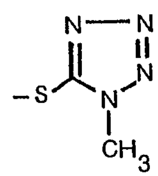 | 1 ba) | 64,5 | 1760, 1655<br>1600, 1150 | 3,45(m,2H), 3,95(s,3H), 4,3(q,2H), 5,0(dd,1H), 5,6(dd,1H), 5,75(d,1H), 6,8–7,2(m,2H, 7,35(m,1H), 7,7(d,2H), 8,0(d,2H), 8,35(s,1H) |
| 23 | 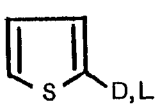 | 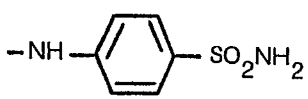 | $-OCOCH_3$ | 1 ba) | 59,5 | 1760, 1655<br>1605, 1150 | 2,05(s,3H), 3,45(m,2H), 5,0(m,2+1H), 5,45(dd,1H), 5,75(d,1H), 6.8–7,2(m,2H), 7,30(m,1H), 7,7(d,2H), 8,0(d,2H), 8,35(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 24 | 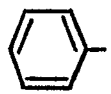 | 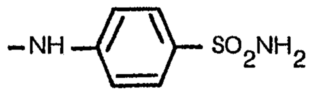 | 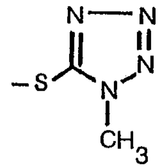 | 1 bg) | 49 | 1760, 1650, 1600, 1150 | 3,4(q,2H), 3,95(s,3H), 4,35 (m,2H), 4,90(d,1H), 5,50(s,1H), 5,60(d,1H), 7,2–7,7(m,7H), 8,0(d,2H), 8,35(s,1H) |
| 25 | 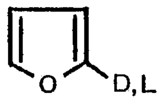 | 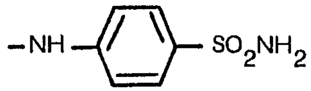 | 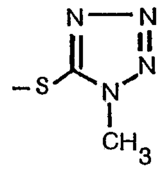 | 1 bb) | 62 | 1760, 1660, 1605, 1150 | 3,45(q,2H), 3,95(s,3H), 4,35 (m,2H), 4,95(dd,1H), 5,45 (dd,1H), 5,70(d,1H), 6,4 (breit,2H), 7,7(m,3H), 8,0 (d,2H), 8,37(s,1H) |
| 26 | 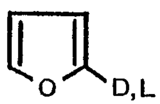 | 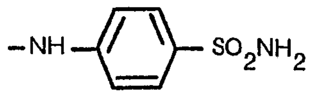 | $-OCOCH_3$ | 1 bb) | 57 | 1760, 1655, 1600, 1150 | 2,05(s,3H), 3,4(m,2H), 4,9 (m,2+1H), 5,40(dd,1H), 5,70 (d,1H), 6,45 (breites Signal, 2H), 7,6(breit,1H), 7,7(d,2H), 8,0(d,2H), 8,35(s,1H) |
| 27 | 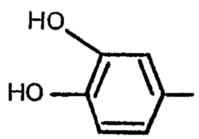 | 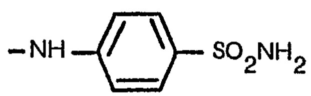 | 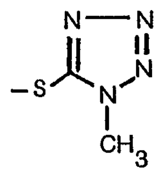 | 1 bc) | 44 | 1760, 1660, 1600, 1150 | 3,40(q,2H), 3,95(s,3H), 4,4 (m,2H), 4,90(d,1H), 5,50(s,1H), 5,60(d,1H), 6,75(m,2H), 7,4 (m,1H), 7,7(d,2H), 8,0(d,2H), 8,35(s,1H) |
| 28 | 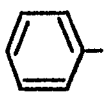 | 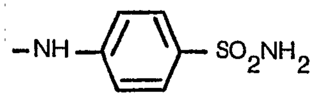 | 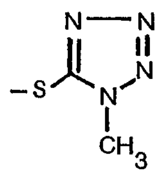 | 1 bd) | 46 | 1760, 1650, 1660, 1150 | 2,50(m,4H), 3,45(m,2H), 3,90 (s,3H), 4,4(m,2H), 4,90(d,1H), 5,2–5,6(m,5H), 7,7(d,2H), 8,0(d,2H), 8,35(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 29 | 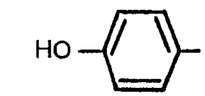 | 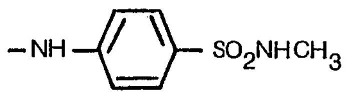 | $-OCOCH_3$ | 1 be) | 72 | 1760, 1660, 1605, 1150 | 2,05(s,3H), 2,50(s,3H), 3,45 (m,2H), 4,9(m,2+1H), 5,45 (s,1H), 5,60(d,1H), 6,75(d,2H), 7,35(d,2H), 7,85(q,4H), 8,35 (s,1H) |
| 30 | 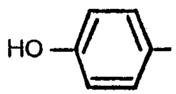 | 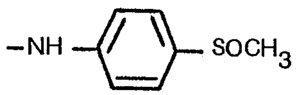 | 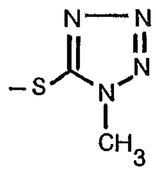 | 1 ax) | 44 | 1760, 1655, 1600 | 2,7(s,3H), 3,45(q,2H), 3,95 (s,3H), 4,35(q,2H), 4,95(d,1H), 5,50(s,1H), 5,60(d,1H), 6,75(d,2H), 7,2–7,95(m,6H), 8,30(s,1H) |
| 31 | 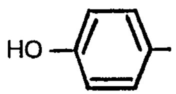 | 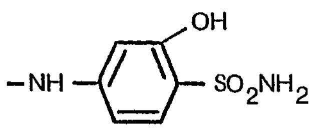 | 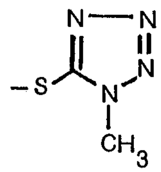 | 1 aw) | 40,5 | 1760, 1660, 1600, 1150 | 3,40(q,2H), 3,95(s,3H), 4,30(q,2H), 4,90(d,1H), 5,45 (s,1H), 5,55(d,1H), 6,80(d,2H), 7,30(m,3H), 7,9(m,2H), 8,35(s,1H) |
| 32 | 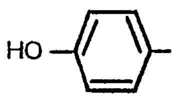 | 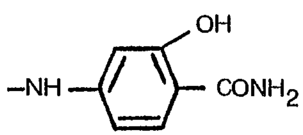 | 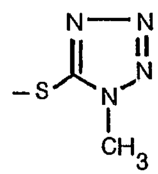 | 1 av) | 46,5 | 1760, 1660, 1600 | 3,45(q,2H), 3,95(s,3H), 4,35 (q,2H), 4,95(d,1H), 5,50 (s,1H), 5,60(d,1H), 6,75 (d,2H), 7,25(m,3H), 7,7 (m,2H), 8,30(s,1H) |
| 33 | 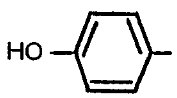 | 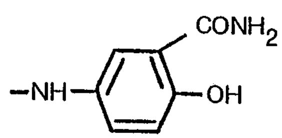 | 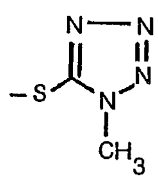 | 1 au) | 51 | 1760, 1650, 1605 | 3,45(q,2H), 3,95(s,3H), 4,4(m,2H), 4,90(d,1H), 5,45 (s,1H), 5,55(d,1H), 6,75 (d,2H), 6,85(d,1H), 7,30 (d,2H), 7,55(m,1H), 8,0 (d,1H), 8,15(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 34 | 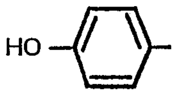 | 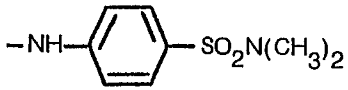 | 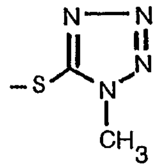 | 1 at) | 68 | 1760, 1660, 1600, 1150 | 2,45(d,6H), 3,45(q,2H), 3,95(s,3H), 4,35(q,2H), 4,90(d,1H), 5,45(s,1H), 5,60(d,1H), 6,75(d,2H), 7,25(d,2H), 7,75(q,4H), 8,35(s,1H) |
| 35 | 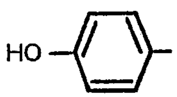 | 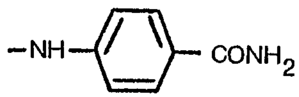 | $-OCOCH_3$ | 1 as) | 63 | 1760, 1640–60, 1600 | 2,05(s,3H), 3,4(m,2H), 4,90(m,2+1H), 5,45(s,1H), 5,60(d,1H), 6,75(d,2H), 7,35(d,2H), 7,75(dd,4H), 8,38(s,1H) |
| 36 | 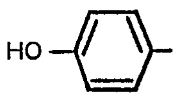 | 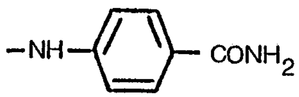 | 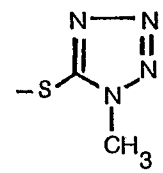 | 1 as) | 69 | 1760, 1650, 1600 | 3,45(q,2H), 3,95(s,3H), 4,35(m,2H), 4,95(d,1H), 5,45(s,1H), 5,55(d,1H), 6,75(d,2H), 7,35(d,2H), 7,5–7,9(m,4H), 8,37(s,1H) |
| 37 | 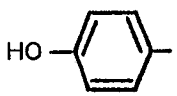 | 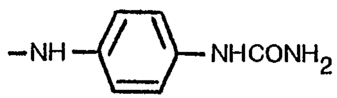 | 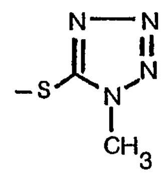 | 1 aq) | 54,5 | 1760, 1660, 1605 | 3,40(m,2H), 3,90(s,3H), 4,40(q,2H), 4,90(d,1H), 5,50(s,1H), 5,60(d,1H), 6,75(d,2H), 7,3(d,2H), 7,40(d,2H), 7,60(d,2H), 8,2(s,1H) |
| 38 | 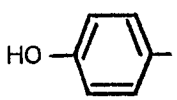 | 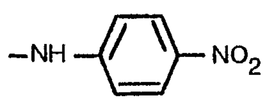 | 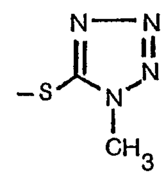 | 1 an) | 44 | 1760, 1655, 1600 | 3,45(q,2H), 3,95(s,3H), 4,40(q,2H), 4,95(d,1H), 5,45(s,1H), 5,60(d,1H), 6,75(d,2H), 7,35(d,2H), 7,6(d,2H), 7,95(d,2H), 8,40(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 39 | 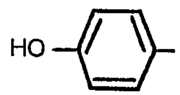 | 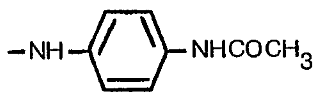 | 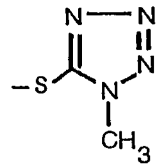 | 1 ap) | 50,5 | 1760, 1660, 1610–1600 | 2,1(s,3H), 3,40(q,2H), 3,95(s,3H), 4,35(q,2H), 4,95(d,1H), 5,50(s,1H), 5,60(d,1H), 6,75(d,2H), 7,3(d,2H), 7,4(d,2H), 7,7(d,2H), 8,3(s,1H) |
| 40 | 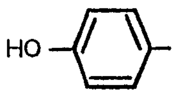 | 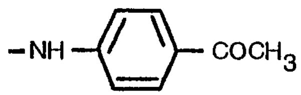 | 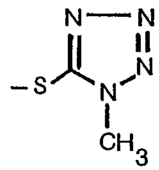 | 1 ao) | 53 | 1760, 1650–70, 1610 | 2,15(s,3H), 3,45(q,2H), 3,95(s,3H), 4,4(q,2H), 4,95(d,1H), 5,50(s,1H), 5,60(d,1H), 6,75(d,2H), 7,40(m,4H), 7,7(d,2H), 8,25(s,1H) |
| 41 | 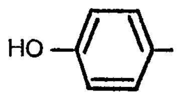 | 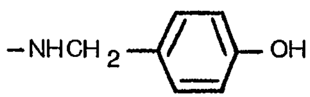 | 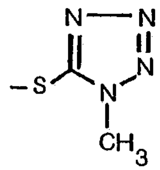 | 1 aj) | 69 | 1760, 1660, 1600 | 3,40(q,2H), 3,95(s,3H), 4,25(breites Signal,2H), 4,40(m,2H), 4,95(d,1H), 5,50(s,1H), 5,60(d,1H), 6,65(m,4H), 7,15(m,4H), 8,05(s,1H) |
| 42 | 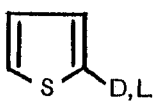 | $-NH(CH_2)_3SO_2NH_2$ | 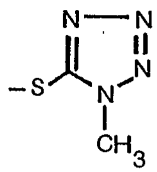 | 1 ad) | 61,5 | 1760, 1660, 1600, 1150 | 1,95(m,2H), 2,8(m,2H), 3,35(m,4H), 3,95(s,3H), 4,4(m,2H), 4,95(dd,1H), 5,45(dd,1H), 5,7(d,1H), 7,0(m,2H), 7,4(d,1H), 8,05(s,1H) |
| 43 | 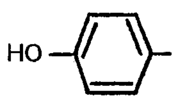 | $-NH(CH_2)_3OH$ | 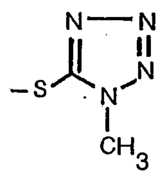 | 1 z) | 59,5 | 1760, 1660, 1605 | 1,85(m,2H), 3,3–3,5(m,4H), 3,65(m,2H), 3,95(s,3H), 4,35(q,2H), 4,95(d,1H), 5,5(s,1H), 5,6(d,1H), 6,75 (d,2H), 7,35(d,2H), 8,05(s,1H) |

Beispiel 44

Natrium-7-{D-$\alpha$-[(4-hydroxy-2-propylamino-5-pyrimidinyl)-ureido]-p-hydroxy-phenylacetamido}-3-acetoxymethyl-ceph-3-em-4-carboxylat

Zu einer Lösung von 3,35 g der Ureidocarbonsäure des Beispiels 1d (0,01 Mol) in 30 ml wasserfreiem Methylenchlorid und 10 ml Dimethylformamid gibt man 1,01 g (0,01 Mol) N-Methylmorpholin. Die Lösung wird auf —15°C abgekühlt und es wird bei dieser Temperatur eine Lösung von 1,1 g (0,01 Mol) Chlorameisensäureäthylester in 5 ml Methylenchlorid zugetropft. Die erhaltene Mischung wird 45 Minuten bei dieser Temperature gehalten. Andererseits gibt man zu einer Suspension von 2,72 g (0,01 Mol) 7-Aminocephalosporansäure in 80 ml wasserfreiem Acetonitril 3 g N,O Bis-trimethylsilylacetamid, wobei eine Lösung erhalten wird. Die Lösung wird auf —20°C abgekühlt und zu der oberen Lösung zugetropft. Danach wird die Mischung bei —10°C 60 Minuten und bei +10°C ebenfalls 60 Minuten lang umgesetzt. Nach dieser Zeit fügt man 5 ml Methanol zu und filtriert von unlöslichem Material ab. Danach wird das Lösungsmittel im Vakuum entfernt. Der Rückstand wird in 100 ml Wasser aufgenommen und die Lösung auf pH 7,5 gestellt. Bei diesem pH-Wert wird zweimal mit Essigsäureäthylester ausgeschüttelt und die organische Phase verworfen. Die wäßrige Phase wird unter Eiskühlung mit verdünnter Salzsäure auf pH 2,9 gestellt, das ausgefallene Produkt abgesaugt, mit wenig Wasser gewaschen und im Vakuum getrocknet. Die wäßrige Lösung wird noch zweimal mit Essigester ausgeschüttelt, die Essigesterphase getrocknet und das Lösungsmittel im Vakuum abdestilliert. Man erhält eine zweite Charge, die laut DC mit dem zunächst ausgefallenen Produkt identisch ist.

Beide Festprodukte werden vereinigt und in 80 ml trockenem Methanol mit der berechneten Menge Natriumäthylhexanoat gelöst. Es wird von etwas unlöslichem Produkt abfiltriert und bis zur vollständigen Fällung Äther hinzugefügt. Das ausgefallene Festprodukt wird abgesaugt und getrocknet.

Ausbeute: 3,18 g Natriumsalz (53%),

IR-Spektrum: 1760, 1655, 1615, 1545 $cm^{-1}$;

NMR-Spektrum (DMSO + $CD_3OD$) Signale bei ppm: 1,0 (t, 3H), 1,65 (q, 2H), 2,05 (s, 3H), 3,2 (q, 3H), 3,45 (q, 2H), 4,80 (q, 2H+d, 1H), 5,50 (s, 1H), 5,65 (d, 1H), 6,75 (d, 2H), 7,30 (d, 2H), 8,05 (s, 1H).

Beispiel 45

Natrium-7-{D-$\alpha$-[(4-hydroxy-2-(4'-hydroxycyclohexylamino)-5-pyrimidinyl)-ureido]-p-hydroxy-phenylacetamido}-3-carbamoyloxymethyl-ceph-3-em-4-carboxylat

Herstellung analog Beispiel 44, ausgehend von 1,32 g (0,005 Mol) 7-Amino-3-carbamoyloxy-methyl-ceph-3-em-4-carbonsäure sowie 2,02 g der Ureidocarbonsäure des Beispiels 1t (0,005 Mol).

Ausbeute an Natriumsalz: 1,47 g (41%),

IR-Spektrum: 1760, 1660, 1610, 1540 $cm^{-1}$;

NMR-Spektrum (DMSO + $CD_3OD$) Signale bei ppm: 1,8 (m, 8H), 3,55 (q, 2H), 3,6—4,1 (m, 1H+1H), 4,8 (breit, 2H+1H), 5,45 (s, 1H), 5,65 (d, 1H), 6,85 (d, 2H), 7,35 (d, 2H), 8,0 (s, 1H).

Beispiel 46

Natrium-7-{D,L-$\alpha$-[(2-cyclopropyl-4-hydroxy-5-pyrimidinyl)-ureido]-2-furylacetamido}-3-[(1-methyl-tetrazol-5-yl)-thiomethyl]-ceph-3-em-4-carboxylat

Herstellung analog Beispiel 44, ausgehend von 310 mg (0,001 Mol) der Ureidocarbonsäure des Beispiels 1c) mit 330 mg (0,001 Mol) 7-Amino-3-[(1-methyl-tetrazol-5-yl)-thiomethyl]-ceph-3-em-4-carbonsäure.

Ausbeute 345 mg Natriumsalz (53,5%),

IR-Spektrum: 1760, 1655, 1610, 1540 $cm^{-1}$;

NMR-Spektrum (DMSO + $CD_3OD$) Signale bei ppm: 1,0 (m, 4H), 1,95 (m, 1H), 3,50 (q, 2H), 3,95 (s, 3H), 4,35 (q, 2H), 4,95 (dd, 1H), 5,45 (dd, 1H), 5,70 (d, 1H), 6,4 (breites d, 2H), 7,6 (s, 1H), 8,45 (s, 1H).

Beispiel 47

Natrium-7-{D-$\alpha$-[4-hydroxy-2-isopropylamino-5-pyrimidinyl)-ureido]-p-hydroxyphenylacetamido}-3-[(2-methylamino-thiadiazol-5-yl)-thiomethyl]-ceph-3-em-4-carboxylat

Herstellung analog Beispiel 44, ausgehend von 670 mg (0,002 Mol) der Ureidocarbonsäure des Beispiels 1e) sowie 720 mg (0,002 Mol) 7-Amino-3[(2-methylamino-thiadiazol-5-yl)-thiomethyl]-ceph-3-em-4-carbonsäure.

Ausbeute: 870 mg Natriumsalz (62%),

IR-Spektrum: 1760, 1665, 1615, 1545 $cm^{-1}$;

NMR-Spektrum (DMSO + $CD_3OD$) Signale bei ppm: 1,15 (d, 6H), 3,0 (s, 3H), 3,65 (q, 2H), 3,95 (m, 1H), 4,15 (m, teilweise verdeckt durch LM = 2H), 5,0 (d, 1H), 5,55 (s, 1H), 5,70 (d, 1H), 6,85 (d, 2H), 7,35 (d, 2H), 8,05 (s, 1H).

Beispiel 48

Natrium-7-{D-$\alpha$-[(2-dimethylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 44, ausgehend von 1,0 g der Ureidocarbonsäure des Beispiels 1i)

(0,0031 Mol) und 1,0 g des in Beispiel 16 eingesetzten Cephalosporinderivate (0,0031 Mol.).

Ausbeute: 1,08 g Natriumsalz (55%),

IR-Spektrum: 1760, 1660, 1615, 1545 $cm^{-1}$;

NMR-Spektrum (DMSO + $CD_3OD$) Signale bei ppm: 3,0 (d, 6H), 3,5 (q, 2H), 3,95 (s, 3H), 4,35 (q, 2H), 4,85 (d, 1H), 5,45 (s, 1H), 5,65 (d, 1H), 6,70 (d, 2H), 7,3 (d, 2H), 8,05 (s, 1H).

Analog wurde Synthetisiert:

Natrium-7-{D-$\alpha$-[(2-diäthylamino-4-hydroxy-5-pyrimidinyl)-ureido]-2-furylacetamido-}-3-[(tetrazol-5-yl)-thiomethyl]-ceph-3-em-4-carboxylat

Beispiel 49

Natrium-7-{D-$\alpha$-[(4-hydroxy-isobutylamino-5-pyrimidinyl)-ureido]-p-hydroxy-phenylacetamido}-3-[(2-acetylamino-thiadiazol-5-yl)-thiomethyl]-ceph-3-em-4-carboxylat

Analog Beispiel 44, ausgehend von 370 mg (0,0011 Mol) der Ureidocarbonsäure des Beispiels 1j) sowie 390 mg (0,001 Mol) 7-Amino-3-[2-acetylamino-thiadiazol-5-yl)-thiomethyl]-ceph-3-em-4-carbonsäure.

Ausbeute: Natriumsalz 275 mg (36%),

IR-Spektrum: 1760, 1655, 1610, 1540 $cm^{-1}$;

NMR-Spektrum (DMSO + $CD_3OD$) Signale bei ppm: 0,9 (d, 6H), 1,8 (m, 1H), 2,3 (s, 3H), 3,1 (m, 2H), 3,70 (q, 2H), 4,25 (q, 2H), 5,0 (d, 1H), 5,5 (s, 1H), 5,65 (d, 1H), 6,85 (d, 2H), 7,4 (d, 2H), 8,0 (s, 1H).

Beispiel 50

Natrium-7-{D-$\alpha$-[(4-hydroxy-2-isobutylamino-5-pyrimidinyl)-ureido]-p-hydroxy-phenylacetamido}-3-[1-methyl-tetrazol-5-yl)-thiomethyl]-ceph-3-em-4-carboxylat

Herstellung analog Beispiel 44, ausgehend von 1,11 g (0,003 Mol) der Ureidocarbonsäure des Beispiels 1j) sowie 1 g des in Beispiel 46 eingesetzten Cephalosporinderivats (0,003 Mol).

Ausbeute an Natriumsalz 1,02 g (52%),

NMR-Spektrum (DMSO + $CD_3OD$) Signale bei ppm: 0,9 (d, 6H), 1,85 (m, 1H), 3,1 (m, 2H), 3,5 (q, 2H), 3,90 (s, 3H), 4,35 (q, 2H), 4,90 (d, 1H), 5,45 (s, 1H), 5,65 (d, 1H), 6,75 (d, 2H), 7,35 (d, 2H), 8,05 (s, 1H).

Analog wurde synthetisiert:

Natrium-7-{D-$\alpha$-[(4-hydroxy-2-isobutylamino-5-pyrimidinyl)-ureido]-p-hydroxy-phenylacetamido}-3-[(tetrazol-5-yl)-thiomethyl]-ceph-3-em-4-carboxylat

Beispiel 51

Natrium-7-{D-$\alpha$-[(2-(3'-methylallylamino)-4-hydroxy-5-pyrimidinyl)-ureido]-p-hydroxy-phenylacetamido}-3-[(1-methyltetrazol-5-yl)-thiomethyl]-ceph-3-em-4-carboxylat

Dieses Cephalosporin wird analog Beispiel 44 hergestellt, indem man von 3,67 g (0,01 Mol) der Ureidocarbonsäure des Biespiels 1k sowie 3,3 g des in Beispiel 16 eingesetzten Cephalosporinderivats ausgeht.

Ausbeute: Natriumsalz: 3,4 g (49%),

IR-Spektrum: 1760, 1670, 1610, 1550 $cm^{-1}$;

NMR-Spektrum (DMSO + $CD_3OD$) Signale bei ppm: 1,7 (d, 3H), 3,5 (1, 2H), 3,8 (breites Signal, 2H), 3,90 (s, 3H), 4,35 (überlagert durch LM), 4,85 (d, 1H), 5,4 (s, 2H), 5,55 (m, 2+1H), 6,7 (d, 2H), 7,25 (d, 2H), 8,0 (s, 1H).

Analog wurden synthetisiert:

Natrium-7-{D,L-$\alpha$-[(2-(3'-methylallylamino)-4-hydroxy-5-pyrimidinyl)-ureido]-2-furylacetamido}-3-[(1-methyltetrazol-5-yl)-thiomethyl]-ceph-3-em-4-carboxylat

Natrium-7-{D-$\alpha$-[(2-(3'-methylallylamino)-4-hydroxy-5-pyrimidinyl)-ureido]-p-hydroxy-phenylacetamido}-3-carbamoyloxymethyl-ceph-3-em-4-carboxylat

Natrium-7-{D,L-$\alpha$-[(4-hydroxy-2-(2'-methylallylamino)-5-pyrimidinyl)-ureido]-m,p-dihydroxy-phenylacetamido}-3-[(1-methyltetrazol-5-yl)-thiomethyl]-ceph-3-em-4-carboxylat.

Beispiel 52

Natrium-7-{D-$\alpha$-[(2-cyclopentylamino-4-hydroxy-5-pyrimidinyl)-ureido]-p-hydroxy-phenylacetamido}-3-[1-methyl-tetrazol-5-yl)-thiomethyl]-ceph-3-em-4-carboxylat

Herstellung analog Beispiel 44, ausgehend von 383 mg (0,001 Mol) der Ureidocarbonsäure des Beispiels 1n) sowie 328 mg (0,001 Mol) des in Beispiel 46 eingesetzten Cephalosporinderivates.

Ausbeute: 425 mg Natriumsalz (59%),

IR-Spektrum: 1760, 1655, 1610, 1540 $cm^{-1}$;

NMR-Spektrum (DMSO + $CD_3OD$) Signale bei ppm: 1,6 (m, 8H), 3,5 (q, 2H), 3,95 (s, 3H), 4,1 (m, 1H, verdeckt durch LM), 4,40 (q, 2H), 4,95 (d, 1H), 5,45 (s, 1H), 5,55 (d, 1H), 6,75 (d, 2H), 7,30 (d, 2H), 8,0 (s, 1H).

Analog wurde hergestellt:

Natrium-7-{D-$\alpha$-[(2-cyclopentylamino-4-hydroxy-5-pyrimidinyl)-ureido]-p-hydroxy-phenylacetamido}-3-[(2-methyl-thiadiazol-5-yl)-thiomethyl]-ceph-3-em-4-carboxylat

Beispiel 53

Natrium-7-{D,L-$\alpha$-[(2-cyclopentylamino-4-hydroxy-5-pyrimidinyl)-ureido]-2-thienyl-acetamido}-3-[(1-methyl-tetrazol-5-yl)-thiomethyl]-ceph-3-em-4-carboxylat

Herstellung analog Beispiel 44, ausgehend von 760 mg (0,002 Mol) der Ureidocarbonsäure des Beispiels 10) sowie 655 mg (0,002 Mol) des Cephalosporinderivates, das auch in Beispiel 46 eingesetzt wurde.

Ausbeute 650 mg Natriumsalz (46%),

IR-Spektrum: 1760, 1665, 1620, 1550 $cm^{-1}$;

NMR-Spektrum (DMSO + $CD_3OD$) Signale bei ppm: 1,65 (m, 8H), 3,45 (q, 2H), 3,95 (s, 3H), 4,05 (m, 1H), 4,35 (q, 2H), 4,9 (dd, 1H), 5,5 (dd, 1H), 5,7 (d, 1H), 6,9 (breit, 2H), 7,30 (d, 1H), 8,05 (s, 1H).

Analog wurde hergestellt:

Natrium-7-{D,L-$\alpha$-[(2-cyclopentylamino-4-hydroxy-5-pyrimidinyl)-ureido]-3-thienyl-acetamido}-3-[(1-methyl-tetrazol-5-yl)-thiomethyl]-ceph-3-em-4-carboxylat,

Natrium-7-{D,L-$\alpha$[(4-hydroxy-2-(4'-hydroxycyclohexylamino)-5-pyrimidinyl)-ureido]-2-furyl-acetamido}-3-[(1-methyl-tetrazol-5-yl)-thiomethyl]-ceph-3-em-4-carboxylat.

Beispiel 54

Natrium-7-{D-$\alpha$-[(2-(2'-äthylmercaptoäthylamino)-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 44, ausgehend von 400 mg der Ureidocarbonsäure des Beispiels 1w) (0,001 Mol) sowie 328 mg (0,001 Mol) der 7-Amino-[(1-methyl-tetrazol-5-yl)-thiomethyl]-ceph-3-em-4-carbonsäure.

Ausbeute an Natriumsalz: 260 mg (35,5%),

IR-Spektrum: 1760, 1655, 1610, 1550 $cm^{-1}$;

NMR-Spektrum (DMSO + $CD_3OD$) Signale bei ppm: 1,3 (d, 3H), 2,7 (m, 4H), 3,5 (q, 2H+m, 2H), 3,9 (s, 3H), 4,40 (q, 2H), 4,85 (d, 1H), 5,45 (s, 1H), 5,65 (d, 1H), 6,85 (d, 2H), 7,35 (d, 2H), 8,0 (s, 1H).

Beispiel 55

Natrium-7-{D,L-$\alpha$-[(4-hydroxy-3-(3'-hydroxypropylamino)-5-pyrimidinyl)-ureido]-2-furyl-acetamido-3-[(1-methyl-tetrazol-5-yl)-thiomethyl]-ceph-3-em-4-carboxylat

Ausgehend von 1,68 g (0,005 Mol) der Ureidocarbonsäure des Beispiels 1y) sowie 1,64 g (0,005 Mol) des Cephalosporinderivates, das auch in Beispiel 54 eingesetzt wurde.

Ausbeute an Natriumsalz: 1,49 g (44%),

IR-Spektrum: 1760, 1660, 1610, 1545 $cm^{-1}$;

NMR-Spektrum (DMSO + $CD_3OD$) Signale bei ppm: 1,85 (m, 2H), 3,3 (m, 2H), 3,45 (q, 2H), 3,6 (m, 2H), 3,95 (s, 3H), 4,35 (q, 2H), 4,95 (dd, 1H), 5,45 (dd, 1H), 5,70 (d, 1H), 6,4 (breit, 2H), 7,6 (s, 1H), 8,0 (s, 1H).

Analog wurden hergestellt:

Natrium-7-{D,L-$\alpha$-[(4-hydroxy-2-(3'-hydroxypropylamino)-5-pyrimidinyl)-ureido]-3-thienyl-acetamido}-3-[(1-methyl-tetrazol-5-yl)-thiomethyl]-ceph-3-em-4-carboxylat

Natrium-7-{D,L-$\alpha$-[(4-hydroxy-2-(3'-hydroxypropylamino)-5-pyrimidinyl)-ureido]-3-thienyl-acetamido}-3-[(1-methyl-tetrazol-5-yl)-thiomethyl]-ceph-3-em-4-carboxylat.

Beispiel 56

Natrium-7-{D,L-$\alpha$-[(2-p-chloranilino-4-hydroxy-5-pyrimidinyl)-ureido]-2-furylacetamido}-3-[(1-methyl-tetrazol-5-yl)-thiomethyl]-ceph-3-em-4-carboxylat

Herstellung analog Beispiel 44 ausgehend von 410 mg (0,001 Mol) der Ureidocarbonsäure des Beispiels 1 af) sowie 328 mg (0,001 Mol), des Cephalosporinderivats, das auch in Beispiel 54 eingesetzt wurde.

Ausbeute an Natriumsalz: 360 mg (48%),

IR-Spektrum: 1760, 1655, 1620, 1550 $cm^{-1}$;

NMR-Spektrum (DMSO + $CD_3OD$) Signale bei ppm: 3,50 (q, 2H), 3,95 (s, 3H), 4,35 (q, 2H), 4,85 (d, 1H), 5,45 (s, 1H), 5,65 (d, 1H), 6,85 (d, 2H), 7,35 (d, 2H), 8,30 (s, 1H).

Analog wurden synthetisiert:

Natrium-7-{D,L-$\alpha$-[(2-p-chloranilino-4-hydroxy-5-pyrimidinyl)-ureido]-2-furylacetamido}-3-[(1-methyl-tetrazol-5-yl)-thiomethyl]-ceph-3-em-4-carboxylat

Natrium-7-{D-$\alpha$-[(4-hydroxy-2-p-hydroxyanilino-5-pyrimidinyl)-ureido]-p-hydroxy-phenylacetamido}-3-carbamoyloxymethyl-ceph-3-em-4-carboxylat

Natrium-7-{D-$\alpha$-[(2-cyclopropyl-4-hydroxy-5-pyrimidinyl)-ureido]-p-hydroxy-phenylacetamido}-3-methyl-ceph-3-em-4-carboxylat

Beispiel 57

Natrium-7-{D-$\alpha$-[(2-p-aminosulfonylanilino-4-hydroxy-5-pyrimidinyl)-ureido]-p-hydroxy-phenylacetamido}-3-[(1-methyltetrazol-5-yl)-thiomethyl]-ceph-3-em-4-carboxylat

Die Synthese erfolgt analog Beispiel 44. Man geht aus von 1,0 g (2,05 m Mol) der Ureidocarbonsäure des Beispiel 1 az) die mit Chlorameisensäureäthylester und Triäthylamin aktiviert wird und setzt anschließend mit der äquimolekularen Menge silylierter 7-Amino-3-[(1-methyltetrazol-5-yl)-thiomethyl]-ceph-3-em-4-carbonsäure um.

Die Aufarbeitung erfolgt analog Beispiel 44.

Das erhaltene Produkt ist laut DC und laut seinen spektroskopischen Eigenschaften identisch mit dem in Beispiel 20 erhaltenen.

Die Ausbeute beträgt 57%.

Analog Beispiel 44 wurden weiter die Cephalosporins der folgenden Tabelle synthetisiert:

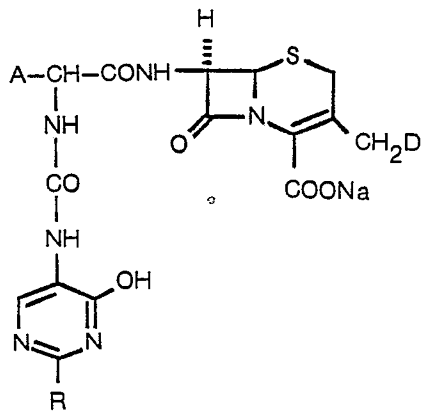

| Beispiel | A | R | D | mit der Ureidocarbonsäure des Beispiels | Ausbeute % | IR cm⁻¹ | NMR-Spektrum ($DMSO/CD_3OD$) Signale bei ppm |
|---|---|---|---|---|---|---|---|
| 58 | 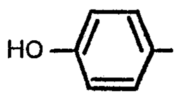 | $-NH(CH_2)_3SO_2NH_2$ | 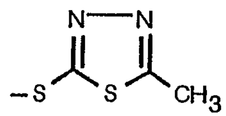 | 1 ab) | 47,5 | 1760, 1660, 1610, 1150 | 2,0(m,2H), 2,7(m,2H), 2,7(s,3H), 3,50(m,4H), 4,45(q,2H), 4,90(d,1H), 5,50(s,1H), 5,65(d,1H), 6,75(d,2H), 7,35(d,2H), 8,05(s,1H) |
| 59 | 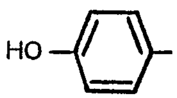 | $-NH(CH_2)_3CONH_2$ | $-OCOCH_3$ | 1 ac) | 42 | 1760, 1655, 1600 | 1,9(t,2H), 2,05(s,3H), 2,5(m,2H), 3,45(m,4H), 4,9(m,2+1H), 5,5(s,1H), 5,6(d,1H), 6,8(d,2H), 7,3(d,2H), 8,05(s,1H) |
| 60 | 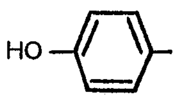 | 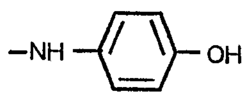 | 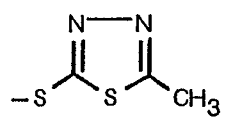 | 1 al) | 51 | 1760, 1660, 1605 | 2,75(s,3H), 3,50(q,2H), 4,45(q,2H), 4,90(d,1H), 5,55(s,2H), 5,65(d,1H), 6,75(d,2H), 7,35(d,2H), 7,45(d,2H), 7,85(d,2H), 8,30(s,1H) |
| 61 | 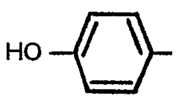 | 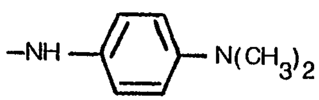 | 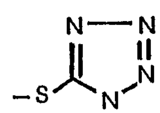 | 1 am) | 34,5 | 1760, 1655, 1595 | 2,95(d,6H), 3,45(q,2H), 4,35(q,2H), 4,95(d,1H), 5,5(s,1H), 5,6(d,1H), 6,80(d,2H), 7,3(d,2H), 7,4(d,2H), 7,7(d,2H), 8,25(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 62 | 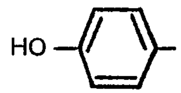 | 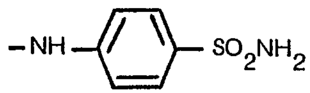 | 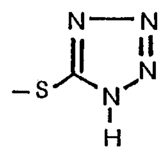 | 1 az) | 68 | 1760, 1660, 1600, 1150 | 3,40(m,2H), 4,4(q,2H), 4,95(d,1H), 5,45(s,1H), 5,55(d,1H), 6,80(d,2H), 7,35(d,2H), 7,7(d,2H), 8,0(d,2H), 8,35(s,1H) |
| 63 | 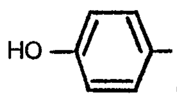 | 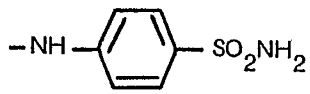 | 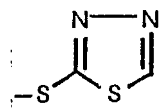 | 1 az) | 60,5 | 1760, 1660, 1605, 1150 | 3,50(q,2H), 4,45(q,2H), 4,90(d,1H), 5,50(s,1H), 5,65(d,1H), 6,75(d,2H), 7,35(d,2H), 7,7(d,2H), 8,0(d,2H), 8,37(s,1H), 9,25(s,1H) |
| 64 | 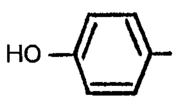 | 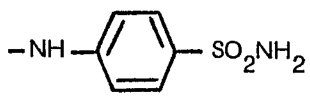 | $-OCONH_2$ | 1 az) | 54 | 1760, 1660, 1610, 1145 | 3,45(q,2H), 4,8(m,2+1H), 5,50(s,1H), 5,60(d,1H), 6,75(d,2H), 7,30(d,2H), 7,7(d,2H), 8,0(d,2H), 8,35(s,1H) |
| 65 | 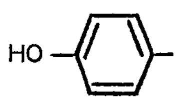 | 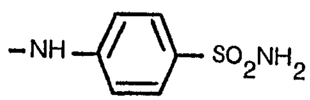 | 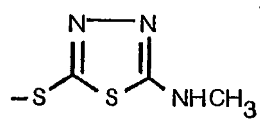 | 1 az) | 69 | 1760, 1650, 1600, 1150 | 2,85(s,3H), 3.4(m,2H), 4,35(q,2H), 4,95(d,1H), 5,5(s,1H), 5,6(d,1H), 6,75(d,2H), 7,3(d,2H), 7,65(d,2H), 7,95(d,2H), 8,35(s,1H), 8,38(s,1H), |
| 66 | 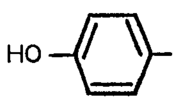 | 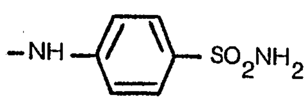 | 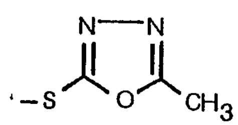 | 1 az) | 46,5 | 1760, 1660, 1600, 1150 | 2,45(s,3H), 3,6(q,2H), 4,2(q,2H), teilweise durch LM verdeckt), 4,90(d,1H), 5,45(s,1H), 5,55 (d,1H), 6,75(d,2H), 7,35(d,2H), 7,65(d,2H), 8,0(d,2H), 8,35(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 67 | 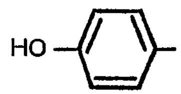 | 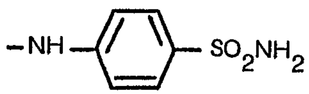 | 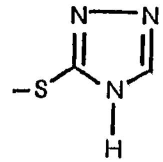 | 1 az) | 53,5 | 1760, 1655, 1600, 1150 | 3,4(q,2H), 4,4(q,2H), 4,95(d,1H), 5,5(s,1H), 5,6(d,1H), 6,75(d,2H), 7,35(d,2H), 7,65(d,2H), 8,0 (d,2H), 8,35(s,1H), 8,36(s,1H) |
| 68 | 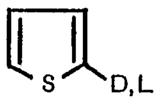 | 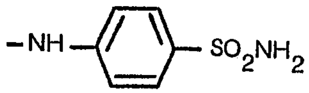 | $-OCONH_2$ | 1 ba) | 51,5 | 1760, 1660, 1605, 1150 | 3,45(q,2H), 4,8(m,2+1H), 5,55(dd,1H), 5,7(d,1H), 6,8–7,2(m,2H), 7,35(m,1H), 7,7(d,2H), 8,0(d,2H), 8,38(s,1H) |
| 69 | 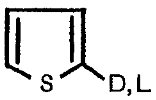 | 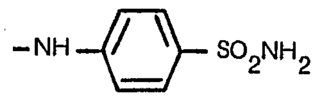 | 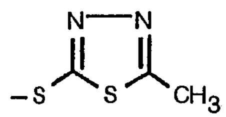 | 1 ba) | 54 | 1760, 1655, 1610, 1150 | 2,7(s,1H), 3,50(q,2H), 4,45(q,2H), 4,9(dd,1H), 5,55(dd,1H), 5,7(d,1H), 6,8–7,2(m,2H), 7,35(m,1H), 7,7(d,2H), 8,0(d,2H), 8,37(s,1H) |
| 70 | 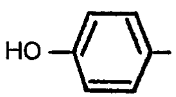 | 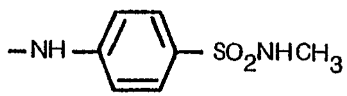 | 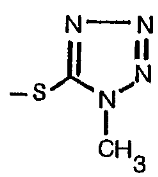 | 1 bf) | 71 | 1760, 1660, 1600, 1150 | 2,40(s,3H), 3,40(m,2H), 3,95(s,3H), 4,35(q,2H), 4,95(d,1H), 5,55(s,1H), 5,60(d,1H), 6,75(d,2H), 7,3(d,2H), 7,80(q,4H), 8,35(s,1H) |
| 71 | 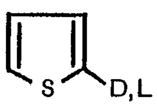 | 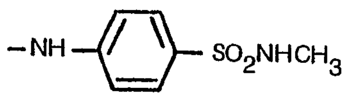 | 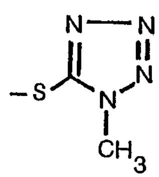 | 1 bf) | 66 | 1760, 1660, 1600, 1145 | 2,42(s,3H), 3,40(m,2H), 3,95(s,3H), 4,4(q,2H), 4,9(dd,1H), 5,55(dd,1H), 5,75(d,1H), 7,05(m,2H), 7,45(m,1H), 7,80(q,2H), 8,35(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 72 | 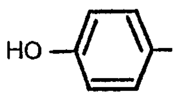 | 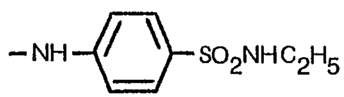 | 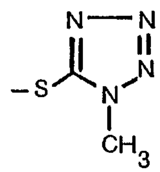 | 1 ar) | 73,5 | 1760, 1665, 1600, 1155 | 1,25(t,3H), 3,3(m,4H), 3,95 (s,3H), 4,4(q,2H), 4,95(d,1H), 5,50(s,1H), 5,60(d,1H), 6,75 (d,2H), 7,35(d,2H), 7,8(q,4H), 8,35(s,1H) |
| 73 | 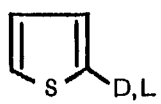 | 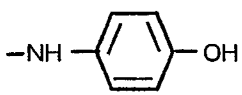 | 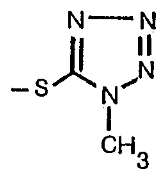 | 1 v) | 66 | 1760, 1660, 1605 | 1,80(m,8H), 3,35(m,2H), 3,5–4,0(m,1+1H,s,3H), 4,35(q,2H), 4,9(dd,1H), 5,50(dd,1H), 5,75(d,1H), 7,0(m,2H), 7,4(m,1H), 8,05(s,1H) |

Beispiel 74

*Natrium-7-{D-α-[(2-cyclopropyl-4-hydroxy-5-pyrimidinyl)-ureido]-p-hydroxy-phenylacetamido}-3-[(7-α-methoxy-3-[(1-methyltetrazol-5-yl-)thiomethyl]-ceph-3-em-4-carboxylat*

Zu einer Lösung von 340 mg (0,001 Mol) der Ureidocarbonsäure des Beispiels 1a) sowie von 525 mg (0,001 Mol) 7-β-Amino-7-α-methoxy-3-[(1-methyl-tetrazol-5-yl-)thiomethyl]-ceph-3-em-4-carbonsäure-diphenylmethylester in 20 ml wasserfreiem Methylenchlorid und 10 ml Dimethylformamid werden 220 mg Dicyclohexylcarbodiimid gegeben. Man rührt 2 Stunden unter Eiskühlung und 4 Stunden bei Raumtemperatur. Ein Dünnschichtchromatogramm des Gemisches zeigt an, daß die Ausgangsprodukte nahezu vollständig verschwunden sind. Dicyclohexylharnstoff wird abfiltriert und die Lösung zur Trockne eingeengt. Der Rückstand wird einer Silicagelsäulenchromatographie unterworfen unter Verwendung eines Gemisches von Methylenchlorid und Methanol im Verhältnis 12:1. Der erhaltene Benzhydrylester wird analog Beispiel 2. mit Trifluoressigsäure/Anisol gespalten und anschließend das Natriumsalz hergestellt.

Ausbeute: 295 mg Natriumsalz (42,5%),

IR-Spektrum: 1760, 1650, 1610, 1540 $cm^{-1}$;

NMR-Spektrum (DMSO + $CD_3OD$) Signale bei ppm: 0,9 (m, 4H), 1,9 (m, 1H), 3,45 (m, 2H), 3,46 (s, 3H), 3,9 (s, 3H), 4,95 (s, 1H), 5,45 (s, 1H), 6,85 (d, 2H), 7,35 (d, 2H), 8,40 (s, 1H).

Beispiel 75

Natrium-7-β-{D-α-[(2-p-aminosulfonylanilino-4-hydroxy-5-pyrimidinyl)-ureido]-p-hydroxy-phenylacetamido}-7-α-methoxy-3-[(1-methyl-tetrazol-5-yl)-thiomethyl]-ceph-3-em-4-carboxylat

In einem Gemisch aus 40 ml wasserfreiem Methylenchlorid und 30 ml wasserfreiem Dimethylformamid werden 2,37 g der Ureidocarbonsäure des Beispiels 1 az) (0,005 Mol) gelöst. Man gibt 525 mg N-Methylmorpholin zu und kühlt auf —20°C ab. Innerhalb von 5 min. tropft man bei dieser Temperatur eine Lösung vom 575 mg Chlorameisensäureäthylester in 5 ml wasserfreiem Methylenchlorid zu. Das Gemisch wird bei —15°C 60 min. gerührt. Bei —15°C wird eine Lösung von 2,62 g 7-β-Amino-7-α-methoxy-3-[(1-methyltetrazol-5-yl)-thiomethyl] - ceph - 3 - em - 4 - carbonsäure - diphenylmethylester (0,005 Mol) in Methylenchlorid zugetropft. Man läßt 60 min. bei —10°C und 120 min. bei +10°C reagieren. Man engt im Vakuum zur Trockne ein und versetzt mit 30 ml Wasser. Das zurückbleibende Festprodukt wird einmal mit 100 ml Methylenchlorid, einmal mit 50 ml Methanol und anschließend mit Äther ausgerührt. Es bleibt ein schwach ockerfarbenes Pulver zurück, das einer Säulenchromatographie an Silicagel mit einem Gemisch von Methylenchlorid/Methanol im Verhältnis 6:1 unterworfen wird.

Die Hauptkomponente wird wie üblich mit 'Trifluoressigsäure/Anisol gespalten und in das Natriumsalz übergeführt.

IR-Spektrum: 1765, 1660, 1600, 1150 $cm^{-1}$;

NMR-Spektrum (DMSO + $CD_3OD$) Signale bei ppm: 3,40 (m, 2H), 3,47 (s, 3H), 3,9 (s, 3H), 4,95 (s, 1H), 5,50 (s, 1H), 6,85 (d, 2H), 7,35 (d, 2H), 7,65 (d, 2H), 8,0 (d, 2H), 8,40 (s, 1H).

Ausgehend vom Amino-Cephalosporinderivat des Beispiels 75 erhält man nach derselben Vorgehensweise folgende Cephalosporine:

Beispiel 76

Natrium-7-β-{D-α-[(4-hydroxy-2-propylamino-5-pyrimidinyl)-ureido]-p-hydroxy-phenylacetamido}-7-α-methoxy-3-[(1-methyltetrazol-5-yl-)thiomethyl]-ceph-3-em-4-carboxylat

mit der Ureidocarbonsäure des Beispiels 1d):

Ausbeute: 38,5%,

IR-Spektrum: 1770, 1670, 1620, 1540 $cm^{-1}$;

NMR-Spektrum (DMSO + $CD_3OD$) Signale bei ppm: 1,0, (t, 3H), 1,6 (q, 2H), 3,2 (t, 2H), 3,50 (q, 2H), 3,50 (s, 3H), 3,95 (s, 3H), 4,35 (1, 2H), 4,95 (s, 1H), 5,50, (s, 1H), 6,85 (d, 2H), 7,3 (d, 2H), 8,0 (s, 1H).

Beispiel 77

Natrium-7-β-{D-α-[(4-hydroxy-2-isopropylamino-5-pyrimidinyl)-ureido]-p-hydroxy-phenylacetamido}-7-α-methoxy-3-[(1-methyltetrazol-5-yl-)thiomethyl]-ceph-3-em-4-carboxylat

mit der Ureidocarbonsäure des Beispiels 1e:

Ausbeute: 46%,

IR-Spektrum: 1765, 1660, 1610, 1550 $cm^{-1}$;

NMR-Spektrum (DMSO + $CD_3OD$) Signale bei ppm: 1,15 (d, 6H), 3,50 (q, 2H), 3,45 (s, 3H), 3,9 (m, 1H+s, 3H), 4,35 (q, 2H), 4,90 (s, 1H), 5,50 (s, 1H), 6,85 (d, 2H), 7,35 (d, 2H), 8,0 (s, 1H).

Beispiel 78

Natrium-7-β-{D,L-α-[(4-hydroxy-2-propylamino-5-pyrimidinyl)-ureido]-2-thienyl-acetamido}-7-α-methoxy-3-[(1-methyl-tetrazol-5-yl-)thiomethyl]-ceph-3-em-4-carboxylat

mit der Ureidocarbonsäure des Beispiels 1h):

Ausbeute: 47%,

IR-Spektrum: 1765, 1655, 1610, 1545 $cm^{-1}$;

NMR-Spektrum (DMSO + $CD_3OD$) Signale bei ppm: 1,0 (5, 3H), 1,65 (q, 2H), 3,2 (m, 2H), 3,50 (q, 2H), 3,50 (s, 3H), 3,90 (s, 3H), 4,30. (q, 2H), 4,90 (s, 1H), 5,75 (s, 1H), 6,9 (breit, 2H), 7,30 (d, 1H), 8,0 (s, 1H).

Beispiel 79

Natrium-7-β-{D-α-[(2-cyclopentylamino-4-hydroxy-5-pyrimidinyl)-ureido]-p-hydroxy-phenylacetamido}-7-α-methoxy-3-[(1-methyltetrazol-5-yl-)thiomethyl]-ceph-3-em-4-carboxylat

mit der Ureidocarbonsäure des Beispiels 1n):

Ausbeute: 52%,

IR-Spektrum: 1765, 1665, 1620, 1550 $cm^{-1}$;

NMR-Spektrum (DMSO + $CD_3OD$) Signale bei ppm: 1,65 (m, 8H), 3,5 (q, 2H), 3,45 (s, 3H), 3,9 (s, 3H), 4,05 (m, verdeckt durch LM), 4,4 (q, 2H), 4,85 (s, 1H), 5,45 (s, 1H), 6,85 (d, 2H), 7,35 (d, 2H), 8,0 (s, 1H).

Beispiel 80

Natrium-7-β-{D-α-[(4-hydroxy-2-(4'-hydroxy-cyclohexylamino)-5-pyrimidinyl)-ureido]-p-hydroxy-phenylacetamido}-7-α-methoxy-3-[(1-methyl-tetrazol-5-yl-)thiomethyl]-ceph-3-em-4-carboxylat

mit der Ureidocarbonsäure des Beispiels 1t):

Ausbeute: 47%,

IR-Spektrum: 1765, 1660, 1615, 1540 $cm^{-1}$;

NMR-Spektrum (DMSO + $CD_3OD$) Signale bei ppm: 1,75 (m, 8H), 3,45 (q, 2H), 3,6—4,0 (breit, m, 1H+1H), 3,50 (s, 3H), 3,95 (s, 3H), 4,35 (q, 2H), 4,85 (s, 1H), 5,45 (s, 1H), 6,85 (d, 2H), 7,35 (d, 2H), 8,05 (s, 1H).

Analog Beispiel 75 wurden, ausgehend vom entsprechenden Cephalosporinderivat noch folgende 7-α-Methoxycephalosporine synthetisiert:

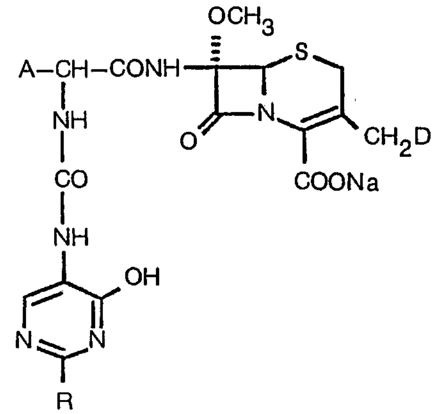

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 81 | 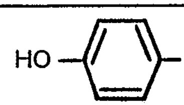 | $-NH(CH_2)_3OH$ | 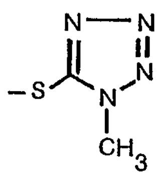 | 1 z) | 44,5 | 1765, 1660, 1600 | 1,85(m,2H), 3,2–3,6(m,6H), 3,45(s,3H), 3,95(s,3H), 4,4(q,2H), 4,95(s,1H), 5,5(s,1H), 6,75(d,2H), 7,35(d,2H), 8,05(s,1H) |
| 82 | 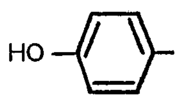 | $-NH(CH_2)_3SO_2NH_2$ | 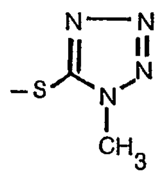 | 1 ab) | 57 | 1765, 1660, 1600, 1150 | 1,95(m,2H), 2,75(m,2H), 3,35(m,4H), 3,50(s,3H), 3,95(s,3H), 4,40(q,2H), 4,95(s,1H), 5,55(s,1H), 6,85(d,2H), 7,35(d,2H), 8,05(s,1H) |
| 83 | 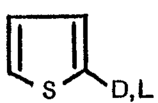 D,L | $-NH(CH_2)_3SO_2NH_2$ | 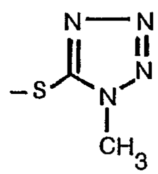 | 1 ad) | 38 | 1765, 1655, 1605, 1145 | 1,9(m,2H), 2,8(m,2H), 3,4(m, 4H), 3,45(s,3H), 3,95(s,3H), 4,35(q,2H), 5,00(d,1H), 5,75(d,1H), 7,0(m,2H), 7,4(d,1H), 8,08(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 84 | 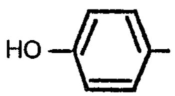 | $-NH(CH_2)_3CONH_2$ | 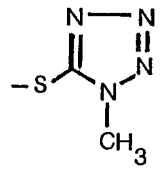 | 1 ac) | 46,5 | 1765, 1650, 1600 | 1,9(t,2H), 2,5(m,2H), 3,45(m,4H + s,3H), 3,90(s,3H), 4,3(m,2H), 4,95(s,1H), 5,55(d,1H), 6,80(d,2H), 7,35(d,2H), 8,05(s,1H) |
| 85 | 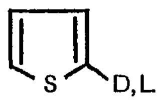 | 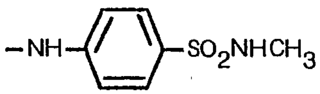 | 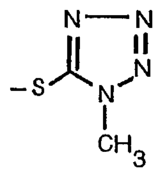 | 1 bf) | 51,5 | 1765, 1660, 1600, 1150 | 2,4(s,3H), 3,45(m,2H+s,3H), 3,95(s,3H), 4,35(q,2H), 4,95(d,1H), 5,7(d,1H), 6,85(d,2H), 7,35(d,2H), 7,85(q,4H), 8,37(s,1H) |
| 86 | 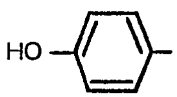 | 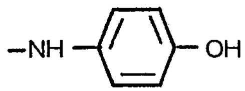 | 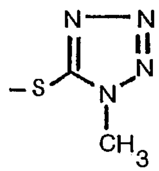 | 1 al) | 41 | 1765, 1660, 1610 | 3,35(m,2H), 3,50(s,3H), 3,95(s,3H), 4,35(q,2H), 4,95(s,1H), 5,50(s,1H), 6,75(d,2H), 7,35(d,2H), 7,45(d,2H), 7,8(d,2H), 8,30(s,1H) |
| 87 | 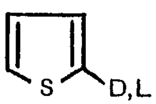 | 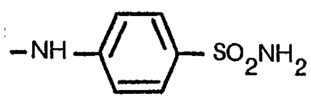 | 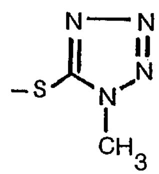 | 1 ba) | 49,5 | 1765, 1655, 1605, 1155 | 3,45(q,2H), 3,50(s,3H), 3,95(s,3H), 4,4(q,2H), 5,0(d,1H), 5,75(d,1H), 7,0(m,2H), 7,4(d,1H), 7,7(d,2H), 8,0(d,2H), 8,38(s,1H) |
| 88 | 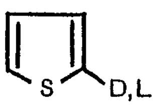 | 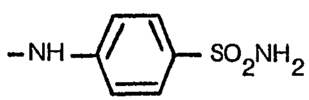 | $-OCONH_2$ | 1 ba) | 50 | 1765, 1660, 1610 | 3,45(s,3H), 3,50(q,2H), 4,8(m,2+1H), 5,70(d,1H), 7,0(m,2H), 7,4(d,1H), 7,7(d,2H), 8,0(d,2H), 8,36(s,1H) |

| Beispiel | A | R | D | mit der Ureidocarbonsäure des Beispiels | Ausbeute % | IR $cm^{-1}$ | NMR-Spektrum ($DMSO/CD_3OD$) Signale bei ppm |
|---|---|---|---|---|---|---|---|
| 89 | 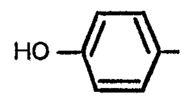 | 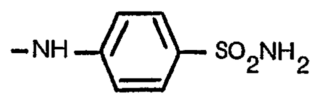 | $-OCOCH_3$ | 1 az) | 61,5 | 1765, 1655, 1600 | 2,05(s,3H), 3,55(q,2H), 3,45(s,3H), 4,85(q,2H+s,1H), 5,50(s,1H), 6,85(d,2H), 7,35(d,2H), 7,7(d,2H), 8,0(d,2H), 8,36(s,1H), |
| 90 | 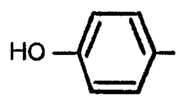 | 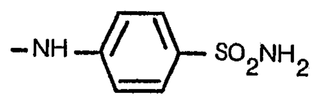 | $-OCONH_2$ | 1 az) | 44 | 1765, 1660, 1610 | 3,45(s,3H), 3,50(q,2H), 4,8(m,2+1H), 5,50(s,1H), 6,85(d,2H), 7,35(d,2H), 7,7(d,2H), 7,95(d,2H), 8,37(s,1H) |
| 91 | 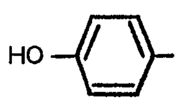 | 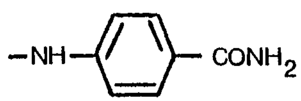 | 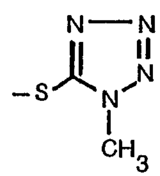 | 1 as) | 56 | 1765, 1670, 1600 | 3,4(m,2+3H), 3,95(s,3H), 4,35(q,2H), 5,50(s,1H), 6,85(d,2H), 7,7(dd,4H), 8,32(s,1H) |

Beispiel 92

Natrium-7-β-{D-α-[(4-hydroxy-2-p-methylaminosulfonylanilino-5-pyrimidinyl)-ureido]-p-hydroxy-phenylacetamido}-7-α-methoxy-3-[(1-methyltetrazol-5-yl)-thiomethyl]-ceph-3-em-4-carboxylat

In einem Gemisch von 40 ml wasserfreiem Methanol und 40 ml wasserfreiem Chloroform werden 965 mg Diphenylmethyl - 7 - β - {D - α - [(4 - hydroxy - 2 - p - methylaminosulfonylanilino - 5 - pyrimidinyl) - ureido] - p - hydroxy - phenylacetamido} - 3 - [(1 - methyl - tetrazol - 5 - yl) - thiomethyl] - ceph - 3 - em - 4 - carboxylat (Zwischenstufe des Beispiels 70) gelöst. Man kühlt auf —70°C ab und gibt bei dieser Temperatur 5 ml einer methanolischen Lösung von Lithiummethoxid zu (1,5 m Mol/ml). Man rührt 3 min. bei —70°C und gibt dann 0,18 ml t-Butylhypochlorit zu. Man rührt weitere 20 min. und fügt dann zunächst 0,3 ml Essigsäure und dann 0,1 ml Triäthylphosphit zu der Lösung hinzu. Dann läßt man die Temperatur auf 20°C ansteigen. Man engt zur Trockne ein, rührt kurz mit Wasser bei pH 7 aus, saugt ab und reinigt den Rückstand durch Säulenchromatographie über eine Merck-Fertigsäule (Silicagel, Methylenchlorid-Methanol 7:1).

Man erhält 480 mg des Diphenylmethylesters der gewünschten Verbindung, der analog den vorhergehenden Beispielen mit Trifluoressigsäure/Anisol gespalten wird und in das Natriumsalz überführt wird.

IR-Spektrum: 1765, 1660, 1605 $cm^{-1}$;

NMR-Spektrum (DMSO + $CD_3OD$) Signale bei ppm: 2,45 (s, 3H), 3,25—3,50 (m, 2H+s, 3H), 3,95 (s, 3H), 4,30 (q, 2H), 4,95 (s, 1H), 5,45 (s, 1H), 6,75 (d, 2H), 7,30 (d, 2H), 7,80 (q, 4H), 8,35 (s, 1H).

Beispiel 93

Diastereomerentrennung des Cephalosporins des Beispiels 22:

Natrium-7-{D,L-α-[(2-aminosulfonylanilino-4-hydroxy-5-pyrimidinyl)-ureido]-2-thienylacetamido}-3-[(1-methyltetrazol-5-yl)-thiomethyl]-ceph-3-em-4-carboxylat

Die in Beispiel 22 erhaltene D,L-Verbindung wird durch präparative Flüssigkeitschromatographie (HPLC) unter Verwendung einer 10 μm Umkehrphase-C 8-Säule (Lichrosorb RP 18- Fa. Merck) in die D und L Form getrennt. Als Eluat wird eine Lösung von 6 g Diammoniumphosphat in 800 ml Wasser und 400 ml Methanol verwendet. Das Eluat wird bei 269 nm (UV) verfolgt. Die getrennten Lösungen werden wie folgt aufgearbeitet:

Das Methanol wird im Vakuum abgezogen und die zurückbleibende wäßrige Lösung mit verdünnter Salzsäure auf pH 3,0 gestellt. Das ausfallende Festprodukt wird abgesaugt und getrocknet.

Diastereomeres I: NMR-Spektrum (DMSO + $CD_3OD$) Signale bei ppm: 3,4 (m, 2H), 3,95 (s, 3H), 4,4 (m, 2H), 5,0 (d, 1H), 5,6 (d, 1H), 5,75 (s, 1H), 7,0 (m, 2H), 7,4 (d, 1H), 7,7 (d, 2H), 8,0 (d, 2H), 8,37 (s, 1H).

Diastereomeres II: NMR-Spektrum (DMSO + $CD_3OD$) Signale bei ppm: 3,4 (m, 2H), 3,95 (s, 3H), 4,45 (m, 2H), 5,05 (d, 1H), 5,65 (d, 1H), 5,80 (s, 1H), 7,0 (m, 2H), 7,35 (d, 1H), 7,7 (d, 2H), 8,0 (d, 2H), 8,37 (s, 1H).

Analog diesem Beispiel gelingt es, auch folgende D,L Verbindungen in die D und L- Isomeren aufzutrennten:

Natrium-7-{D,L-α-[(2-cyclopropyl-4-hydroxy-5-pyrimidinyl)-ureido]-2-thienylacetamido}-3-[(1-methyl-tetrazol-5-yl)-thiomethyl]-ceph-3-em-4-carboxylat

Natrium-7-{D,L-α-[(4-hydroxy-2-(4'-hydroxy-cyclohexylamino)-5-pyrimidinyl)-ureido]-2-thienylacetamido}-3-[(1-methyltetrazol-5-yl)-thiomethyl]-ceph-3-em-4-carboxylat

Natrium-7-{D,L-α-[4-hydroxy-2-propylamino-5-pyrimidinyl)-ureido]-2-thienyl-acetamido}-7-α-methoxy-3-[(1-methyltetrazol-5-yl-)thiomethyl]-ceph-3-em-4-carboxylat

Natrium-7-{D,L-α-[(2-(3'-aminosulfonylpropylamino)-4-hydroxy-5-pyrimidinyl)-ureido]-2-thienylacetamido}-3-[(1-methyltetrazol-5-yl)-thiomethyl]-ceph-3-em-4-carboxylat

Natrium-7-β-{D,L-α-[(2-p-aminosulfonylanilino-4-hydroxy-5-pyrimidinyl)-ureido]-2-thienylacetamido}-7-α-methoxy-3-[(1-methyl-tetrazol-5-yl)-thiomethyl]-ceph-3-em-4-carboxylat

Natrium-7-β-{D,L-α-[(2-p-aminosulfonylanilino-4-hydroxy-5-pyrimidinyl)-ureido]-2-thienyl-acetamido}-7-α-methoxy-3-aminocarbonyloxymethyl-ceph-3-em-4-carboxylat

Natrium-7-{D,L-α-[(2-p-aminosulfonylanilino-4-hydroxy-5-pyrimidinyl)-ureido]-2-furylacetamido}-3-[(1-methyl-tetrazol-5-yl)-thiomethyl]-ceph-3-em-4-carboxylat

Beispiel 94

Natrium-7-{D-α-[(4-hydroxy-5-pyrimidinyl)-ureido]-phenylacetamido]-3-methyl-ceph-3-em-4-carboxylat

Eine Suspension von 2,26 g Cephalexin-Monohydrat (0,0073 Mol) in 80 ml Tetrahydrofuran und 20 ml Wasser wird mit Triäthylamin unter Eiskühlung in Lösung gebracht.

800 mg (0,0073 Mol) 5-Amino-4-hydroxy-pyrimidin werden in Tetrahydrofuran gelöst, mit 1 ml Triäthylamin versetzt und unter Eiskühlung zu 750 mg Phosgen, gelöst in 18 ml Tetrahydrofuran, getropft. Das entstandene Gemisch wird im Vakuum auf 40 ml eingeengt und unter Eiskühlung zu der oben hergestellten Lösung getropft. Dabei wird der pH mit Hilfe von Triäthylamin auf 7,5 gehalten. Die erhaltene Lösung wird bei 5°C eine Stunde und bei Raumtemperatur eine weitere Stunde gerührt. Nach dieser Zeit wird Tetrahydrofuran im Vakuum abgezogen, der Rückstand mit 20 ml Wasser verdünnt und zweimal mit Essigester ausgeschüttelt. Dann wird die wäßrige Phase mit Essigester überschichtet und unter Kühlen und Rühren langsam ein pH-Wert von 2,5 eingestellt. Die Essigesterschicht wird abgetrennt, die wäßrige Phase noch einmal mit Essigester ausgeschüttelt, die beiden organischen Phasen vereinigt und das Lösemittel im Vakuum, abdestilliert. Auf übliche Weise wird das Natriumsalz hergestellt.

Ausbeute: 2,49 g (68%),

IR-Spektrum: 1760, 1655, 1610, 1540 $cm^{-1}$;

NMR-Spektrum (DMSO + $CD_3OD$) Signale bei ppm: 2,0 (s, 3H), 3,40 (q, 2H), 5,05 (d, 1H), 5,45 (s, 1H), 5,65 (d, 1H), 7,45 (m, 5H), 8,1 (s, 1H), 8,50 (s, 1H).

Setzt man wie im Beispiel 94 beschrieben, Cephalexin-monohydrat ein, so erhält man folgende Cephalosporine:

Biespiel 95

Natrium-7-{D-$\alpha$-[(2-cyclopropyl-4-hydroxy-5-pyrimidinyl)-ureido]-phenyl-acetamido}-3-methyl-ceph-3-em-4-carboxylat

mit dem Umsetzungsprodukt von 5-Amino-2-cyclopropyl-4-hydroxypyrimidin mit Phosgen:

Ausbeute: 81%,

IR-Spektrum: 1760, 1660, 1610, 1540 $cm^{-1}$;

NMR-Spektrum (DMSO + $CD_3OD$) Signale bei ppm: 1,0 (m, 4H), 1,9 (m, 1H), 2,0 (s, 3H), 3,4 (q, 2H), 4,95 (d, 1H), 5,45 (s, 1H), 5,60 (d, 1H), 7,4 (s, 5H), 8,4 (s, 1H).

Beispiel 96

Natrium-7-{D-$\alpha$-[(4-hydroxy-2-(4'-hydroxy-cyclohexylamino)-5-pyrimidinyl)-ureido]-phenyl-acetamido}-3-methyl-ceph-3-em-4-carboxylat

Mit dem Umsetzungsprodukt von 5-Amino-(4'-hydroxy-cyclohexylamino)-pyrimidin mit Phosgen. (nach Silylierung).

Ausbeute: 64%,

IR-Spektrum: 1760, 1660, 1610, 1550 $cm^{-1}$;

NMR-Spektrum (DMSO + $CD_3OD$) Signale bei ppm: 1,8 (m, 8H), 2,05 (s, 3H), 3,4 (q, 2H), 3,6—4,0 (breites m, 1H+1H), 4,95 (d, 1H), 5,45 (s, 1H), 5,65 (d, 1H), 7,4 (s, 5H), 8,05 (s, 1H).

Beispiel 97

Natrium-7-{D-$\alpha$-[(2-p-chlorbenzylamino-4-hydroxy-5-pyrimidinyl)-ureido]-phenyl-acetamido}-3-methyl-ceph-3-em-4-carboxylat

Mit dem Umsetzungsprodukt von 5-Amino-2-p-chlorbenzylamino-4-hydroxy-pyrimidin mit Phosgen:

Ausbeute: 71%,

IR-Spektrum: 1760, 1655, 1610, 1545 $cm^{-1}$;

NMR-Spektrum (DMSO + $CD_3OD$) Signale bei ppm: 2,0 (s, 3H), 3,35 (q, 2H), 4,95 (d, 1H), 5,4 (s, 1H), 5,65 (d, 1H), 6,8 (d, 2H), 7,4 (m, 7H), 8,0 (s, 1H).

Beispiel 98

Natrium-7-{D-$\alpha$-[(2,4-dihydroxy-5-pyrimidinyl)-ureido]-p-hydroxy-phenyl-acetamido}-3-acetoxymethyl-ceph-3-em-4-carboxylat

635 mg (0,005 Mol) 5-Amino-2,4-dihydroxy-pyrimidin werden in 50 ml Tetrahydrofuran suspendiert und bis zur Lösung mit Trimethylsilyldiäthylamin behandelt.

Das Tetrahydrofuran wird im Vakuum abdestilliert. Das zurückbleibende Produkt wird in 30 ml Tetrahydrofuran gelöst und unter Eiskühlung zu einer Lösung von 500 mg Phosgen in Tetrahydrofuran getropft. Anschließend wird zur Entfernung von nicht umgesetztem Phosgen Stickstoff durch die Lösung geblasen.

Die weitere Umsetzung mit 7 - [D - $\alpha$ - Amino - p - hydroxyphenyl - acetamido] - 3 - acetoxymethyl - ceph - 3 - em - 4 - carbonsäure erfolgt analog Beispiel 33.

Ausbeute: 830 mg Natriumsalz (29%),

IR-Spektrum: 1760, 1665, 1505, 1545 $cm^{-1}$;

NMR-Spektrum (DMSO + $CD_3OD$) Signale bei ppm: 2,05 (s, 3H), 3,55 (q, 2H), 4,8 (m, 2+1H), 5,45 (s, 1H), 5,65 (d, 2H), 6,75 (d, 2H), 7,3 (d, 2H), 8,15 (s, 1H).

Beispiel 99

Natrium-7-{D-$\alpha$-[(2-amino-4-hydroxy-5-pyrimidinyl)-ureido]-phenyl-acetamido}-3-methyl-ceph-3-em-4-carboxylat

Aus dem Phosgen-Addukt von 2,5-Diamino-4-hydroxy-pyrimidin (nach Silylierung, wie in Beispiel 98 beschrieben) und dem Cephalexin-monohydrat. Das entstandene Cephalosporin wird bei pH 3,0 aus Wasser ausgefällt, abgesaugt, getrocknet und wie oben beschrieben, in das Natriumsalz übergeführt.

Ausbeute: 26%,

IR-Spektrum: 1760, 1665, 1615, 1545 $cm^{-1}$;

NMR-Spektrum (DMSO + $CD_3OD$) Signale bei ppm: 2,0 (s, 3H), 3,45 (q, 2H), 4,90 (d, 1H), 5,40 (s, 1H), 5,60 (d, 2H), 7,4 (m, 5H), 8,0 (s, 1H).

Beispiel 100

Natrium-7-{D-$\alpha$-[(4-hydroxy-2-methylamino-5-pyrimidinyl)-ureido]-p-hydroxy-phenyl-acetamido}-3-[(1-methyl-tetrazol-5-yl)-thiomethyl]-ceph-3-em-4-carboxylat

Herstellung analog Beispiel 98 ausgehend von 405 mg 7-[D-$\alpha$-Amino-(p-hydroxyphenyl-acetamido])-3-[(1-methyl-tetrazol-5-yl)-thiomethyl]-ceph-3-em-4-carbonsäure (0,001 Mol) sowie dem Phosgen-Umsetzungsprodukt von 140 mg 5-Amino-4-hydroxy-2-methylamino-pyrimidin (0,001 Mol).

Bei der Aufarbeitung wird das entstandene Cephalosporin bei pH 2,8 aus Wasser ausgefällt, abgesaugt, getrocknet und in bekannter Weise in das Natriumsalz überführt.

Ausbeute: 315 mg (31,5%),

IR-Spektrum: 1760, 1655, 1610, 1540 $cm^{-1}$;

NMR-Spektrum (DMSO + $CD_3OD$) Signale bei ppm: 2,85 (s, 3H), 3,5 (q, 2H), 3,9 (s, 3H), 4,35 (q, 2H), 4,85 (d, 1H), 5,45 (s, 1H), 5,65 (d, 1H), 6,85 (d, 2H), 7,35 (d, 2H), 8,05 (s, 1H).

Beispiel 101

Natrium-7-{D-$\alpha$-[(4-hydroxy-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 94 ausgehend von dem Umsetzungsprodukt von 280 mg 5-Amino-4-hydroxy-2-methoxy-pyrimidin (0,002 Mol) mit Phosgen sowie 920 mg (0,002 Mol) 7-[D-$\alpha$-Amino-p-hydroxyphenylacetamido]-3-[(1-methyl-tetrazol-5-yl) thiomethyl-ceph-3-em-4-carboxylat.

Ausbeute: 735 mg (53%) Natriumsalz,

IR-Spektrum: 1760, 1660, 1615, 1545 $cm^{-1}$;

NMR-Spektrum (DMSO + $CD_3OD$) Signale bei ppm: 3,5 (q, 2H), 3,85 (s, 3H), 3,95 (s, 3H), 4,35 (q, 2H), 4,85 (d, 1H), 5,45 (s, 1H), 5,65 (d, 1H), 6,85 (d, 2H), 7,35 (d, 2H), 8,1 (s, 1H).

Beispiel 102

Natrium-7-{D-$\alpha$-[(4-hydroxy-2-propylamino-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 33 ausgehend von dem Umsetzungsprodukt von 1,70 g (0,01 Mol) 5-Amino-4-hydroxy-2-propyl-amino-pyrimidin mit Phosgen sowie 4,61 g (0,01 Mol) des in Beispiel 101 eingesetzten Cephalosporins. Aufarbeitung analog Beispiel 99.

Ausbeute: 4,0 (61%) Natriumsalz,

IR-Spektrum: 1760, 1660, 1610, 1540 $cm^{-1}$;

NMR-Spektrum (DMSO + $CD_3OD$) Signale bei ppm: 1,0 (t, 3H), 1,65 (q, 2H), 3,2 (t, 2H), 3,50 (q, 2H), 3,9 (s, 3H), 4,35 (q, 2H), 4,85 (d, 1H), 5,50 (s, 1H), 5,65 (d, 1H), 6,85 (d, 2H), 7,35 (d, 2H), 8,0 (s, 1H).

Beispiel 103

Natrium-7-{D-$\alpha$-[(4-hydroxy-2-isopropylamino-5-pyrimidinyl)-ureido]-p-hydroxy-phenylacetamido}-3-acetoxymethyl-ceph-3-em-4-carboxylat

Herstellung analog Beispiel 98 ausgehend vom Umsetzungsprodukt von 505 mg (0,003 Mol) 5-Amino-4-hydroxy-2-isopropylamino-pyrimidin mit Trimethylsilyldiäthylamin und Phosgen sowie 1,22 g (0,003 Mol) des Cephalosporin-Derivates des Beispiels 98. Aufarbeitung analog Beispiel 99.

Ausbeute: 980 mg (61%) Natriumsalz;

IR-Spektrum: 1760, 1660, 1615, 1535 $cm^{-1}$;

NMR-Spektrum: (DMSO + $CD_3OD$) Signale bei ppm: 1,15 (d, 6H), 2,05 (s, 3H), 3,55 (q, 2H), 3,90 (breit, 1H), 4,80 (m, 2 + 1H), 5,45 (s, 1H), 5.65 (d, 1H), 6,85 (d, 2H), 7,3 (d, 2H), 8,0 (s, 1H).

Beispiel 104

Natrium-7-{D,L-$\alpha$-[(4-hydroxy-2-propylamino-5-pyrimidinyl)-ureido]-2-thienyl-acetamido}-3-[(1-methyl-tetrazol-5-yl)-thiomethyl]-ceph-3-em-4-carboxylat

Eine Suspension von 435 mg (0,00093 Mol) 7-(D,L-$\alpha$-Amino-2-thienyl-acetamido-3-[(1-methyl-tetrazol-5-yl)-thiomethyl]-3-cephem-4-carbonsäure in 10 ml trockenem Acetonitril wird mit 1 ml Bistrimethyl-silylacetamid versetzt. Nach Erhalt einer homogenen Lösung wird das Umsetzungsprodukt von 160 mg (0,00095 Mol) 5-Amino-4-hydroxy-2-propylamino-pyrimidin mit Trimethylsilyldiäthylamin und 950 mg Phosgen, gelöst in Tetrahydrofuran, zugetropft. Die weitere Umsetzung erfolgte analog Beispiel 99.

Analog wurde hergestellt:

Natrium-7-{D-$\alpha$-[(2-äthylamino-4-hydroxy-5-pyrimidinyl)-ureido]-p-hydroxy-phenylacetamido}-3-[(2-methylamino-thiadiazol-5-yl)-thiomethyl]-ceph-3-em-4-carboxylat.

Beispiel 105

Natrium-7-{D-$\alpha$-[(2-allylamino-4-hydroxy-5-pyrimidinyl)-ureido]-phenylacetamido}-3-acetoxymethyl-ceph-3-em-4-carboxylat

Herstellung analog Beispiel 94, ausgehend von dem Umsetzungsprodukt vom 332 mg (0,002 Mol) 5-Amino-2-allylamino-4-hydroxy-pyrimidin mit 200 mg Phosgen und 850 mg Cephaloglycin-Dihydrat (0,002 Mol). Aufarbeitung analog Beispiel 98.

Ausbeute: 680 mg Natriumsalz (56,5%);
IR-Spektrum: 1760, 1660, 1610, 1540 $cm^{-1}$;
NMR-Spektrum: (DMSO + $CD_3OD$) Signale bei ppm: 2,05 (s, 3H), 3,4 (q, 2H), 3,9 (breites Signal, 2H), 4,85 (m, 2H + d, 1H), 5,0—5,5 (m, 3H), 5,45 (s, 1H), 5,65 (d, 1H), 6,0 (m, 1H), 7,45 (5H), 8,05 (s, 1H).

Beispiel 106

Natrium-7-{D-α-[(2-allylamino-4-hydroxy-5-pyrimidinyl)-ureido]-phenylacetamido}-3-[(1-methyl-tetrazol-5-yl)-thiomethyl]-ceph-3-em-4-carboxylat

Herstellung analog Beispiel 94, wobei man von 3,2 g (0,005 Mol) des entsprechenden Cephalosporinderivates sowie von 0,83 g (0,005 Mol) 5-Amino-2-allylamino-4-hydroxy-pyrimidin, das man zuvor mit 500 mg Phosgen umgesetzt hat, ausgeht.
Ausbeute: 1,52 g Natriumsalz (48%);
IR-Spektrum: 1760, 1660, 1615, 1545 $cm^{-1}$;
NMR-Spektrum: (DMSO + $CD_3OD$) Signale bei ppm: 3,5 (q, 2H), 3,95 (s, 3H + breites Signal 2H), 4,4 (q, 2H), 4,85 (d, 1H), 5,0—5,5 (m, 4H), 5,65 (d, 1H), 5,95 (m, 1H), 7,45 (m, 5H), 8,0 (s, 1H).
Analog wurde hergestellt:
Natrium-7-{D-α-[(2-(3′-methylallylamino)-4-hydroxy-5-pyrimidinyl)-ureido]-p-hydroxy-phenylacetamido}-3-[(1,2,4-thiadiazol-5-yl)-thiomethyl]-ceph-3-em-4-carboxylat
Natrium-7-{D-α-[(4-hydroxy-2-propargylamino-5-pyrimidinyl)-ureido]-p-hydroxy-phenylacetamido}-3-[(2-dimethylamino-thiadiazol-5-yl)-thiomethyl]-ceph-3-em-4-carboxylat
Natrium-7-{D-α-[(2-cyclopropylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat
Natrium-7-{D-α-[(2-cyclopropylmethylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat
Natrium-7-{D-α-[(2-cyclohexylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat
Natrium-7-{D-α-[(2-cyclohexylamino-4-hydroxy-5-pyrimidinyl)-ureido]-p-hydroxy-phenylacetamido}-3-[(1,2,4-thiadiazol-5-yl)-thiomethyl]-ceph-3-em-4-carboxylat

Beispiel 107

Natrium-7-{D-α-[(4-hydroxy-2-(4′-hydroxy-cyclohexylamino)-5-pyrimidinyl)-ureido]-p-hydroxy-phenylacetamido}-3-acetoxymethyl-ceph-3-em-4-carboxylat

Dieses Cephalosporin wird analog Beispiel 98 hergestellt. Man geht aus von 810 mg 7-(D-α-Amino-p-hydroxy-phenylacetamido)-3-acetoxymethyl-ceph-3-em-4-carboxylat (0,002 Mol) sowie dem Umsetzungsprodukt von 450 mg 5-Amino-4-hydroxy-2-(4′-hydroxycyclohexylamino)-pyrimidin (0,002 Mol) mit Trimethylsilyldiäthylamin und 200 mg Phosgen.
Die Aufarbeitung erfolgte analog Beispiel 99.
Ausbeute: 615 mg Natriumsalz (44,5%);
IR-Spektrum: 1760, 1660, 1540 $cm^{-1}$;
NMR-Spektrum: (DMSO + $CD_3OD$) Signale bei ppm: 1,75 (m, 8H), 2,05 (s, 3H), 3,35 (q, 2H), 3,5—4,0 (m, 1 + 1H), 4,80 (m, 2 + 1H), 5,45 (s, 1H), 5,65 (d, 1H), 6,8 (d, 2H), 7,35 (d, 2H), 8,0 (s, 1H).

Beispiel 108

Natrium-7-{D-α-[(4-hydroxy-2-(2′-methoxy-äthylamino)-5-pyrimidinyl)-ureido]-p-hydroxy-phenylacetamido}-3-[(1-methyltetrazol-5-yl)-thiomethyl]-ceph-3-em-4-carboxylat

Herstellung analog Beispiel 94, ausgehend von 550 mg des Cephalosporin des Beispiels 106 (0,00123 Mol) und 220 mg 5-Amino-4-hydroxy-2-(2′-methoxyäthylamino)-pyrimidin (0,0012 Mol).
Aufarbeitung analog Beispiel 99.
Ausbeute: 450 mg Natriumsalz (59%);
IR-Spektrum: 1760, 1660, 1610, 1540 $cm^{-1}$;
NMR-Spektrum: (DMSO + $CD_3OD$) Signale bei ppm: 3,0—3,5 (m, 2 + 2 + 2H), 3,55 (s, 3H), 3,9 (s, 3H), 4,35 (q, 2H), 4,85 (d, 1H), 5,45 (s, 1H), 5,65 (d, 1H), 6,8 (d, 2H), 7,35 (d, 2H), 8,0 (s, 1H).
Analog wurde hergestellt:
Natrium-7-{D-α-[(4-hydroxy-2-(2′-methoxy-äthylamino)-5-pyrimidinyl)-ureido]-p-hydroxy-phenylacetamido}-3-[(1,3,4-thiadiazol-5-yl)-thiomethyl]-ceph-3-em-4-carboxylat.

Beispiel 109

Natrium-7-{D-α-[(4-hydroxy-2-(3′-hydroxy-propylamino)-5-pyrimidinyl)-ureido]-p-hydroxy-phenylacetamido}-3-acetoxymethyl-ceph-3-em-4-carboxylat

Herstellung analog Beispiel 98, ausgehend von 1,84 g (0,01 Mol) 5-Amino-2-(3′-hydroxy-propylamino)-4-hydroxy-5-pyrimidin, das mit Trimethylsilyldiäthylamin und mit 1 g Phosgen und dann mit 4,05 g (0,01 Mol) 7-D-α-Amino-p-hydroxy-phenylacetamido-3-acetoxymethyl-ceph-3-em-4-carbonsäure umgesetzt wurde.
Ausbeute: 3,65 g Natriumsalz (50%);
IR-Spektrum: 1760, 1670, 1620, 1550 $cm^{-1}$;

NMR-Spektrum: (DMSO + $CD_3OD$) Signale bei ppm: 1,85 (m, 2H), 2,05 (s, 3H), 3,0—3,7 (m, 2 + 2 + 2H), 4,85 (m, 2 + 1H), 5,40 (s, 1H), 5,55 (d, 1H), 6,85 (d, 2H), 7,3 (d, 2H), 8,05 (s, 1H).

Ausgehend von demselben Cephalosporinderivat wurden analog hergestellt:

Natrium-7-{D-α-[(2-p-hydroxybenzylamino-4-hydroxy-5-pyrimidinyl)-ureido]-p-hydroxy-phenylacetamido}-3-acetoxymethyl-ceph-3-em-4-carboxylat

Natrium-7-{D-α-[(4-hydroxy-2-m,p-dioxymethylen-benzylamino-5-pyrimidinyl)-ureido]-p-hydroxy-phenylacetamido}-3-acetoxymethyl-ceph-3-em-4-carboxylat

Natrium-7-{D-α-[(2-p-dimethylamino-anilino-4-hydroxy-5-pyrimidinyl)-ureido]-p-hydroxy-phenylacetamido}-3-acetoxymethyl-ceph-3-em-4-carboxylat

## Beispiel 110

Natrium-7-{D-α-[(4-hydroxy-2-p-hydroxy-anilino-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 98, ausgehend von 218 mg (0,001 Mol) 5-Amino-4-hydroxy-2-p-hydroxy-anilino-pyrimidin, das nach der Reaktion mit Trimethylsilyldiäthylamin und Phosgen mit 460 mg des entsprechenden Cephalosporinderivats des Beispiel 108 (0,001 Mol) umgesetzt wurde.

Ausbeute: 280 mg Natriumsalz (38,5%);

IR-Spektrum: 1760, 1660, 1610, 1540 $cm^{-1}$;

NMR-Spektrum: (DMSO + $CD_3OD$) Signale bei ppm: 3,5 (q, 2H), 3,9 (s, 3H), 4,35 (q, 2H), 4,85 (d, 1H), 5,40 (s, 1H), 5,60 (d, 2H), 6,8 (d, 2H), 7,4 (m, 4H), 7,7 (d, 2H), 8,30 (s, 1H).

Mit der Vorgehensweise des Beispiels 98 bzw. 99 werden noch folgende Cephalosporinderivate hergestellt:

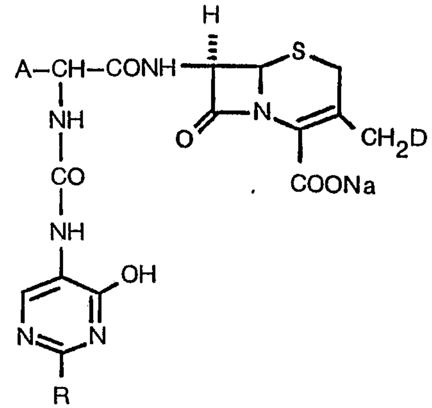

| Beispiel | A | R | D | Ausbeute % | IR-Spektrum $cm^{-1}$ | NMR-Spektrum (DMSO + $CD_3OD$) |
|---|---|---|---|---|---|---|
| 111 | 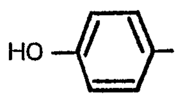 | 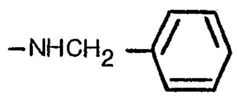 | $-OCOCH_3$ | 62 | 1760, 1660, 1605 | 2,05(s,3H), 3,40(q,2H), 4,2(bs,2H),4,8(m,2H), 4,9(d,1H), 5,5(s,1H), 5,6(d,1H), 6,80(d,2H), 7,4(m,7H), 8,05(s,1H) |
| 112 | 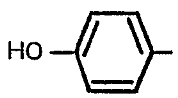 | 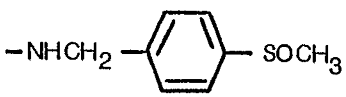 | 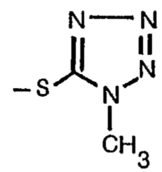 | 49,5 | 1760, 1655, 1600, 1150 | 2,7(s,3H), 3,4(q,2H), 3,95(s,3H), 4,4(m,2+2H), 4,95(d,1H), 5,5(s,2H), 5,65(d,1H), 6,85(d,2H), 7,35(m,4H), 7,7(d,2H), 8,05(s,1H) |
| 113 | 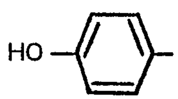 | 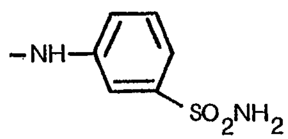 | 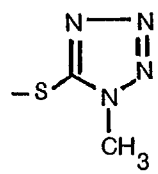 | 57 | 1760, 1650, 1600, 1150 | 3,35(q,2H), 3,95(s,3H), 4,3(q,2H), 5,0(d,1H), 5,55(s,1H), 5,65(d,1H), 6,85–7,0(m,3H), 7,3(m,3H), 8,31(s,1H), 8,75(s,1H) |

| Beispiel | A | R | D | Ausbeute % | IR-Spektrum $cm^{-1}$ | NMR-Spektrum (DMSO + $CD_3OD$) |
|---|---|---|---|---|---|---|
| 114 | 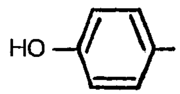 | 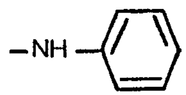 | $-OCOCH_3$ | 56 | 1760, 1660, 1600 | 2,05(s,3H), 3,4(q,2H), 4,8(m,2+1H), 5,50(s,1H), 5,60(d,1H), 6,85(d,2H), 7,3–7,7(m,7H), 8,30(s,1H) |
| 115 | 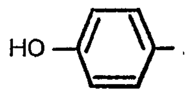 | 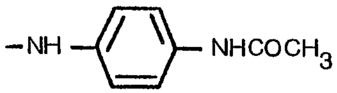 | $-OCOCH_3$ | 64,5 | 1760, 1655, 1599 | 2,05(s,3H), 2,1(s,3H), 3,4(q,2H), 4,7–5,0 (m,2+1H), 5,55(s,1H), 5,65(d,1H), 6,75(d,2H), 7,3–7,5(m,4H), 7,7(d,2H), 8,3(s,1H) |
| 116 | 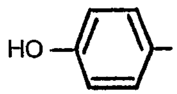 | 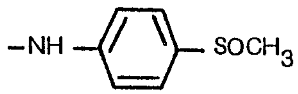 | $-OCOCH_3$ | 47 | 1760, 1665, 1610 | 2,05(s,3H), 2,75(s,3H), 3,40(q,2H), 4,7–5,0(m,2+1H), 5,60(d,1H), 6,75(d,2H), 7,2–8,0(m,6H), 8,35(s,1H) |
| 117 | 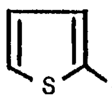 D,L | 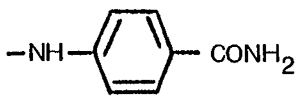 | 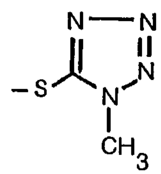 | 62 | 1760, 1660, 1600 | 3,35(m,2H), 3,95(s,3H), 4,3(q,2H), 5,05(dd,1H), 5,60(dd,1H), 5,70(d,1H), 7,0(m,2H), 7,4(m,1H), 7,75(m,4H), 8,35(s,1H), |
| 118 | 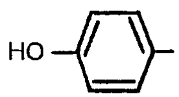 | 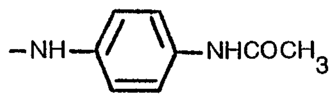 | $-OCOCH_3$ | 60 | 1760, 1665, 1610 | 2,05(s,3H), 3,4(q,2H), 4,8(m,2+1H), 5,5(s,1H), 5,6(d,1H), 6,80(d,2H), 7,35(dd,4H), 7,6(d,2H), 8,2(s,1H) |

| Beispiel | A | R | D | Ausbeute % | IR-Spektrum $cm^{-1}$ | NMR-Spektrum (DMSO + $CD_3OD$) |
|---|---|---|---|---|---|---|
| 119 | 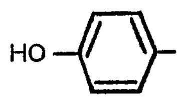 | $-NHCOCH_3$ | $-OCOCH_3$ | 49 | 1760, 1650, 1600 | 2,05(s,3H), 2,25(s,3H), 3,45(q,2H), 4,8(m,2H), 5,0(d,1H), 5,5(s,1H), 5,6(d,1H), 6,85(d,2H), 7,35(d,2H), 8,25(s,1H) |
| 120 | 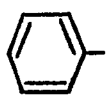 | 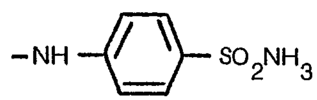 | $-OCOCH_3$ | 71 | 1760, 1660, 1610, 1150, | 2.0(s,3H), 3,4(q,2H), 4,95(d,1H), 5,45(s,1H), 5,60(d,1H), 7,4(breites s,5H), 7,7(d,2H), 8,0(d,2H), 8,37(s,1H) |

Beispiel 121

Natrium-7-{D-$\alpha$-[(4-hydroxy-2-isopropylamino-5-pyrimidinyl)-ureido]-p-hydroxy-phenylacetamido}-3-[(2-methyl-oxadiazol-5-yl)-thiomethyl]-ceph-3-em-4-carboxylat

In 10 ml einer Phosphorsäure-Pufferlösung von pH 6,3 löst man 360 mg des in Beispiel 103 erhaltenen Cephalosporinderivats. Zu dieser Lösung gibt man 70 mg 5-Methyl-2-mercapto-1,3,4-oxadiazol und erhitzt 6 Stunden unter Stickstoff auf 70°C, wobei der pH-Wert auf 6,0—6,5 gehalten wird. Nach dieser Zeit wird die Reaktionsflüssigkeit abgekühlt und mit Essigester zweimal ausgeschüttelt. Anschließend wird unter Kühlen 2n Salzsäure bis zu einen pH-Wert von 2,9 zugesetzt. Das ausgefallene Produkt wird abgesaugt, mit wenig Wasser gewaschen und getrocknet. Der Rückstand wird auf die übliche Weise in das Natriumsalz überführt.

Man erhält 235 mg (61%);

IR-Spektrum: 1760, 1660, 1615, 1545 $cm^{-1}$;

NMR-Spektrum: (DMSO + $CD_3OD$): 1,15 (d, 6H), 2,45 (s, 3H), 3,6 (q, 2H), 3,9 (m, breit 1H), 4,2 (q, 2H, teilweise verdeckt durch LM), 4,95 (d, 1H), 5,45 (s, 1H), 5,60 (d, 1H), 6,8 (d, 2H), 7,35 (d, 2H), 8,0 (s, 1H).

Analog wurde aus demselben Cephalosporinderivat mit 4-Mercapto-1,2,3-triazol in 56% Ausbeute hergestellt:

Natrium-7-{D-$\alpha$-[(4-hydroxy-2-isopropylamino-5-pyrimidinyl)-ureido]-p-hydroxy-phenylacetamido}-3-[(1,2,3-triazol-4-yl)-thiomethyl]-ceph-3-em-4-carboxylat

Beispiel 122

Natrium-7-{D-$\alpha$-[(4-hydroxy-2-(4'-hydroxy-cyclohexylamino)-5-pyrimidinyl)-ureido]-p-hydroxyphenylacetamido}-3-[(1-methyl-tetrazol-5-yl)-thiomethyl]-ceph-3-em-4-carboxylat

Herstellung analog Beispiel 121, wobei man von 680 mg des in Beispiel 107 erhaltenen Cephalosporinderivats ausgeht und dieses mit 135 mg 5-Mercapto-1-methyl-tetrazol umsetzt.

Ausbeute: 410 mg Natriumsalz (58%);

IR-Spektrum: 1760, 1660, 1610, 1535 $cm^{-1}$;

NMR-Spektrum: (DMSO + $CD_3OD$) Signale bei ppm: 1,8 (m, 8H), 3,5 (q, 2H), 3,6—4,0 (breites m, 1 + 1H), 3,95 (s, 3H), 4,35 (q, 2H), 4,85 (d, 1H), 5,40 (s, 1H), 5,60 (d, 1H), 6,8 (d, 2H), 7,3 (d, 2H), 8,0 (s, 1H).

Ausgehend von dem im Beispiel 107 erwähnten Cephalosporinderivat wurden weiter folgende neue Cephalosporine hergestellt:

Beispiel 123

Natrium-7-{D-$\alpha$-[(4-hydroxy-2-(4'-hydroxy-cyclohexylamino)-5-pyrimidinyl)-ureido]-p-hydroxyphenylacetamido}-3-[(1,3,4-thiadiazol-5-yl)-thiomethyl]-ceph-3-em-4-carboxylat

durch Umsetzung mit 2-Mercapto-1,3,4-thiadiazol in 68,5%-iger Ausbeute.

Beispiel 124

Natrium-7-{D-$\alpha$-[(4-hydroxy-2-(4'-hydroxy-cyclohexylamino)-5-pyrimidinyl)-ureido]-p-hydroxyphenylacetamido}-3-[(tetrazol-5-yl)-thiomethyl]-ceph-3-em-4-carboxylat

durch Umsetzung mit 5-Mercapto tetrazol in 56%-iger Ausbeute.

Beispiel 125

Natrium-7-{D-$\alpha$-[(4-hydroxy-2-(3'-hydroxypropylamino)-5-pyrimidinyl)-ureido]-p-hydroxyphenylacetamido}-3-[(2-methyl-1,3,4-oxadiazol-5-yl)-thiomethyl]-ceph-3-em-4-carboxylat

Herstellung analog Beispiel 121.

Man geht aus von 640 mg des in Beispiel 109 erhaltenen Cephalosporinderivates (0,001 Mol) und setzt dieses mit 180 mg 5-Mercapto-2-methyl-1,3,4-oxadiazol um.

Ausbeute: 455 mg Natriumsalz (65%);

IR-Spektrum: 1760, 1670, 1615, 1550 $cm^{-1}$;

NMR-Spektrum: (DMSO + $CD_3OD$) Signale bei ppm: 1,80 (m, 2H), 2,45 (s, 3H), 3,0—3,7 (m, 2 + 2 + 2H), 4,2 (q, 2H), teilweise durch LM verdeckt), 4,94 (d, 1H), 5,45 (s, 1H), 5,65 (d, 1H), 6,85 (d, 2H), 7,35 (d, 2H), 8,05 (s, 1H).

Setzt man das in Beispiel 109 erhaltene 3-Acetoxymethyl-cephalosporinderivat ein, so erhält man folgende Cephalosporinderivate:

Beispiel 126

Natrium-7-{D-$\alpha$-[(4-hydroxy-2-(3'-hydroxypropylamino)-5-pyrimidinyl)-ureido]-p-hydroxyphenylacetamido}-3-[(2-formylamino-thiadiazol-5-yl)-thiomethyl]-ceph-3-em-4-carboxylat

durch Umsetzung mit 2-Formylamino-5-mercapto-thiadiazol in 44%iger Ausbeute.

Beispiel 127

Natrium-7-{D-$\alpha$-[(4-hydroxy-2-(3'-hydroxypropylamino)-5-pyrimidinyl)-ureido]-p-hydroxyphenylacetamido}-3-[(1,2,4-thiadiazol-5-yl)-thiomethyl]-ceph-3-em-4-carboxylat

durch Umsetzung mit 5-Mercapto-1,2,4-thiadiazol in 53%iger Ausbeute.

Beispiel 128

Natrium-7-{D-α-[(4-hydroxy-2-(3'-hydroxypropylamino)-5-pyrimidinyl)-ureido]-p-hydroxy-phenylacetamido}-3-(4-aminocarbonyl-pyridinium)-ceph-3-em-4-carboxylat

durch Umsetzung mit 4-Aminocarbonyl-pyridin in 38,5%iger Ausbeute.

Beispiel 129

Natrium-7-{D-α-[(4-hydroxy-2-(3'-hydroxypropylamino)-5-pyrimidinyl-ureido]-p-hydroxy-phenylacetamido}-3-[(2-methylamino-thiadiazol-5-yl)-thiomethyl]-ceph-3-em-4-carboxylat

durch Umsetzung mit 2-Methylamino-5-mercapto-1,3,4-thiadiazol in 61%iger Ausbeute.

Analog wurden synthetisiert:

Natrium-7-{D-α-[(2-m,p-dioxymethylen-benzylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Natrium-7-{D-α-[(p-dimethylamino-anilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Natrium-7-{D-α-[(p-dimethylamino-anilino-4-hydroxy-5-pyrimidinyl)-ureido]-p-hydroxy-phenylacetamido}-3-[(2-formylamino-1,3,4-thiadiazol-5-yl)-thiomethyl]-ceph-3-em-4-carboxylat

Natrium-7-{D-α-[(p-dimethylamino-anilino-4-hydroxy-5-pyrimidinyl)-ureido]-p-hydroxy-phenylacetamido}-3-[(2-methyl-1,3,4-oxadiazol-5-yl)-thiomethyl]-ceph-3-em-4-carboxylat

Natrium-7-{D-α-[(2-acetylamino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Beispiel 130

Natrium-7-{D-α-[(2-p-Aminosulfonylanilino-4-hydroxy-5-pyrimidinyl)-ureido]-p-hydroxy-phenylacetamido}-3-[(4'-aminocarbonyl-pyridino)-methyl]-ceph-3-em-4-carboxylat

Ein Gemisch von 2 m Mol der Verbindung des Beispiels 21 sowie 3 m Mol 4-Pyridincarboxamid, 4 g Kaliumthiocyanat und 5 ml Wasser werden 8 Stunden auf 50°C erhitzt. Die erhaltene Lösung wird über eine mit dem Ionenaustauscherharz Amberlite XAD-2 gefüllte Säule gegeben, die zunächst mit etwa 1 l Wasser gewaschen wird. Anschließend eluiert man die gewünschte Verbindung mit einem 7:3 Gemisch von Wasser und Methanol. Aus dem Eluat wird das Methanol abdestilliert und die Lösung gefriergetrocknet.

NMR-Spektrum ($D_2O$): 3,6 (m, 2H), 5,1 (d, 1H), ~5,4 (q, 2H), 5,7 (s, 1H), 5,8 (d, 1H), 7,7 (d, 2H), 8,0 (d, 2H), 8,3 (d, 2H), 8,38 (s, 1H), 9,1 (d, 2H).

Beispiel 131

Verwendet man statt 4-Pyridincarboxamid Pyridin, so erhält man auf die gleiche Weise:

7-{D-α-[(2-p-Aminosulfonylanilino-4-hydroxy-5-pyrimidinyl)-ureido]-p-hydroxy-phenylacetamido}-3-(pyridinomethyl)-ceph-3-em-4-carboxylat

NMR-Spektrum: ($D_2O$) 3,65 (m, 2H), 5,1 (d, 1H), 5,45 (q, 2H), 5,7 (s, 1H), 5,8 (d, 1H), 7,7 (d, 2H), 8,05 (d, 2H), 8,1, 8,5, 8,9 (m, 5H), 8,38 (s, 1H).

Beispiel 132

7-{D-α-[(4-Hydroxy-3-(3'-hydroxypropylamino)-5-pyrimidinyl)-ureido]-p-hydroxy-phenylacetamido}-[(4'-aminocarbonyl-pyridino)-methyl]-ceph-3-em-4-carboxylat

Durch Umsetzung des Cephalosporins des Beispiels 109 mit 4-Aminocarbonyl-pyridin in 38,5%iger Ausbeute.

Ausgehend von dem Cephalosporin des Beispiels 21 erhält man nach der Methode des Beispiels 121 folgende Cephalosporine:

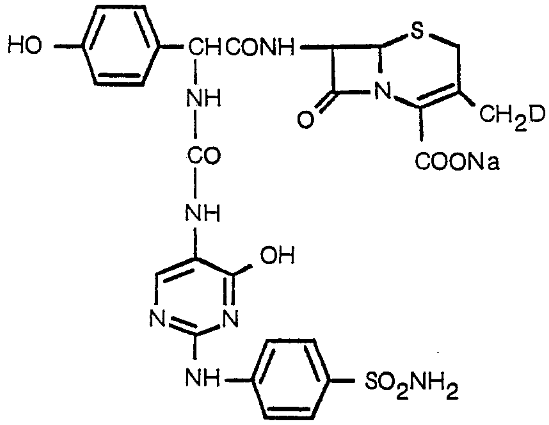

| Beispiel | D | Ausbeute % | IR $cm^{-1}$ | NMR |
|---|---|---|---|---|
| 133 | 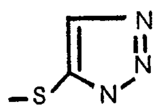 | 46 | 1760, 1660, 1600, 1145 | 3,3(q,2H), 4,4(q,2H), 5,0(d,1H), 5,55(s,1H), 5,65(d,1H), 6,75(d,2H), 7,3(d,2H), 7,7(d,2H), 8,0(d,2H+s,1H), 8,37(s,1H) |
| 134 | 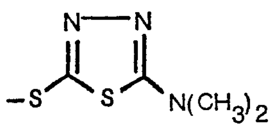 | 55 | 1765, 1665, 1610, 1150 | 2,90(d,6H), 3,4(m,2H), 4,35(q,2H), 4,95(d,1H), 5,55(s,1H), 5,65(s,1H), 6,75(d,2H), 7,3(d,2H), 7,65(d,2H), 7,95(d,2H), 8,38(s,1H) |
| 135 | 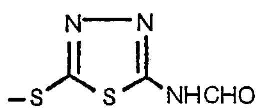 | 34.5 | 1760, 1660, 1630, 1610, 1150 | 3,3(m,2H), 4,3(m,2H), 5,0(d,1H), 5,55(s,1H), 5,65(d,1H), 6,75(d,2H), 7,35(d,2H), 7,7(d,2H), 8,0(d,2H), 8,37(s,1H) |
| 136 | 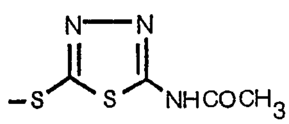 | 41 | 1760, 1660, 1635, 1600, 1150 | 2,3(s,3H), 3,15(m,2H), 4,3(q,2H), 5,0(d,1H), 5,50(s,1H), 5,65(d,1H), 6,75(d,2H), 7,35(d,2H), 7,7(d,2H), 8,0(d,2H), 8,35(s,1H) |
| 137 | 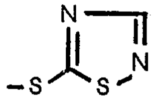 | 72 | 1760, 1660, 1600, 1150 | 3,3(m,2H), 4,25(q,2H), 5,05(d,1H), 5,55(s,1H), 5,65(d,1H), 6,75(d,2H), 7,35(d,2H), 7,7(d,2H), 8,0(d,2H), 8,37(s,1H) |
| 138 | 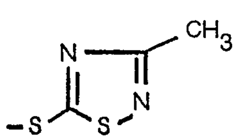 | 68 | 1760, 1655, 1595, 1145 | 2,5(s,3H), 3,3(m,2H), 4,25(q,2H), 5,05(d,1H), 5,55(s,1H), 5,65(d,1H), 6,75(d,2H), 7,35(d,2H), 7,7(d,2H), 8,0(d,2H), 8,3(s,1H), 8,36(s,1H) |

Analog wird, ausgehend von der entsprechenden 3-Acetoxy-methylverbindung folgendes Cephalosporin synthetisiert:

Natrium-7-{D-α-[(2-anilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Beispiel 139

Pivaloyloxymethyl-7-{D-α-[(2-cyclopropyl-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Man rührt eine Lösung von 900 mg (0,0013 Mol) des Natriumsalzes des Beispiels 3 und 325 mg Pivaloyloxymethyljodid in 15 ml Dimethylformamid während einer Stunde bei Raumtemperatur. Anschließend gibt man 50 ml Essigsäureäthylester und 50 ml 0,1 m-Natriumhydrogencarbonat-Lösung zu. Die Essigesterschicht wird dann nacheinander mit Wasser, verdünnter Salzsäure, Wasser und gesättigter Natriumchloridlösung gewaschen, über Magnesiumsulfat getrocknet und im Vakuum zur Trockne eingeengt. Der Rückstand wird mit wasserfreiem Äther verrührt und abgesaugt.

Ausbeute: 670 mg (66%);

IR-Spektrum: 1770, 1735,

NMR-Spektrum: ($CDCl_3$ + $CD_3OD$) Signale bei ppm: 0,9 M, 4H), 1,10 (s, 9H), 1,95 (m, 1H), 3,6 (m, 2H), 4,0 (s, 3H), 4,5 (m, 2H), 4,95 (d, 1H), 5,5 (s, 1H), 5,75 (d, 1H), 5,85 (dd, 2H), 6,9 (d, 2H), 7,4 (d, 2H), 8,4 (s, 1H).

Nach dieser Methode wurden auch die folgenden Ester hergestellt:

Pivaloyloxymethyl-7-{D-α-[(4-hydroxy-2-isopropylamino-5-pyrimidinyl-ureido]-p-hydroxy-phenylacetamido}-3-[(1-methyl-tetrazol-5-yl)-thiomethyl]-ceph-3-em-4-carboxylat

Acetyloxymethyl-7-{D-α-[(4-hydroxy-2-(4′-hydroxy-cyclohexyl-amino-5-pyrimidinyl)-ureido]-p-hydroxy-phenylacetamido}-3-[(1-methyl-tetrazol-5-yl)-thiomethyl]-ceph-3-em-4-carboxylat

Pivaloyloxymethyl-7-{D-α-[(4-hydroxy-2-(3′-hydroxy-propyl-amino-5-pyrimidinyl)-ureido]-p-hydroxy-phenylacetamido}-3-[(1-methyl-tetrazol-5-yl)-thiomethyl]-ceph-3-em-4-carboxylat

Beispiel 140

Pivaloyloxymethyl-7-{D-α-[(4-hydroxy-2-(3′-methylallylamino-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 139, ausgehend von 3,4 g des Natriumsalzes des Beispiels 51, das mit 1,2 g Pivaloyloxymethyljodid ungesetzt wurde.

Ausbeute: 2,62 g (68%)

IR-Spektrum: 1770, 1740 $cm^{-1}$;

NMR-Spektrum: ($CDCl_3$ + $CD_3OD$) Signale bei ppm: 1,10 (s, 9H), 1,75 (d, 3H), 3,55 (q, 2H), 3,85 (breit, 2H), 3,95 (s, 3H), 4,45 (m, 2H), 4,95 (d, 1H), 5,55 (s, 1H), 5,65 (m, 2 + 1H), 5,8 (dd, 2H), 6,75 (d, 2H), 7,35 (d, 2H), 8,0 (s, 1H).

Beispiel 141

Pivaloyloxymethyl-7-{D-α-[(2-p-aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel 139, ausgehend von dem Natriumsalz, das in Beispiel 20 synthethisiert wurde, und das mit Pivaloyloxymethyljodid umgesetzt wird.

Ausbeute: 64%;

IR-Spektrum: 1770, 1730 $^{-1}$;

NMR-Spektrum: ($CDCH_3$ + $CD_3OD$) Signale bei ppm: 1,10 (s, 9H), 3,50 (q, 2H), 3,95 (s, 3H), 4,40 (q, 2H), 5,0 (d, 1H), 5,60 (s, 1H), 5,65 (d, 1H), 5,80 (dd, 2H), 6,75 (d, 2H), 7,3 (d, 2H), 7,7 (d, 2H), 8,0 (d, 2H), 8,38 (s, 1H).

Beispiel 142

Natrium-7-{D-α-[(4-hydroxy-2-p-methylsulfonylanilino-5-pyrimidinyl)-ureido]-p-hydroxy-phenylacetamido-3-[(1-methyl-tetrazol-5-yl)-thiomethyl]-ceph-3-em-4-carboxylat

Herstellung analog Beispiel 93, ausgehend von 1,0 g 7 - [D - α - Amino - (p - hydroxy - phenylacetamido)] - 3 - [(1 - methyl - tetrazol - 5 - yl) - thiomethyl] - ceph - 3 - em - 4 - carbonsäure (2,47 m Mol) sowie dem Umsetzungsprodukt von 690 mg 5 - Amino - 4 - hydroxy - 2 - p - methyl - sulfonylanilino - pyrimidin mit Trimethylsilyldiäthylamin und Phosgen.

Ausbeute: 940 mg Natriumsalz (52%);

IR-Spektrum: 1765, 1650, 1600, 1150 $cm^{-1}$;

NMR-Spektrum: (DMSO + $CD_3OD$) Signale bei ppm: 3,1 (s, 3H), 3,45 (q, 2H), 3,95 (s, 3H), 4,25 (m, 2H), 4,95 (d, 1H), 5,5 (s, 1H), 5,60 (d, 1H), 6,80 (d, 2H), 7,35 (d, 2H), 7,85 (dd, 4H), 8.30 (s, 1H).

Beispiele zur Herstellung pharmazeutischer Anwendungsformen:

Beispiel I

Tabletten enthaltend Pivaloyloxymethyl-7-{D-α-[(2-p-amino-sulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Ein Gemisch bestehend aus 2 kg Wirksubstanz, 5 kg Lactose, 1,8 kg Kartoffelstärke, 0,1 kg

Magnesiumstearat und 0,1 kg Talk wird in üblicher Weise zu Tabletten gepreßt, derart, daß jede Tablette 200 mg Wirkstoff enthält.

Beispiel II

Dragees enthaltend Pivaloyloxymethyl-7-{D-$\alpha$-[(2-p-amino-sulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

Analog Beispiel I werden Tabletten gepreßt, die anschließend in üblicher Weise mit einem Überzug, bestehend aus Zucker, Kartoffelstärke, Talk und Tragant überzogen werden.

Beispiel III

Kapseln enthaltend Pivaloyloxymethyl-7-{D-$\alpha$-[(2-p-aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

5 kg Wirksubstanz werden in üblicher Weise in Hartgelatinekapseln gefüllt, derart, daß jede Kapsel 500 mg des Wirkstoffes enthält.

Beispiel IV

Natrium-7-{D-$\alpha$-[(2-p-aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat

In einem aseptischen Bereich wurde 251 g Wirkstoff in 2008 ml destilliertem Wasser zur Injektion aufgelöst. Die Lösung wurde durch ein Millipore-Filter (Porengröße 0,22 um, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der allgemeinen Formel I oder die physiologisch unbedenklichen Salze dieser Verbindungen enthalten.

**Patentansprüche für die Vertragsstaaten: BE CH DE FR GB IT LI LU NL SE**

1. Cephalosporine der allgemeinen Formeln I bzw. I'

(I)

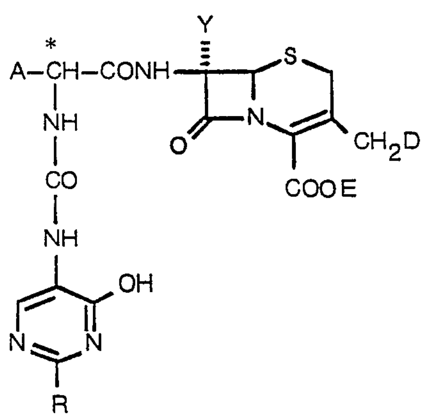

bzw.

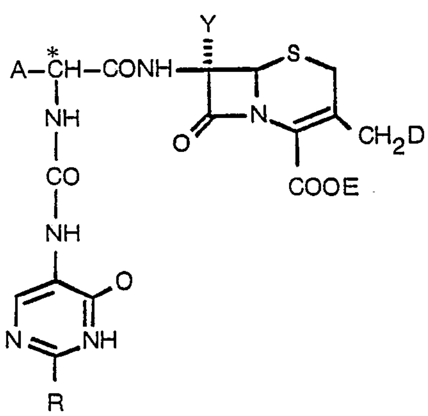

(I')

in welchen A, D, E, Y und R die folgenden Bedeutungen besitzen:

A die Phenyl-, 4-Hydroxyphenyl-, 2- oder 3-Thienyl-, 2-Furyl- oder 3,4-Dihydroxyphenylgruppe,

Y ein Wasserstoffatom oder die Methoxygruppe,

D ein Wasserstoffatom, die Acetoxy-, Aminocarbonyloxy-, Pyridinium- oder Aminocarbonylpyridiniumgruppe oder die Gruppe —S—Het, in der Het die 1-Methyl-tetrazol-5-yl-, Tetrazol-5-yl-, 3-Methyl-1,2,4-thiadiazol-5-yl-, 1,2,4-Thiadiazol-5-yl-, 1,3,4-Thiadiazol-5-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder 1,2,4-Triazol-3-yl-Gruppe bedeutet,

R ein Wasserstoffatom, den Cyclopropylrest, die Hydroxygruppe, die Methoxygruppe, eine Gruppe der Allgemeinen Formel

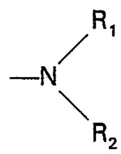

in der $R_1$ und $R_2$ gleich oder voneinander verschieden sein können und Wasserstoffatome, aliphatische, verzweigte oder unverzweigte Kohlenwasserstoffreste, die eventuell eine oder zwei Doppelbindungen oder eine Dreifachbindung und 1 bis 4 Kohlenstoffatome enthalten können, Cycloalkylreste mit 3 bis 6 Kohlenstoffatomen, durch einen Cycloalkylrest substituierte Methylgruppen mit 3 bis 6 Kohlenstoffatomen im Cycloalkylrest darstellen,

R bedeutet aber auch eine Gruppe der allgemeinen Formel —NH—Z—X, in der

Z eine geradkettige oder verzweigte Alkylgruppe mit 1 bis 3 Kohlenstoffatomen oder eine durch den Substituenten X substituierte Cycloalkylgruppe mit 5 oder 6 Kohlenstoffatomen bedeutet und

X die Hydroxygruppe, die Aminocarbonyl-, die Aminosulfonylgruppe oder die Aminocarbonylaminogruppe oder eine Gruppe der allgemeinen Formeln

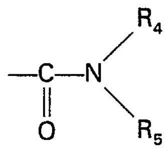 $—NHCOR_5$, 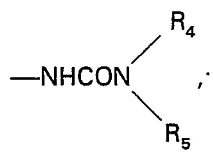 , $—NHSO_2R_4$,

$—OR_4$, $—OCOR_5$, $—SR_4$, $—SO—R_4$ und $—SO_2—R_4$

sein kann, wobei in diesen Formeln

$R_4$ eine geradkettige oder verzweigte Alkylgruppe mit 1 bis 3 Kohlenstoffatomen und $R_5$ Wasserstoff oder eine Alkylgruppe mit 1 bis 3 Kohlenstoffatomen darstellen,

R bedeutet weiter eine Gruppe der allgemeinen Formel

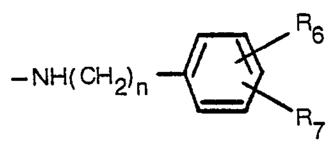

in der n null oder 1 ist und $R_6$ und $R_7$, die gleich oder voneinander verschieden sein können, Wasserstoff, Halogen, Alkylamino-, Dialkylamino-, Hydroxy-, Alkoxy-, aliphatische Acylamino-, Aminocarbonylamino-, Nitro-, Alkylsulfonylamino-, Alkylcarbonyl-, Aminocarbonyl-, Alkylsulfinyl-, Alkylsulfonyl-, Aminosulfonyl- oder Alkyl- und Dialkylaminosulfonylgruppen bedeuten, wobei jeder Alkylrest in den genannten Gruppen 1 bis 3 Kohlenstoffatome enthalten kann,

E ein Wasserstoffatom oder eine in vitro oder vivo leicht abspaltbare Schutzgruppe, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können, und, falls E Wasserstoff bedeutet, deren physiologisch verträgliche Salze mit anorganischen oder organischen Basen, steht D jedoch für Pyridinium oder Aminocarbonylpyridinium, dann haben die Verbindungen die allgemeine Formel Ia

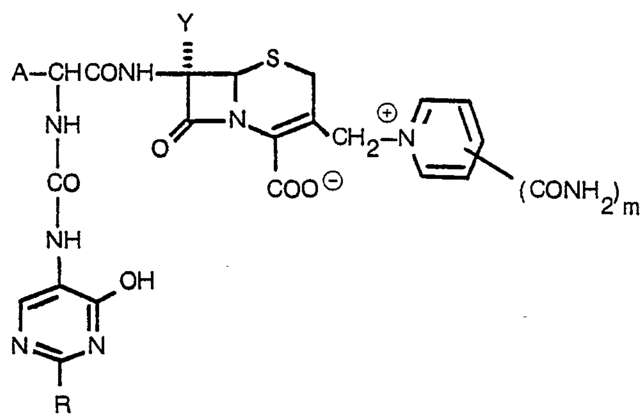

(Ia)

in der D, Y und R wie oben definiert sind und m die Zahl 0 oder 1 darstellt.

2. Cephalosporine der allgemeinen Formeln I, I′ oder Ia gemäß Anspruch 1, worin

A die 4-Hydroxyphenyl- oder 2-Thienylgruppe,

Y ein Wasserstoffatom oder die Methoxygruppe,

D die Acetoxy- oder Pyridinium-Gruppe oder die Gruppe —S—Het, in der Het die 1-Methyltetrazol-5-yl-, 2-Methyl-1,3,4-thiadiazol-5-yl-, 2-Methylamino-1,3,4-thiadiazol-5-yl oder 2-Acetylamino-1,3,4-thiadiazol-5-ylgruppe darstellt,

R die Cyclopropyl-, n-Propylamino-, 4-Hydroxy-cyclohexylamino- oder 3-Hydroxy-n-propylaminogruppe oder eine Gruppe der allgemeinen Formel

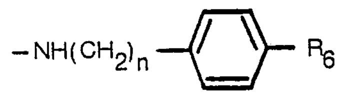

in der n null oder 1 ist und $R_6$ ein Wasserstoffatom, die Hydroxy-, Aminocarbonyl-, Aminosulfonyl- oder Methylaminosulfonylgruppe darstellt und

E ein Wasserstoffatom, oder eine wie im Anspruch 1 erwähnte Estergruppe bedeuten und, falls E ein Wasserstoffatom ist, deren physiologisch verträgliche Salze mit anorganischen oder organischen Basen.

3. Cephalosporine der allgemeinen Formeln I bzw. I' gemäß den Ansprüchen 1 und 2, dadurch gekennzeichnet, daß E die Benzyl-, Diphenylmethyl-, Trityl-, t-Butyl-, die 2,2,2-Trichloräthyl- oder die Trimethylsilylgruppe, eine Alkanoyloxyalkylgruppe mit 1 bis 5 Kohlenstoffatomen im Alkanoylrest und 1 bis 3 Kohlenstoffatomen im Alkenylrest, oder die Phthalidyl- oder Idanylgruppe bedeutet.

4. Cephalosporine der allgemeinen Formeln I bzw. I' gemäß Anspruch 3, dadurch gekennzeichnet, daß E die Acetoxymethyl-, Propionyloxymethyl-, 2-Acetoxyäthyl- oder Pivaloyloxymethylgruppe bedeutet.

5. Cephalosporine der allgemeinen Formeln I, I' oder Ia gemäß den Ansprüchen 1 bis 4, dadurch gekennzeichnet, daß für diese Verbindungen die D=R-Konfiguration zutrifft.

6. Natrium-7-$\beta$-D-$\alpha$-[(2-p-aminosulfonylanilino-4-hydroxy-5-pyrimidinyl)-ureido]-p-hydroxy-phenylacetamido-7$\alpha$-methoxy-3-[(1-methyl-tetrazol-5-yl)-thiomethyl]-ceph-3-em-4-carboxylat.

7. 7-D-$\alpha$-[(2-p-Aminosulfonylanilino-4-hydroxy-5-pyrimidinyl)-ureido]-p-hydroxy-phenylacetamido-3-(pyridino-methyl)-ceph-3-em-4-carboxylat.

8. Arzneimittel, enthaltend eine oder mehrere Verbindungen der allgemeinen Formeln I, I' oder Ia neben den üblichen Träger- und/oder Hilfsstoffen.

9. Verfahren zur Herstellung der Cephalosporine der allgemeinen Formeln I bzw. I'

(I) 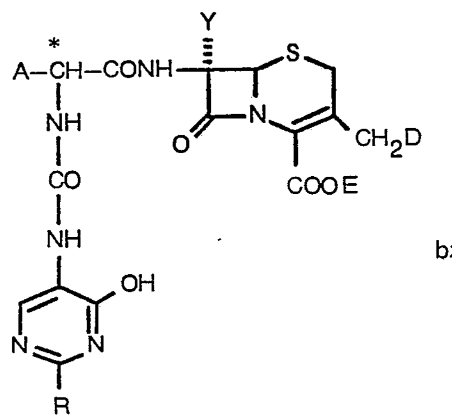 bzw. 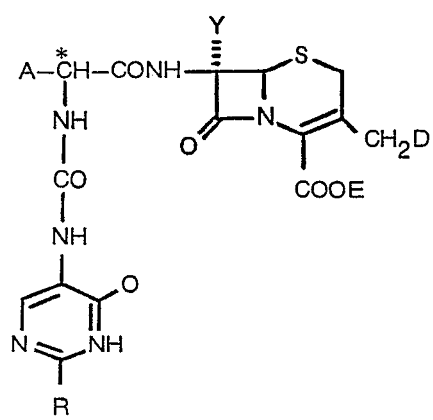 (I')

in welchen A, D, E, Y und R wie im Anspruch 1 definiert sind, und falls E ein Wasserstoff bedeutet, von deren physiologisch verträglichen Salzen mit anorganischen oder organischen Baden oder, sofern D für Pyridinium oder Aminocarbonylpyridinium steht, von Verbindungen der allgemeinen Formel Ia

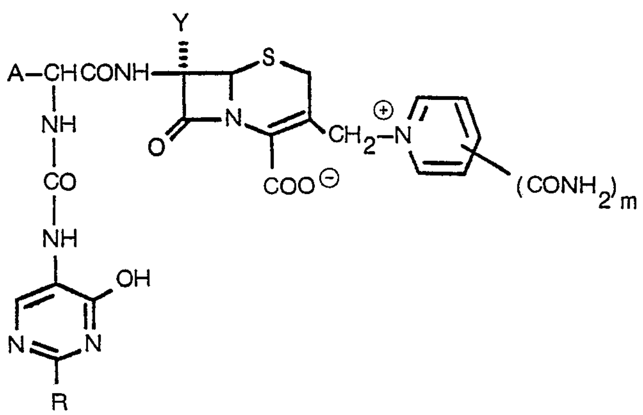 (Ia)

in der A, Y und R wie oben definiert sind und m die Zahl 0 oder 1 darstellt, dadurch gekennzeichnet, daß

a) zur Herstellung von Verbindungen der allgemeinen Formel I, in der D die angegebenen Bedeutung mit Ausnahme der einer Pyridinium- oder Aminocarbonylpyridiniumgruppe besitzt, eine Verbindung der allgemeinen Formel II

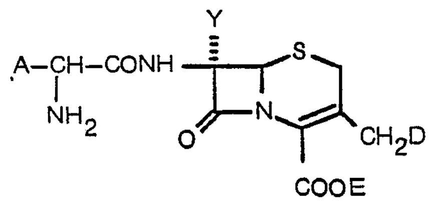

(II)

in der A, Y und E die oben angegebene Bedeutung und D die oben angegebene Bedeutung mit Ausnahme der einer Pyridinium- oder Aminocarbonylpyridiniumgruppe besitzen, mit einem Pyrimidinderivat der allgemeinen Formel III

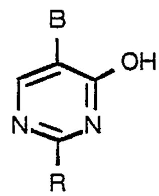

(III)

in der R wie oben definiert ist und B die Gruppe —NCO oder ein reaktives Derivat der Gruppe —NHCOOH bedeutet, wie die Gruppen —NHCOCl, —NHCOBr oder

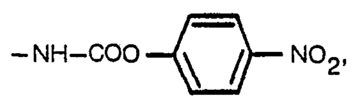

oder mit Gemischen von solchen Pyrimidinderivaten der allgemeinen Formel III, in der B teils die eine und teils die andere der vorstehend genannten Bedeutungen besitzt, bei Temperaturen zwischen —20 und +50°C in einem pH-Bereich zwischen 2,0 und 9,0 umgesetzt wird oder

b) zur Herstellung von Verbindungen der allgemeinen Formel I, in der D die oben angegebenen Bedeutungen mit Ausnahme einer Pyridinium- oder Aminocarbonylpyridiniumgruppe besitzt, Ureidocarbonsäuren der allgemeinen Formel IV

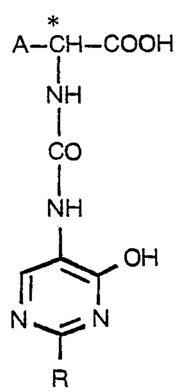

(IV)

in der A und die oben angegebenen Bedeutungen besitzen, oder ihre Salze oder reaktive Derivate mit 7-Amino-cephalosporansäurederivaten der allgemeinen Formel V

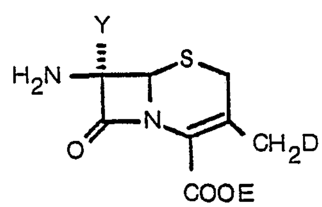

(V)

in der E und Y wie oben definiert sind, und D die oben angegebenen Bedeutungen mit Ausnahme der einer Pyridinium- oder Aminocarbonylpyridiniumgruppe besitzt, in einem Lösungsmittel bei Temperaturen zwischen —40 und +40°C zur Reaktion gebracht werden oder

c) zur Herstellung von Verbindungen der allgemeinen Formel I, in der D entweder die Gruppe —SHet mit den oben für Het angegebenen Bedeutungen oder die Pyridinium- oder Aminocarbonylpyridiniumgruppe bedeutet, und E für Wasserstoff steht, eine Verbindung der allgemeinen Formel VI

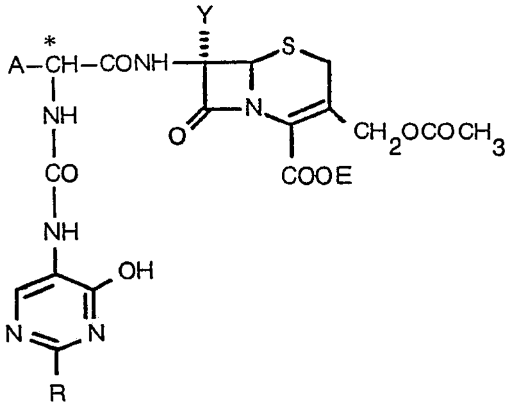

(VI)

in der A, R und Y die oben angegebenen Bedeutungen haben und E Wasserstoff bedeutet, entweder mit einer Verbindung der allgemeinen Formel VII

Het—S—M (VII)

in der Het die oben angegebenen Bedeutungen hat und M für ein Wasserstoffatom oder ein Alkalimetall- oder ein Erdalkalimetall atom steht oder mit Pyridin bzw. 4-Aminocarbonylpyridin in einem Lösungsmittel bei Temperaturen zwischen 0 und 100°C in einem pH-Bereich zwischen 2 und 10 zu einer Verbindung der allgemeinen Formel I, in der D die Bedeutungen einer —SHet, Pyridinium- oder 4-Aminocarbonylpyridiniumgruppe und E die eines Wasserstoffatoms besitzt, umgesetzt wird oder

d) zur Herstellung einer Verbindung der allgemeinen Formel I, in der Y die Methoxygruppe darstellt, eine Verbindung der allgemeinen Formel I, in der Y ein Wasserstoffatom bedeutet, in Anwesenheit von Methanol mit einem Alkalimetall-methylat der allgemeinen Formel $M^{\oplus}OCH_3^{\ominus}$, worin $M^{\oplus}$ ein Alkalimetall ion bedeutet, und dann mit einem Halogenierungsmittel in einem inerten Lösungsmittel bei Temperaturen zwischen −120 und −10°C umgesetzt wird und, gewünschtenfalls anschließend eine so erhaltene Verbindung der allgemeinen Formeln I bzw. I', worin E eine in vitro oder in vivo leicht abspaltbare Schutzgruppe darstellt, in die freie Carbonsäure der allgemeinen Formeln I bzw. I', in der E ein Wasserstoffatom ist, übergeführt wird und/oder eine Verbindung der allgemeinen Formeln I bzw. I', in der E ein Wasserstoffatom bedeutet, in ihre Ester oder, mittels anorganischer oder organischer Basen, in die entsprechenden Salze übergeführt wird.

**Patentansprüche den Vertragsstaat: AT**

1. Verfahren zur Herstellung der Cephalosporine der allgemeinen Formeln I bzw.I'

(I) 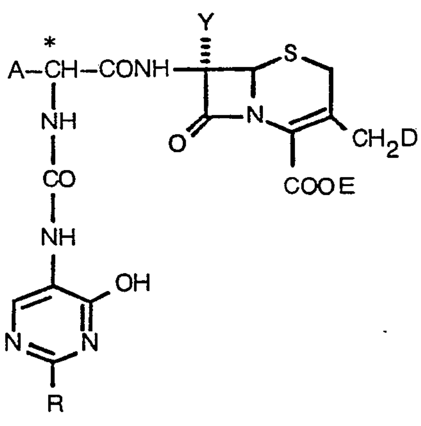

bzw.

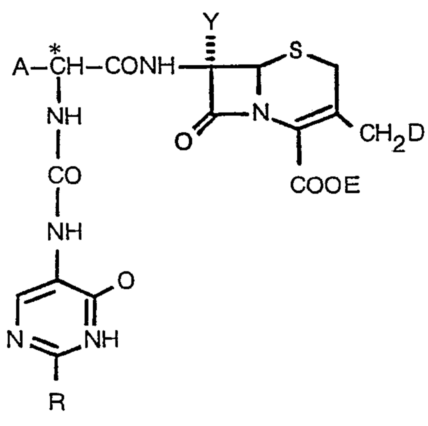 (I')

in welchen A, D, E, Y und R die folgenden Bedeutungen besitzen:

A die Phenyl-, 4-Hydroxyphenyl-, 3- oder 3-Thienyl-, 2-Furyl- oder 3,4-Dihydroxyphenylgruppe,

Y ein Wasserstoffatom oder die Methoxygruppe,

D ein Wasserstoffatom, die Acetoxy-, Aminocarbonyloxy-, Pyridinium- oder Aminocarbonylpyridiniumgruppe oder die Gruppe —S—Het, in der Het die 1-Methyl-tetrazol-5-yl-, Tetrazol-5-yl-, 3-Methyl-1,2,4-thiadiazol-5-yl-, 1,2,4-Thiadiazol-5-yl-, 1,3,4-Thiadiazol-5-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder 1,2,4-Triazol-3-yl-Gruppe bedeutet,

R ein Wasserstoffatom, den Cyclopropylrest, die Hydroxygruppe, die Methoxygruppe, eine Gruppe der Allgemeinen Formel

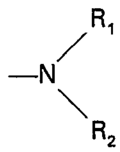

in der $R_1$ und $R_2$ gleich oder voneinander verschieden sein können und Wasserstoffatome, aliphatische, verzweigte oder unverzweigte Kohlenwasserstoffreste, die eventuell eine oder zwei Doppelbindungen oder eine Dreifachbindung und 1 bis 4 Kohlenstoffatome enthalten können, Cycloalkylreste mit 3 bis 6 Kohlenstoffatomen, durch einen Cycloalkylrest substituierte Methylgruppen mit 3 bis 6 Kohlenstoffatomen im Cycloalkylrest darstellen,

R bedeutet aber auch eine Gruppe der allgemeinen Formel —NH—Z—X, in der

Z eine geradkettige oder verzweigte Alkylgruppe mit 1 bis 3 Kohlenstoffatomen oder eine durch den Substituenten X substituierte Cycloalkylgruppe mit 5 oder 6 Kohlenstoffatomen bedeutet und

X die Hydroxygruppe, die Aminocarbonyl-, die Aminosulfonylgruppe oder die Aminocarbonylaminogruppe oder eine Gruppe der allgemeinen Formeln

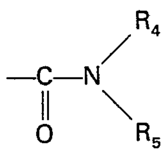 $—NHCOR_5$, 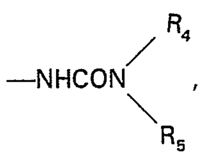 , $—NHSO_2R_4$,

$—OR_4$, $—OCOR_5$, $—SR_4$, $—SO—R_4$ und $—SO_2—R_4$

sein kann, wobei in diesen Formeln

$R_4$ eine geradkettige oder verzweigte Alkylgruppe mit 1 bis 3 Kohlenstoffatomen und $R_5$ Wasserstoff oder eine Alkylgruppe mit 1 bis 3 Kohlenstoffatomen darstellen,

R bedeutet weiter eine Gruppe der allgemeinen Formel

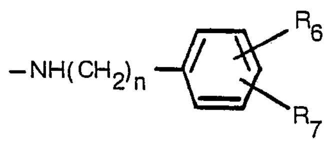

in der n null oder 1 ist und $R_6$ und $R_7$, die gleich oder voneinander verschieden sein können, Wasserstoff, Halogen, Alkylamino-, Dialkylamino-, Hydroxy-, Alkoxy-, aliphatische Acylamino-, Aminocarbonylamino-, Nitro-, Alkylsulfonylamino-, Alkylcarbonyl-, Aminocarbonyl-, Alkylsulfinyl-, Alkylsulfonyl-, Aminosulfonyl- oder Alkyl- und Dialkylaminosulfonylgruppen bedeuten, wobei jeder Alkylrest in den genannten Gruppen 1 bis 3 Kohlenstoffatome enthalten kann,

E ein Wasserstoffatom oder eine in vitro oder vivo leicht abspaltbare Schutzgruppe, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können, und, falls E Wasserstoff bedeutet, von deren physiologisch verträglichen Salzen mit anorganischen oder organischen Basen oder sofern D jedoch für Pyridinium oder Aminocarbonylpyridinium steht, von Verbindungen der allgemeinen Formel Ia

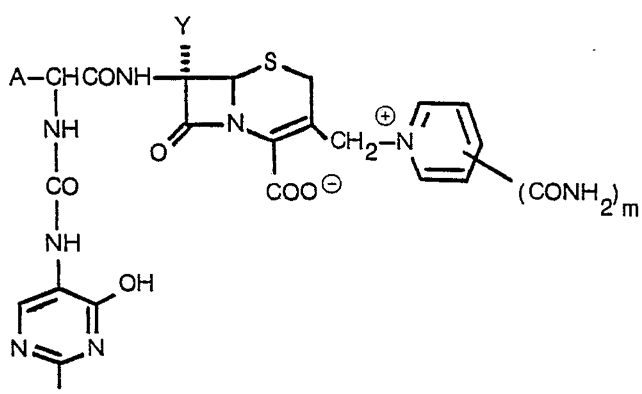 (Ia)

in der A, Y und R wie oben definiert sind und m die Zahl 0 oder 1 darstellt, dadurch gekennzeichnet, daß

a) zur Herstellung von Verbindungen der allgemeinen Formel I, in der D die angegebene Bedeutung mit Ausnahme der einer Pyridinium- oder Aminocarbonylpyridiniumgruppe besitzt, eine Verbindung der allgemeinen Formel II

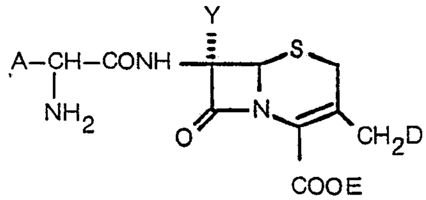

(II)

in der A, Y und E die oben angegebene Bedeutung und D die oben angegebene Bedeutung mit Ausnahme der einer Pyridinium- oder Aminocarbonylpyridiniumgruppe besitzen, mit einem Pyrimidinderivat der allgemeinen Formel III

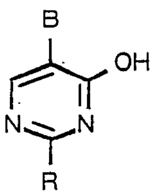

(III)

in der R wie oben definiert ist und B die Gruppe —NCO oder ein reaktives Derivat der Gruppe —NHCOOH bedeutet, wie die Gruppen —NHCOCl, —NHCOBr oder

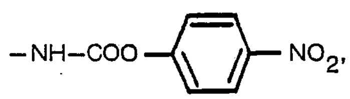

oder mit Gemischen von solchen Pyrimidinderivaten der allgemeinen Formel III, in der B teils die eine und teils die andere der vorstehend genannten Bedeutungen besitzt, bei Temperaturen zwischen —20 und +50°C in einem pH-Bereich zwischen 2,0 und 9,0 umgesetzt wird oder

b) zur Herstellung von Verbindungen der allgemeinen Formel I, in der D die oben angegebenen Bedeutungen mit Ausnahme einer Pyridinium- oder Aminocarbonylpyridiniumgruppe besitzt, Ureidocarbonsäuren der allgemeinen Formel IV

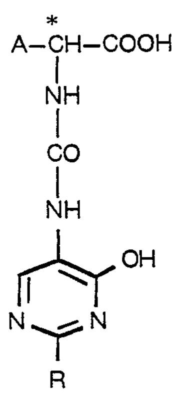

(IV)

in der A und R die oben angegebenen Bedeutungen besitzen, oder ihre Salze oder reaktive Derivate mit 7-Amino-cephalosporansäurederivaten der allgemeinen Formel V

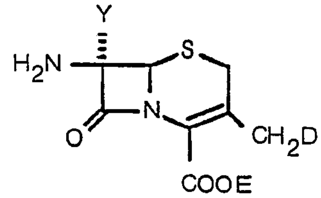

(V)

in der E und Y wie oben definiert sind, und D die oben angegebenen Bedeutungen mit Ausnahme der einer Pyridinium- oder Aminocarbonylpyridiniumgruppe besitzt, in einem Lösungsmittel bei Temperaturen zwischen —40 und +40°C zur Reaktion gebracht werden und, gewünschtenfalls anschließend

c) zur Herstellung von Verbindungen der allgemeinen Formel I, in der D entweder die Gruppe

—SHet mit den oben für Het angegebenen Bedeutungen oder die Pyridinium- oder Aminocarbonylpyridiniumgruppe bedeutet, und E für Wasserstoff steht, eine Verbindung der allgemeinen Formel VI

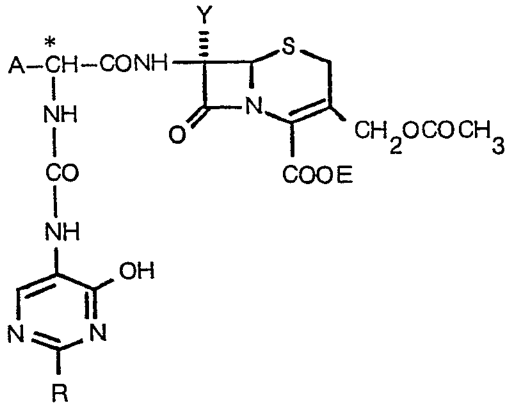

(VI)

in der A, R und Y die oben angegebenen Bedeutungen haben und E Wasserstoff bedeutet, entweder mit einer Verbindung der allgemeinen Formel VII

Het—S—M (VII)

in der Het die oben angegebenen Bedeutungen hat und M für ein Wasserstoffatom oder ein Alkalimetall- oder ein Erdalkalimetall atom steht oder mit Pyridin bzw. 4-Aminocarbonylpyridin in einem Lösungsmittel bei Temperaturen zwischen 0 und 100°C in einem pH-Bereich zwischen 2 und 10 zu einer Verbindung der allgemeinen Formel I, in der D die Bedeutungen einer —SHet, Pyridinium- oder 4-Aminocarbonylpyridiniumgruppe und E die eines Wasserstoffatoms besitzt, umgesetzt wird oder

d) zur Herstellung einer Verbindung der allgemeinen Formel I, in der Y die Methoxygruppe darstellt, eine Verbindung der allgemeinen Formel I, in der Y ein Wasserstoffatom bedeutet, in Anwesenheit von Methanol mit einem Alkalimetall-methylat der allgemeinen Formel $M^{\oplus}OCH_3^{\ominus}$, worin $M^{\oplus}$ ein Alkalimetall ion bedeutet, und dann mit einem Halogenierungsmittel in einem inerten Lösungsmittel bei Temperaturen zwischen —120 und —10°C umgesetzt wird und, gewünschtenfalls anschließend eine so erhaltene Verbindung der allgemeinen Formeln I bzw. I', worin E eine in vitro oder in vivo leicht abspaltbare Schutzgruppe darstellt, in die freie Carbonsäure der allgemeinen Formeln I bzw. I', in der E ein Wasserstoffatom ist, übergeführt wird und-oder eine Verbindung der allgemeinen Formeln I bzw. I', in der E ein Wasserstoffatom bedeutet, in ihre Ester oder, mittels anorganischer oder organischer Basen, in die entsprechenden Salze übergeführt wird.

2. Verfahren gemäß Anspruch 1a, dadurch gekennzeichnet, daß zur Herstellung von Verbindungen der allgemeinen Formeln I bzw. I', in der E ein Wasserstoffatom bedeutet, die Ausgangsprodukte der allgemeinen Formel II in Form ihrer anorganischen oder organischen Salze eingesetzt werden und die Umsetzung mit Pyrimidinderivaten der allgemeinen Formel II entweder in Wasser oder in Mischungen von Wasser mit in Wasser mischbaren organischen Lösungsmitteln unter Zusatz von Basen oder in einem Gemenge aus Wasser und mit Wasser nichtmischbaren Lösungsmitteln in einem pH-Bereich zwischen 6,5 und 8,0 durchgeführt wird.

3. Verfahren gemäß Anspruch 1a, dadurch gekennzeichnet, daß Silylderivate und Derivate mit leicht abspaltbaren Schutzgruppen der Verbindungen der allgemeinen Formel II mit Pyrimidinen bzw. deren Derivaten der allgemeinen Formel III in wasser- und hydroxylgruppenfreien bzw. aprotischen Lösungsmitteln, gegebenenfalls in Gegenwart von Basen, umgesetzt werden.

4. Verfahren gemäß Anspruch 1b, dadurch gekennzeichnet, daß man als reaktive Derivate der Ureidocarbonsäuren der allgemeinen Formel IV deren Säureanhydride, deren reaktive Ester, deren reaktive Amide, deren Säurenhalogenide oder Säureazide und als 7-Aminocephalosporansäurederivate der allgemeinen Formel V solche Verbindungen verwendet, bei welchen E die eingangs erwähnten Bedeutungen mit Ausnahme der eines Wasserstoffs besitzt, und die Umsetzung in einem Lösungsmittel, gegebenenfalls in Anwesenheit einer Base und/oder eines Kondensationsmittels erfolgt.

5. Verfahren gemäß Anspruch 1c, dadurch gekennzeichnet, daß die Umsetzung in einem stark polaren Lösungsmittel bei einem pH-Wert von 4 bis 8 Durchgeführt wird.

6. Verfahren gemäß Anspruch 1d, dadurch gekennzeichnet, daß eine Verbindung der allgemeinen Formeln I, I' oder Ia, worin Y ein Wasserstoffatom bedeutet, in einem inerten Mittel gelöst oder suspendiert, mit 2 bis 6 Äquivalenten eines Alkalimetallmethylats in überschüssigem Methanol zusammengebracht und anschließend mit einem Halogenierungsmittel bei Temperaturen zwischen —100 und —50°C umgesetzt und die Reaktion nach erfolgter Halogenierung durch Säurezusatz abgebrochen wird.

7. Verfahren gemäß Anspruch 1, dadurch gekennzeichnet, daß ein Alkalisalz der Cephalosporansäure der allgemeinen Formeln I bzw I' mit einem Pivaloyloxymethylhalogenid oder Halogenmethylacetat, Halogenmethylpropionat oder Halogenäthylacetat zu dessen entsprechenden Estern umgesetzt wird.

8. Verfahren gemäß Anspruch 1 zur Herstellung eines Indanyl- oder Phthalidylesters der allgemeinen Formeln I bzw. I', dadurch gekennzeichnet, daß man eine Säure der allgemeinen Formeln I bzw. I', in denen E ein Wasserstoffatom bedeutet, in Gegenwart eines Kondensationsmittels mit 5-Indanol in einem inerten Lösungsmittel bei 20 bis 35°C oder ein Salz einer Säure der allgemeinen Formeln I bzw. I' mit Bromphthalid in einem polaren organischen Lösungsmittel umsetzt.

**Claims for the Contracting States: BE CH DE FR GB IT LI LU NL SE**

1. Cephalosporins of general formula I or I'

(I) 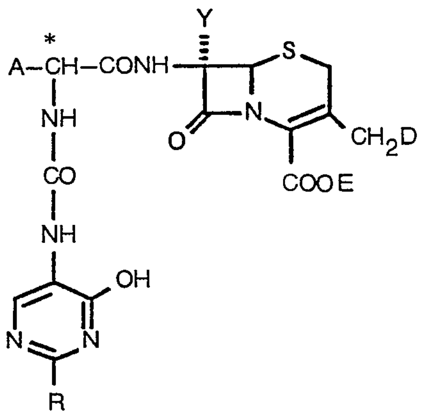

or

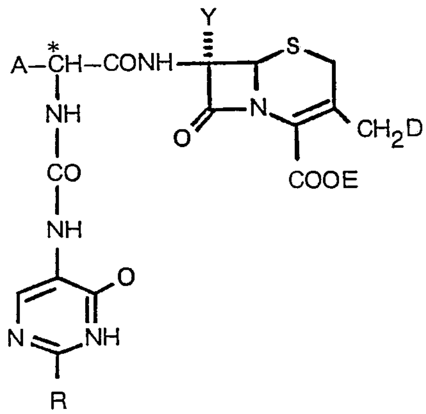 (I')

wherein A, D, E, Y and R have the following meanings:

A represents a phenyl, 4-hydroxyphenyl, 2- or 3-thienyl, 2-furyl or 3,4-dihydroxyphenyl group,

Y represents a hydrogen atom or a methoxy group,

D represents a hydrogen atom, an acetoxy, aminocarbonyloxy, pyridinium or aminocarbonylpyridinium group or the group —S—Het, wherein Het represents a 1-methyl-tetrazol-5-yl, tetrazol-5-yl, 3-methyl-1,2,4-thiadiazol-5-yl, 1,2,4-thiadiazol-5-yl, 1,3,4-thiadiazol-5-yl, 2-methyl-1,3,4-thiadiazol-5-yl, 2-methylamino-1,3,4-thiadiazol-5-yl, 2-dimethylamino-1,3,4-thiadiazol-5-yl, 2-formylamino-1,3,4-thiadiazol-5-yl, 2-acetylamino-1,3,4-thiadiazol-5-yl, 2-methyl-1,3,4-oxadiazol-5-yl, 1,2,3-triazol-4-yl or 1,2,4-triazol-3-yl group,

R represents a hydrogen atom, a cyclopropyl group, a hydroxy group, a methoxy group, a group of general formula

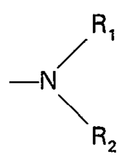

wherein $R_1$ and $R_2$ may be the same or different and represent hydrogen atoms, aliphatic, branched or unbranched hydrocarbon groups which may optionally contain 1 or 2 double bonds or a triple bond and 1 to 4 carbon atoms, cycloalkyl groups with 3 to 6 carbon atoms, methyl groups substituted by a cycloalkyl group with 3 to 6 carbon atoms in the cycloalkyl group,

but R may also represent a group of general formula —NH—Z—X wherein

Z represents a straight-chained or branched alkyl group with 1 to 3 carbon atoms or a cycloalkyl group with 5 or 6 carbon atoms substituted by substituent X, and

X may be a hydroxy group, an aminocarbonyl, aminosulphonyl or aminocarbonylamino group or a group of general formula

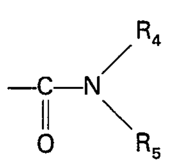 $—NHCOR_5$, 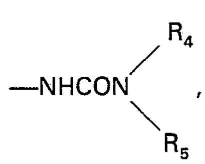 , $—NHSO_2R_4$,

$—OR_4$, $—OCOR_5$, $—SR_4$, $—SO—R_4$ or $—SO_2—R_4$

whilst in these formulae

$R_4$ represents a straight-chained or branched alkyl group with 1 to 3 carbon atoms and $R_5$ represents hydrogen or an alkyl group with 1 to 3 carbon atoms,

R further represents a group of general formula

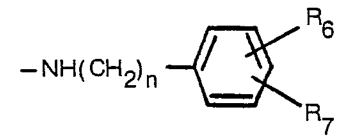

wherein n represents 0 or 1 and $R_6$ and $R_7$, which may be the same or different, represent hydrogen, halogen, alkylamino, dialkylamino, hydroxy, alkoxy, aliphatic acylamino, aminocarbonylamino, nitro, alkylsulfphonylamino, alkylcarbonyl, aminocarbonyl, alkylsulphinyl, alkylsulphonyl, aminosulphonyl or alkyl- and dialkylaminosulphonyl groups, whilst each alkyl group in the above mentioned groups may contain 1 to 3 carbon atoms.

E represents a hydrogen atom or a protecting group which is easily split off *in vitro* or *in vivo*, more particularly an ester-forming group which may be removed by hydrogenolysis or hydrolysis or other treatments under mild conditions, or an ester-forming group which can easily be split off in the living organism and, if E represents hydrogen, the physiologically acceptable salts thereof with inorganic or organic bases, but if D represents pyridinium or aminocarbonylpyridinium the compounds have the general formula Ia

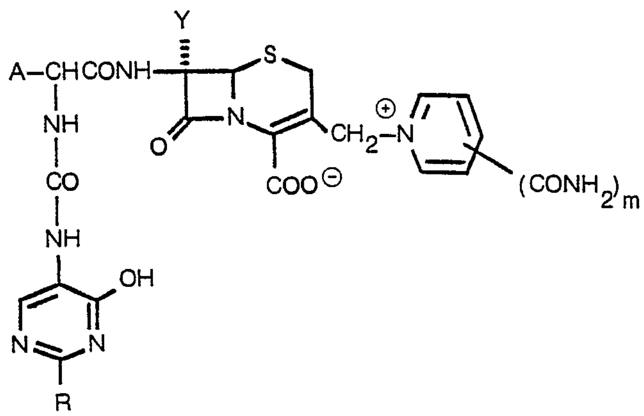

(Ia)

wherein D, Y and R as hereinbefore defined and m represents the number 0 or 1.

2. Cephalosporins of general formula I, I' or Ia as claimed in claim 1, wherein

A represents a 4-hydroxyphenyl or 2-thienyl group,

Y represents a hydrogen atom or a methoxy group,

D represents an acetoxy or pyridinium group or the group —S—Het wherein Het represents a 1-methyl-tetrazol-5-yl, 2-methyl-1,3,4-thiadiazol-5-yl, 2-methylamino-1,3,4-thiadiazol-5-yl or 2-acetyl-amino-1,3,4-thiadiazol-5-yl group,

R represents a cyclopropyl, n-propylamino, 4-hydroxy-cyclohexylamino or 3-hydroxy-n-propylamino group or a group of general formula

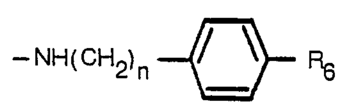

wherein n is 0 or 1 and $R_6$ represents a hydrogen atom, a hydroxy, aminocarbonyl, aminosulphonyl or methylaminosulphonyl group, and

E represents a hydrogen atom or an ester group as mentioned in claim 1 and, if E is a hydrogen atom, the physiologically acceptable salts thereof with inorganic or organic bases.

3. Cephalosporins of general formula I or I' as claimed in claims 1 and 2, characterised in that E represents a benzyl, diphenylmethyl, trityl, t-butyl, 2,2,2-trichloroethyl or trimethylsilyl group, an alkanoyloxyalkyl group with 1 to 5 carbon atoms in the alkanoyl group and 1 to 3 carbon atoms in the alkylene group, or the phthalidyl or idanyl group.

4. Cephalosporins of general formula I or I' as claimed in claim 3, characterised in that E represents an acetoxymethyl, propionyloxymethyl, 2-acetoxyethyl or pivaloyloxymethyl group.

5. Cephalosporins of general formula I, I' or Ia as claimed in claims 1 to 4, characterised in that these compounds have the D=R configuration.

6. Sodium - 7 - $\beta$ - D - $\alpha$ - [(2 - p - aminosulphonylanilino - 4 - hydroxy - 5 - pyrimidinyl) - ureido] - p - hydroxy - phenylacetamido - 7$\alpha$ - methoxy - 3 - [(1 - methyl - tetrazol - 5 - yl) - thiomethyl] - ceph - 3 - em - 4 - carboxylate.

7. 7 - D - $\alpha$ - [(2 - p - aminosulphonylanilino - 4 - hydroxy - 5 - pyrimidinyl) - ureido] - p - hydroxy - phenylacetamido - 3 - (pyridinomethyl) - ceph - 3 - em - 4 - carboxylate.

8. Pharmaceutical compositions containing one or more compounds of general formula I, I' or Ia together with the conventional carriers and/or excipients.

9. Process for the preparation of the cephalosporins of general formula I or I'

(I) 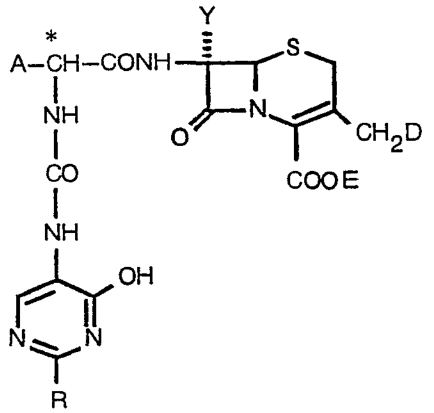

or

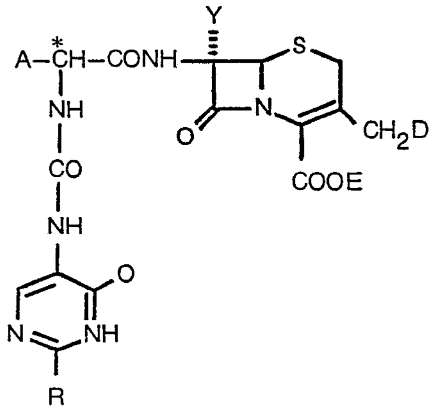 (I')

wherein A, D, E, Y and R are defined as in claim 1 and, if E represents a hydrogen, of the physiologically acceptable salts thereof with inorganic or organic bases or, if D represents pyridinium or aminocarbonylpyridinium, of compounds of general formula Ia

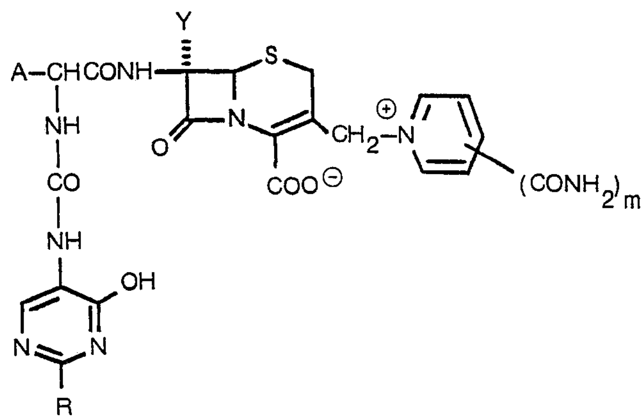 (Ia)

wherein A, Y and R are as hereinbefore defined and m represents the number 0 or 1, characterised in that

a) in order to prepare compounds of general formula I wherein D has the meanings given above, with the exception of a pyridinium or aminocarbonylpyridinium group, a compound of general formula II

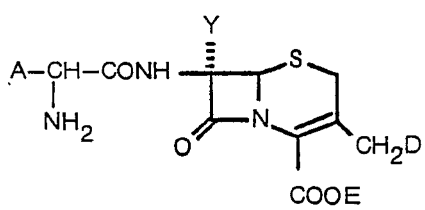 (II)

wherein A, Y and E are as hereinbefore defined and D has the above meanings with the exception of a pyridinium or aminocarbonylpyridinium group, is reacted with a pyrimidine derivative of general formula III

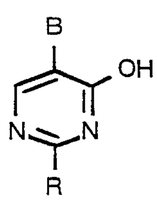 (III)

wherein R is as hereinbefore defined and B represents the group —NCO or a reactive derivative of the group —NHCOOH, such as the groups

—NHCOCl, —NHCOBr or 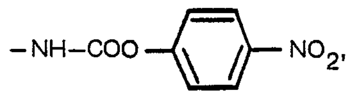

or with mixtures of pyrimidine derivatives of general formula III wherein B is defined partly according to the first and partly according to the second of the two definitions given above, at temperatures of between −20 and +50°C in a pH range of between 2.0 and 9.0, or

b) in order to prepare compounds of general formula I wherein D has the above meanings with the exception of a pyridinium or aminocarbonylpyridinium group, ureidocarboxylic acids of general formula IV

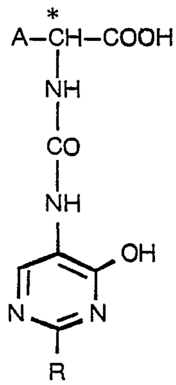

(IV)

wherein A and R are as hereinbefore defined, or the salts or reactive derivatives thereof are reacted with 7-amino-cephalosporanic acid derivatives of general formula V

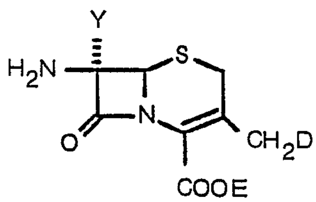

(V)

wherein E and Y are as hereinbefore defined and D has the above meanings with the exception of a pyridinium or aminocarbonylpyridinium group, in a solvent at temperatures of between −40 and +40°C, or

c) in order to prepare compounds of general formula I wherein D represents either the group —SHet wherein Het is as hereinbefore defined or the pyridinium or aminocarbonylpyridinium group, and E represents hydrogen, a compound of general formula VI

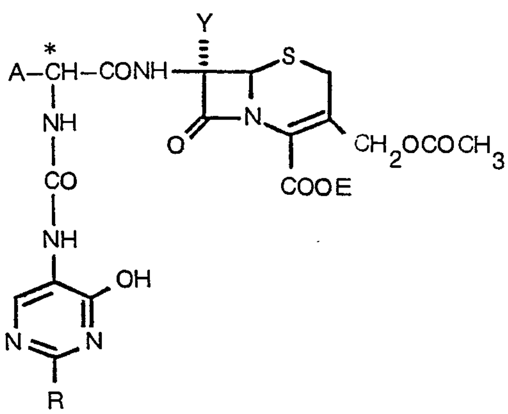

(VI)

wherein A, R and Y are as hereinbefore defined and E represents hydrogen, is reacted either with a compound of general formula VII

Het—S—M (VII)

wherein Het is as hereinbefore defined and M represents a hydrogen atom or an alkali metal or alkaline earth metal atom or with pyridine or 4-aminocarbonylpyridine in a solvent at temperatures of between 0 and 100°C in a pH range of between 2 and 10 to yield a compound of general formula I wherein D represents an —SHet, pyridinium or 4-aminocarbonylpyridinium group and E represents a hydrogen atom, or

d) in order to prepare a compound of general formula I wherein Y represents a methoxy group, a compound of general formula I wherein Y represents a hydrogen atom is reacted in the presence of methanol with an alkali metal methoxide of general formula $M^{\oplus}OCH_3^{\ominus}$, wherein $M^{\oplus}$ represents an alkali metal ion, and then with a halogenating agent in an inert solvent at temperatures of between −120 and −10°C and, if desired, a compound of general formula I or I' thus obtained wherein E represents a protecting group which can easily be split off *in vitro* or *in vivo* is converted into the free carboxylic acid of general formula I or I' wherein E is a hydrogen atom, and/or a compound of general formula I or I' wherein E represents a hydrogen atom may be converted into the esters thereof or into the corresponding salts by means of inorganic or organic bases.

**Claims for the Contracting State: AT**

1. Process for the preparation of cephalosporins of general formula I or I'

(I) 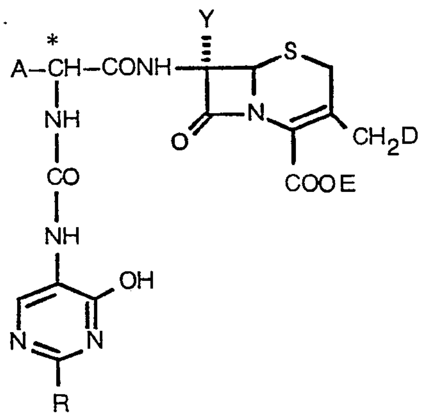

or

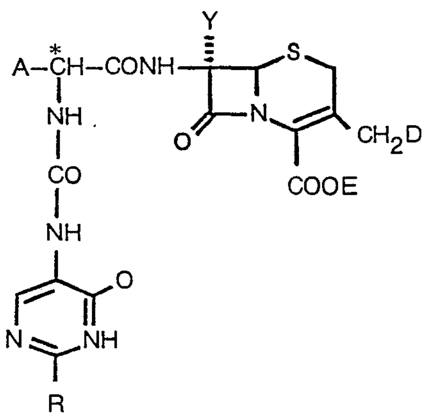 (I')

wherein A, D, E, Y and R have the following meanings:

A represents a phenyl, 4-hydroxyphenyl, 2- or 3-thienyl, 2-furyl or 3,4-dihydroxyphenyl group,

Y represents a hydrogen atom or a methoxy group,

D represents a hydrogen atom, an acetoxy, aminocarbonyloxy, pyridinium or aminocarbonylpyridinium group or the group —S—Het, wherein Het represents a 1-methyl-tetrazol-5-yl, tetrazol-5-yl, 3-methyl-1,2,4-thiadiazol-5-yl, 1,2,4-thiadiazol-5-yl, 1,3,4-thiadiazol-5-yl, 2-methyl-1,3,4-thiadiazol-5-yl, 2-methylamino-1,3,4-thiadiazol-5-yl, 2-dimethylamino-1,3,4-thiadiazol-5-yl, 2-formylamino-1,3,4-thiadiazol-5-yl, 2-acetylamino-1,3,4-thiadiazol-5-yl, 2-methyl-1,3,4-oxadiazol-5-yl, 1,2,3-triazol-4-yl or 1,2,4-triazol-3-yl group,

R represents a hydrogen atom, a cyclopropyl group, a hydroxy group, a methoxy group, a group of general formula

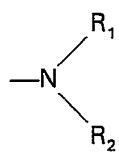

wherein $R_1$ and $R_2$ may be the same or different and represent hydrogen atoms, aliphatic, branched or unbranched hydrocarbon groups which may optionally contain 1 or 2 double bonds or a triple bond and 1 to 4 carbon atoms, cycloalkyl groups with 3 to 6 carbon atoms, methyl groups substituted by a cycloalkyl group with 3 to 6 carbon atoms in the cycloalkyl group,

but R may also represent a group of general formula —NH—Z—X wherein

Z represents a straight-chained or branched alkyl group with 1 to 3 carbon atoms or a cycloalkyl group with 5 or 6 carbon atoms substituted by substituent X, and

X may be a hydroxy group, an aminocarbonyl, aminosulphonyl or aminocarbonylamino group or a group of general formula

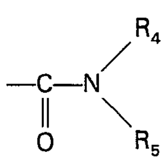 $—NHCOR_5$, 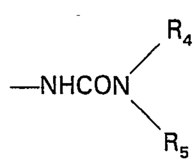 , $—NHSO_2R_4$,

$—OR_4$, $—OCOR_5$, $—SR_4$, $—SO—R_4$ or $—SO_2—R_4$

whilst in these formulae

$R_4$ represents a straight-chained or branched alkyl group with 1 to 3 carbon atoms and $R_5$ represents hydrogen or an alkyl group with 1 to 3 carbon atoms,

R further represents a group of general formula

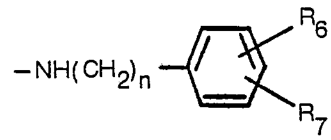

wherein n represents 0 or 1 and $R_6$ and $R_7$, which may be the same or different, represent hydrogen, halogen, alkylamino, dialkylamino, hydroxy, alkoxy, aliphatic acylamino, aminocarbonylamino, nitro, alkylsulfphonylamino, alkylcarbonyl, aminocarbonyl, alkylsulphinyl, alkylsulphonyl, aminosulphonyl or alkyl- and dialkylaminosulphonyl groups, whilst each alkyl group in the above mentioned groups may contain 1 to 3 carbon atoms.

E represents a hydrogen atom or a protecting group which is easily split off *in vitro* or *in vivo*, more particularly an ester-forming group which may be removed by hydrogenolysis or hydrolysis or other treatments under mild conditions, or an ester-forming group which can easily be split off in the living organism and, if E represents hydrogen, the physiologically acceptable salts thereof with inorganic or organic bases or if D represents pyridinium or aminocarbonylpyridinium, compounds of general formula Ia

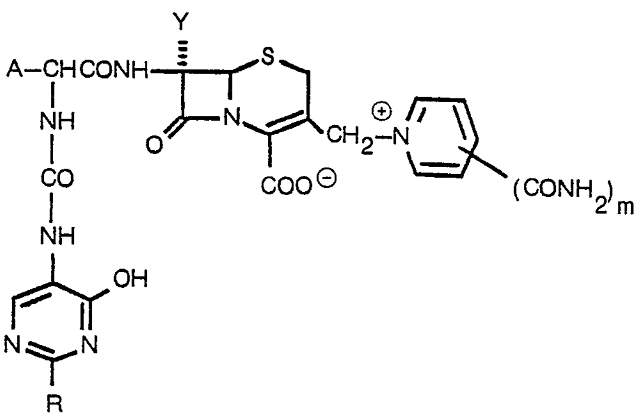

(Ia)

wherein A, Y and R are as hereinbefore defined and m represents the number 0 or 1, characterised in that

a) in order to prepare compounds of general formula I wherein D has the meanings given above, with the exception of a pyridinium or aminocarbonylpyridinium group, a compound of general formula II

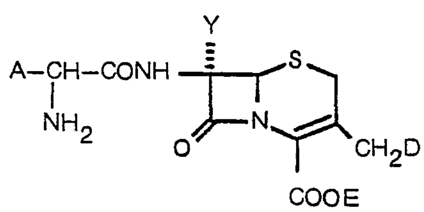

(II)

wherein A, Y and E are as hereinbefore defined and D has the above meanings with the exception of a pyridinium or aminocarbonylpyridinium group, is reacted with a pyrimidine derivative of general formula III

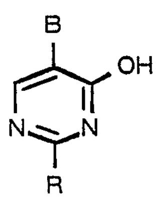

(III)

wherein R is as hereinbefore defined and B represents the group —NCO or a reactive derivative of the group —NHCOOH, such as the groups

—NHCOCl, —NHCOBr or 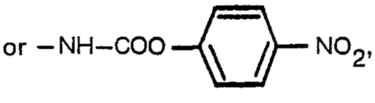

or with mixtures of pyrimidine derivatives of general formula III wherein B is defined partly according to the first and partly according to the second of the two definitions given above, at temperatures of between −20 and +50°C in a pH range of between 2.0 and 9.0, or

b) in order to prepare compounds of general formula I wherein D has the above meanings with the exception of a pyridinium or aminocarbonylpyridinium group, ureidocarboxylic acids of general formula IV

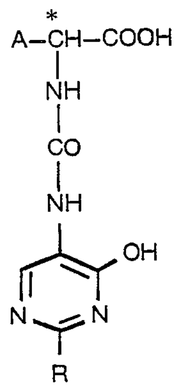

(IV)

wherein A and R are as hereinbefore defined, or the salts or reactive derivatives thereof are reacted with 7-amino-cephalosporanic acid derivatives of general formula V

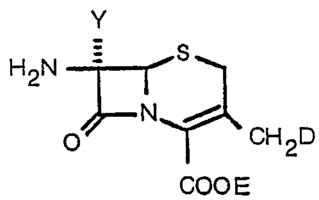

(V)

wherein E and Y are as hereinbefore defined and D has the above meanings with the exception of a pyridinium or aminocarbonylpyridinium group, in a solvent at temperatures of between −40 and +40°C, or

c) in order to prepare compounds of general formula I wherein D represents either the group —SHet wherein Het is as hereinbefore defined or the pyridinium or aminocarbonylpyridinium group, and E represents hydrogen, a compound of general formula VI

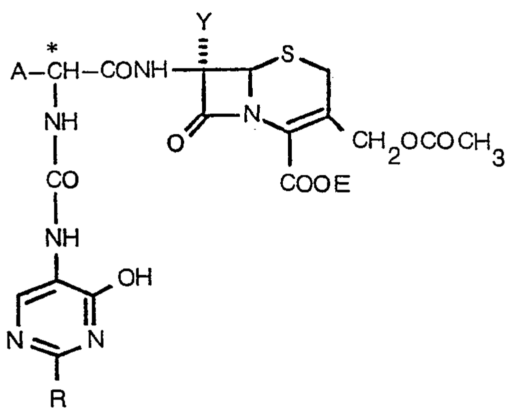

(VI)

wherein A, R and Y are as hereinbefore defined and E represents hydrogen, is reacted either with a compound of general formula VII

Het—S—M (VII)

wherein Het is as hereinbefore defined and M represents a hydrogen atom or an alkali metal or alkaline earth metal atom or with pyridine or 4-aminocarbonylpyridine in a solvent at temperatures of between 0 and 100°C in a pH range of between 2 and 10 to yield a compound of general formula I wherein D represents an —SHet, pyridinium or 4-aminocarbonylpyridinium group and E represents a hydrogen atom, or

d) in order to prepare a compound of general formula I wherein Y represents a methoxy group, a compound of general formula I wherein Y represents a hydrogen atom is reacted in the presence of methanol with an alkali metal methoxide of general formula $M^{\oplus}OCH_3^{\ominus}$, wherein $M^{\oplus}$ represents an alkali metal ion, and then with a halogenating agent in an inert solvent at temperatures of between −120

and −10°C and, if desired, a compound of general formula I or I′ thus obtained wherein E represents a protecting group which can easily be split off *in vitro* or *in vivo* is converted into the free carboxylic acid of general formula I or I′ wherein E is a hydrogen atom, and/or a compound of general formula I or I′ wherein E represents a hydrogen atom may be converted into the esters thereof or into the corresponding salts by means of inorganic or organic bases.

2. Process as claimed in claim 1a, characterised in that in order to prepare compounds of general formula I or I′ wherein E represents a hydrogen atom, the starting products of general formula II are used in the form of their inorganic or organic salts and the reaction with pyrimidine derivatives of general formula II is carried out either in water or in mixtures of water and water-miscible organic solvents with the addition of bases or in a mixture of water and water-immiscible solvents in a pH range of between 6.5 and 8.0.

3. Process as claimed in claim 1a, characterised in that silyl derivatives and derivatives with easily removable protecting groups of the compounds of general formula II are reacted with pyrimidines or the derivatives thereof of general formula III in solvents which are free from water and hydroxyl groups or aprotic solvents, optionally in the presence of bases.

4. Process as claimed in claim 1b, characterised in that the reactive derivatives of the ureidocarboxylic acids of general formula IV used are the acid anhydrides, reactive esters, reactive amides, acid halides or acid azides thereof and the 7-aminocephalosporanic acid derivatives of general formula V used are compounds wherein E has the meanings given hereinbefore with the exception of hydrogen, and the reaction is carried out in a solvent, optionally in the presence of a base and/or a condensing agent.

5. Process as claimed in claim 1c, characterised in that the reaction is carried out in a strongly polar solvent at a pH of from 4 to 8.

6. Process as claimed in claim 1d, characterised in that a compound of general formula I, I′ or Ia, wherein Y represents a hydrogen atom, is dissolved or suspended in an inert agent, then combined with 2 to 6 equivalents of an alkali metal methoxide in excess methanol and subsequently reacted with a halogenating agent at temperatures of between −100 and −50°C and after halogenation has been carried out the reaction is stopped by the addition of acid.

7. Process as claimed in claim 1, characterised in that an alkali metal salt of cephalosporanic acid of general formula I or I′ is reacted with a pivaloyloxymethyl halide or halomethyl acetate, halomethyl-propionate or haloethyl acetate to form the corresponding esters thereof.

8. Process as claimed in claim 1 for the preparation of an indanyl or phthalidyl ester of general formula I or I′, characterised in that an acid of general formula I or I′ wherein E represents a hydrogen atom is reacted, in the presence of a condensing agent, with 5-indanol in an inert solvent at 20 to 35°C or a salt of an acid of general formula I or I′ is reacted with bromine phthalide in a polar organic solvent.

**Revendications pour les Etats contractants: BE CH DE FR GB IT LI LU NL SE**

1. Céphalosporines de formule générales I ou respectivement I′

(I) 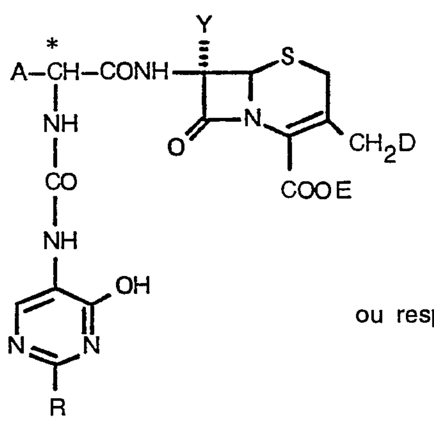

ou respectivement

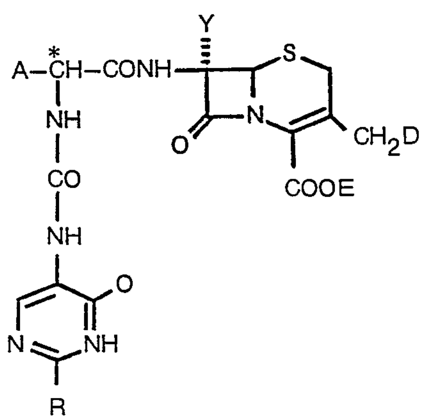 (I′)

dans lesquelles A, D, E, Y et R possèdent les significations suivantes:

A représente le groupe phényle, 4-hydroxyphényle, 2- ou 3-thiényle, 2-furyle ou 3,4-dihydroxy-phényle,

Y représente un atome d'hydrogène ou le groupe méthoxy,

D représente un atome d'hydrogène, le groupe acétoxy, aminocarbonyloxy, pyridinium ou aminocarbonylpyridinium ou le groupe —S—Het, dans lequel Het représente le groupe 1-méthyl-tétrazol-5-yle, tétrazol-5-yle, 3-méthyl-1,2,4-thiadiazol-5-yle, 1,2,4-thiadiazol-5-yle, 1,3,4-thiadiazol-5-yle, 2-méthyl-1,3,4-thiadiazol-5-yle, 2-méthylamino-1,3,4-thiadiazol-5-yle, 2-diméthylamino-1,3,4-thiadiazol-5-yle, 2-formylamino-1,3,4-thiadiazol-5-yle, 2-acétylamino-1,3,4-thiadiazol-5-yle, 2-méthyl-1,3,4-oxadiazol-5-yle, 1,2,3-triazol-4-yle ou 1,2,4-triazol-3-yle,

R représente un atome d'hydrogène, le radical cyclopropyle, le groupe hydroxy, le groupe méthoxy, un groupe de formule générale

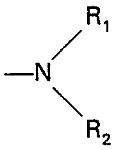

dans laquelle $R_1$ et $R_2$ peuvent être identiques ou différentes l'un de l'autre et représentent des atomes d'hydrogène, des radicaux hydrocarbonés aliphatiques, ramifiés ou non ramifiés qui peuvent contenir éventuellement une ou deux doubles liaisons ou une triple liaison et 1 à 4 atomes de carbone, des radicaux cycloalcoyle avec 3 à 6 atomes de carbone, des groupes méthyle substitués par un radical cycloalcoyle avec 3 à 6 atomes de carbone dans le radical cycloalcoyle,

R représente cependant également un groupe de formule générale —NH—Z—X, dans laquelle

Z représente un groupe alcoyle rectiligne ou ramifié avec 1 à 3 atomes de carbone ou un groupe cycloalcoyle substitué par le substituant X avec 5 ou 6 atomes de carbone et X peut être le groupe hydroxy, le groupe aminocarbonyle, le groupe aminosulfonyle ou le groupe aminocarbonylamino ou un groupe de formules générales

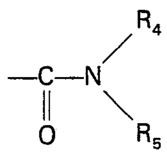 $—NHCOR_5$, 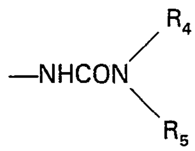 , $—NHSO_2R_4$,

$—OR_4$, $—OCOR_5$, $—SR_4$, $—SO—R_4$ et $—SO_2—R_4$,

dans ces formules

$R_4$ représentant un groupe alcoyle rectiligne ou ramifié avec 1 à 3 atomes de carbone et $R_5$ l'hydrogène ou un groupe alcoyle avec 1 à 3 atomes de carbone,

R représente en outre un groupe de formule générale

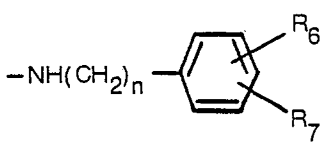

dans laquelle n est zéro ou 1 et $R_6$ et $R_7$ qui peuvent être identiques ou différents l'un de l'autre représentent hydrogène, halogène, des groupes alcoylamino, dialcoylamino, hydroxy, alcoxy, acylamino aliphatique, aminocarbonylamino, nitro, alcoylsulfonyl-amino, alcoylcarbonyle, aminocarbonyle, alcoylsulfinyle, alcoylsulfonyle, aminosulfonyle ou alcoyl- et dialcoylaminosulfonyle, chaque radical alcoyle pouvant contenir dans les groupes mentionnés 1 à 3 atomes de carbone,

E représente un atome d'hydrogène ou un groupe protecteur facilement clivable in vitro ou in vivo, en particulier des groupes formant des esters qui peuvent être éliminés pas hydrogénolyse ou hydrolyse ou d'autres traitements dans des conditions douces ou des groupes formant des esters qui peuvent être facilement clivés dans l'organisme vivant, et, lorsque E représente hydrogène, leurs sels physiologiquement supportables avec des bases minérales ou organiques, cependant si D remplace pyridinium ou aminocarbonylpyridinium, alors les composés ont la formule générale Ia

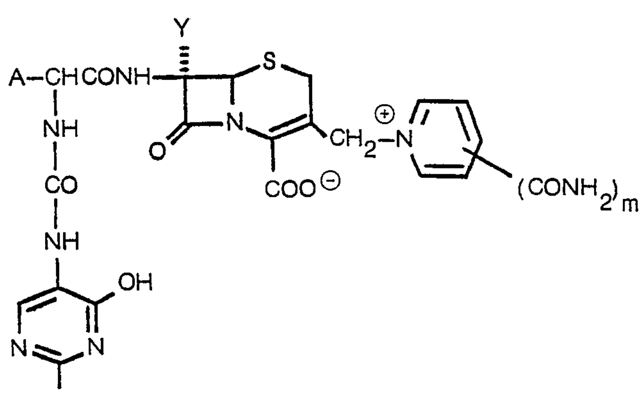

(Ia)

dans laquelle D, Y et R sont définis comme plus haut et m représente le nombre 0 ou 1.

2. Céphalosporines de formules générales I, I' ou Ia selon la revendication 1, dans lesquelles A représente le groupe 4-hydroxyphényle ou 2-thiényle,

Y représente un atome d'hydrogène ou le groupe méthoxy,

D représente le groupe acétoxy ou pyridinium ou le groupe —S—Het, dans lequel Het représente le groupe 1-méthyltétrazol-5-yle, 2-méthyl-1,3,4-thiadiazol-5-yle, 2-méthylamino-1,3,4-thiadiazol-5-yle ou 2-acétylamino-1,3,4-thiadiazol-5-yle.

R représente le groupe cyclopropyle, n-propylamino, 4-hydroxy-cyclohexylamino ou 3-hydroxy-n-propylamino ou un groupe de formule générale

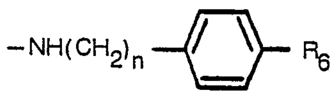

dans laquelle n est zéro ou 1 et $R_6$ un atome d'hydrogène, le groupe hydroxy, aminocarbonyle, aminosulfonyle ou méthylaminosulfonyle et

E représente un atome d'hydrogène ou un groupe ester tel qu'indiqué dans la revendication 1 et, lorsque que E est un atome d'hydrogène, leurs sels physiologiquement supportables avec des bases minérales ou organiques.

3. Céphalosporines de formules générales I ou respectivement I' selon la revendications 1 et 2, caractérisées en ce que E représente le groupe benzyle, diphénylméthyle, trityle, t-butyle, 2,2,2-trichloroéthyle ou triméthylsilyle, un groupe alcanoyloxyalcoyle avec 1 à 5 atomes de carbone dans le radical alcanoyle et 1 à 3 atomes de carbone dans le radical alcoylène, ou le groupe phtalidyle ou indanyle.

4. Céphalosporines de formules générales I ou respectivement I' selon la revendication 3, caractérisées en ce que E représente le groupe acétoxyméthyle, propionyloxyméthyle, 2-acétoxyéthyle ou pivaloyloxyméthyle.

5. Céphalosporines de formules générales I, I' ou Ia selon les revendications 1 à 4, caractérisées en ce que pour ces composés correspond la configuration D=R.

6. Le 7 - $\beta$ - D - $\alpha$ - [(2 - p - aminosulfonylanilino - 4 - hydroxy - 5 - pyrimidinyl) - uréido] - p - hydroxy - phénylacétamido - 7$\alpha$ - méthoxy - 3 - [(1 - méthyl - tétrazol - 5 - yl) - thiométhyl] - céph - 3 - ème - 4 - carboxylate de sodium.

7. Le 7 - $\beta$ - D - $\alpha$ - [(2 - p - aminosulfonylanilino - 4 - hydroxy - 5 - pyrimidinyl) - uréido] - p - hydroxy - phénylacétamido - 3 - (pyridinométhyl) - céph - 3 - ème - 4 - carboxylate.

8. Médicament contenant un ou plusieurs composés de formules générales I, I' ou Ia conjointement aux excipients et/ou adjuvants habituels.

9. Procédé pour la préparation des céphalosporines de formules générales I ou respectivement I'

(I) 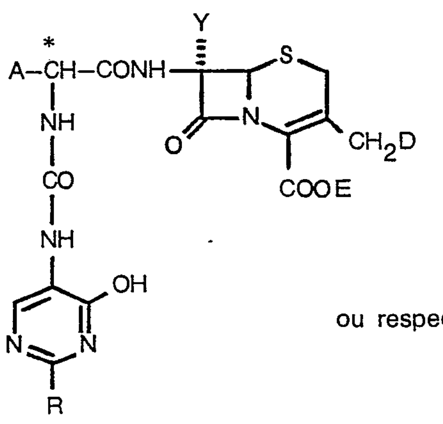

ou respectivement

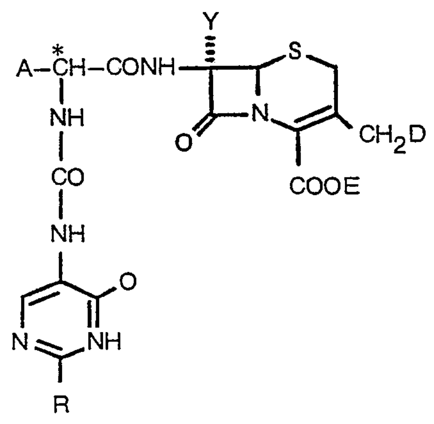 (I')

dans lesquelles A, D, E, Y et R sont définis comme dans la revendication 1, et lorsque E représente un hydrogène, de leurs sels physiologiquement supportables avec des bases minérales ou organiques, dans la mesure où D remplace pyridinium ou aminocarbonylpyridinium, de composés de formule générale Ia

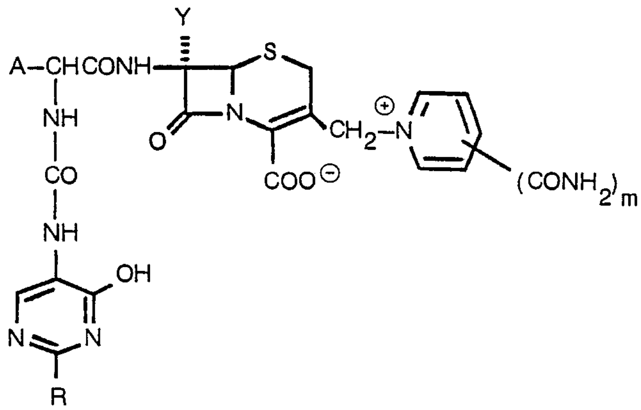

(Ia)

dans laquelle A, Y et R sont définis comme plus haut et m représente le nombre 0 ou 1, caractérisé en ce que

a) pour la préparation de composés de formule générale I dans laquelle D possède la signification indiquée à l'exception de celle d'un groupe pyridinium ou aminocarbonylpyridinium, on fait réagir un composé de formule générale II

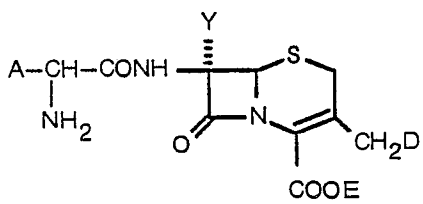

(II)

dans laquelle A, Y et E possèdent la signification indiquée plus haut et D possède la signification indiquée plus haut à l'exception de celle d'un groupe pyridinium ou aminocarbonylpyridinium, avec un dérivé de pyrimidine de formule générale III

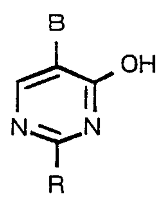

(III)

dans laquelle R est défini comme plus haut et B représente le groupe —NCO ou un dérivé réactif du groupe —NHCOOH, tel que les groupes —NHCOCl, —NHCOBr ou

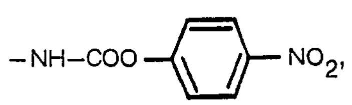

ou avec des mélanges de tels dérivés de pyrimidine de formule générale III dans laquelle B possède en partie l'une et en partie les autres des significations mentionnées plus haut, à des températures entre −20 et +50°C dans un domaine de pH entre 2,0 et 9,0 ou

b) pour la préparation de composés de formule générale I dans laquelle D possède les significations indiquées plus haut à l'exception de celle d'un groupe pyridinium ou aminocarbonylpyridinium, on amène à réaction des acides uréidocarboxyliques de formule générale IV

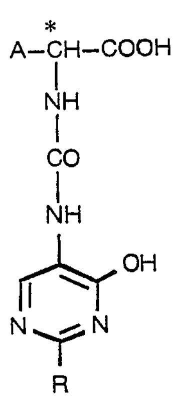

(IV)

dans laquelle A et R possèdent les significations indiquées plus haut, ou leurs sels ou dérivés réactifs avec des dérivés d'acide 7-amino-céphalosporanique de formule générale V

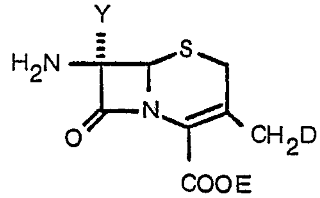

(V)

dans laquelle E et Y sont définis comme plus haut, et D possède les significations indiquées plus haut à l'exception de celle d'un groupe pyridinium ou aminocarbonylpyridinium, dans un solvant à des températures entre −40 et +40°C ou

c) pour la préparation de composés de formule générale I dans laquelle D représente soit le groupe —SHet avec les significations indiquées plus haut pour Het ou le groupe pyridinium ou aminocarbonylpyridinium, et E remplace l'hydrogène, on fait réagir un composé de formule générale VI

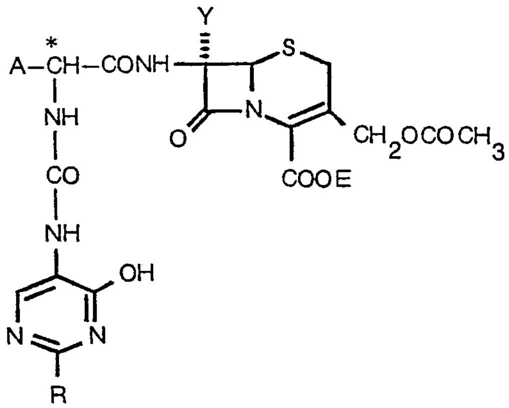

(VI)

dans laquelle A, R et Y ont les significations indiquées plus haut et E représente l'hydrogène, soit avec un composé de formule générale VII

Het—S—M (VII)

dans laquelle Het a les significations indiquées plus haut et M remplace un atome d'hydrogène ou un atome de métal alcalin ou un atome de métal alcalino-terreux soit avec la pyridine ou respectivement la 4-aminocarbonylpyridine dans un solvant à des températures entre 0 et 100°C dans un domaine de pH entre 2 et 10 pour donner un composé de formule générale I dans laquelle D possède les significations d'un groupe —SHet, pyridinium ou 4-aminocarbonylpyridinium et E celle d'un atome d'hydrogène, ou

d) pour la préparation d'un composé de formule générale I dans laquelle Y représente le groupe méthoxy, on fait réagir un composé de formule générale I dans laquelle Y représente un atome d'hydrogène, en présence de méthanol avec un méthylate de métal alcalin de formule générale $M^{\oplus}OCH_3^{\ominus}$, dans laquelle $M^{\oplus}$ représente un ion de métal alcalin, puis avec un agent d'halogénation dans un solvant inerte à des températures entre −120 et −10°C, éventuellement on transforme ensuite un composé ainsi obtenu de formules générales I ou respectivement I' dans lesquelles E représente un groupe protecteur facilement clivable in vitro ou in vivo, en l'acide carboxylique libre de formules générales I ou respectivement I', dans lesquelles E est un atome d'hydrogène et/ou on transforme un composé de formules générales I ou respectivement I' dans lesquelles E représente un atome d'hydrogène en leurs esters ou, au moyen de bases minérales ou organiques, en les sels correspondants.

**Revendications pour l'Etat contractant: AT**

1. Procédé pour la préparation des séphalosporines de formules générales I ou respectivement I'

(I) 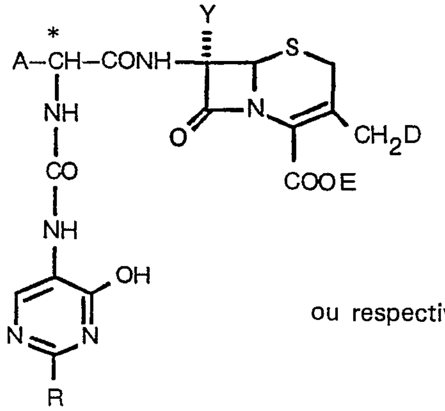 ou respectivement 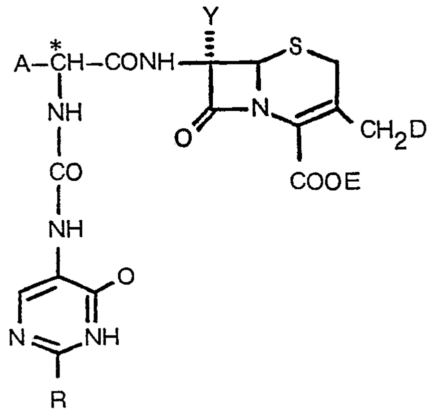 (I')

dans lesquelles A, D, E, Y et R possèdent les significations suivantes:

A représente le groupe phényle, 4-hydroxyphényle, 2- ou 3-thiényle, 2-furyle ou 3,4-dihydroxyphényle,

Y représente un atome d'hydrogène ou le groupe méthoxy,

D représente un atome d'hydrogène, le groupe acétoxy, aminocarbonyloxy, pyridinium ou aminocarbonylpyridinium ou le groupe —S—Het, dans lequel Het représente le groupe 1-méthyl-tétrazol-5-yle, tétrazol-5-yle, 3-méthyl-1,3,4-thiadiazol-5-yle, 1,2,4-thiadiazol-5-yle, 1,3,4-thiadiazol-5-yle, 2-méthyl-1,3,4-thiadiazol-5-yle, 2-méthylamino-1,3,4-thiadiazol-5-yle, 2-diméthylamino-1,3,4-thiadiazol-5-yle, 2-formylamino-1,3,4-thiadiazol-5-yle, 2-acétylamino-1,3,4-thiadiazol-5-yle, 2-méthyl-1,3,4-oxadiazol-5-yle, 1,2,3-triazol-4-yle ou 1,2,4-triazol-3-yle,

R représente un atome d'hydrogène, le radical cyclopropyle, le groupe hydroxy, le groupe méthoxy, un groupe de formule générale

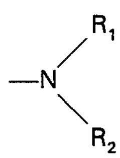

dans laquelle $R_1$ et $R_2$ peuvent être identiques ou différentes l'un de l'autre et représentent des atomes d'hydrogène, des radicaux hydrocarbonés aliphatiques, ramifiés ou non ramifiés qui peuvent contenir éventuellement une ou deux doubles liaisons ou une triple liaison et 1 à 4 atomes de carbone, des radicaux cycloalcoyle avec 3 à 6 atomes de carbone, des groupes méthyle substitués par un radical cycloalcoyle avec 3 à 6 atomes de carbone dans le radical cycloalcoyle,

R représente cependant également un groupe de formule générale —NH—Z—X, dans laquelle

Z représente un groupe alcoyle rectiligne ou ramifié avec 1 à 3 atomes de carbone ou un groupe cycloalcoyle substitué par le substituant X avec 5 ou 6 atomes de carbone et X peut être le groupe hydroxy, le groupe aminocarbonyle, le groupe aminosulfonyle ou le groupe aminocarbonylamino ou un groupe de formules générales

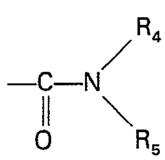 $—NHCOR_5$, 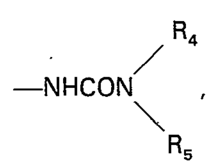 , $—NHSO_2R_4$,

$—OR_4$, $—OCOR_5$, $—SR_4$, $—SO—R_4$ et $—SO_2—R_4$,

dans ces formules

$R_4$ représentant un groupe alcoyle rectiligne ou ramifié avec 1 à 3 atomes de carbone et $R_5$ l'hydrogène ou un groupe alcoyle avec 1 à 3 atomes de carbone,

R représente en outre un groupe de formule générale

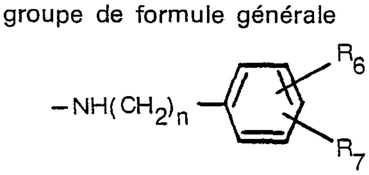

dans laquelle n est zéro ou 1 et $R_6$ et $R_7$ qui peuvent être identiques ou différents l'un de l'autre représentent hydrogène, halogène, des groupes alcoylamino, dialcoylamino, hydroxy, alcoxy, acylamino aliphatique, aminocarbonylamino, nitro, alcoylsulfonylamino, alcoylcarbonyle, aminocarbonyle, alcoylsulfinyle, alcoylsulfonyle, aminosulfonyle ou alcoyl- et dialcoylaminosulfonyle, chaque radical alcoyle pouvant contenir dans les groupes mentionnés 1 à 3 atomes de carbone,

E représente un atome d'hydrogène ou un groupe protecteur facilement clivable in vitro ou in vivo, en particulier des groupes formant des esters qui peuvent être éliminés pas hydrogénolyse ou hydrolyse ou d'autres traitements dans des conditions douces ou des groupes formant des esters qui peuvent être facilement clivés dans l'organisme vivant, et, lorsque E représente hydrogène, leurs sels physiologiquement supportables avec des bases minérales ou organiques ou cependant dans le mesure où D remplace pyridinium ou aminocarbonylpyridinium, de composés de formule générale Ia

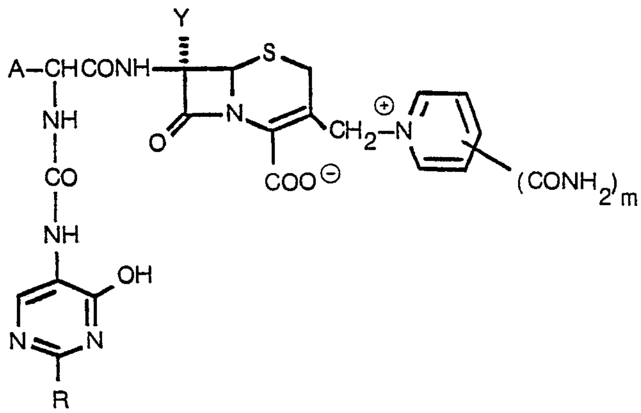

(Ia)

dans laquelle D, Y et R sont définis comme plus haut et m représente le nombre 0 ou 1, caractérisé en ce que

a) pour la préparation de composés de formule générale I dans laquelle D possède la signification indiquée à l'exception de celle d'un groupe pyridinium ou aminocarbonylpyridinium, on fait réagir un composé de formule générale II

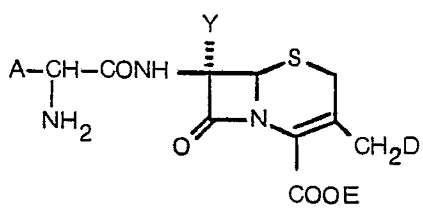

(II)

dans laquelle A, Y et E possèdent la signification indiquée plus haut et D possède la signification indiquée plus haut à l'exception de celle d'un groupe pyridinium ou aminocarbonylpyridinium, avec un dérivé de pyrimidine de formule générale III

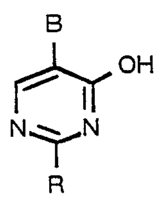

(III)

dans laquelle R est défini comme plus haut et B représente le groupe —NCO ou un dérivé réactif du groupe —NHCOOH, tel que les groupes —NHCOCl, —NHCOBr ou

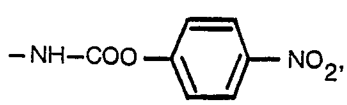

ou avec des mélanges de tels dérivés de pyrimidine de formule générale III dans laquelle B possède en partie l'une et en partie les autres des significations mentionnées plus haut, à des températures entre —20 et +50°C dans un domaine de pH entre 2,0 et 9,0 ou

b) pour la préparation de composés de formule générale I dans laquelle D possède les significations indiquées plus haut à l'exception de celle d'un groupe pyridinium ou aminocarbonylpyridinium, on amène à réaction des acides uréidocarboxyliques de formule générale IV

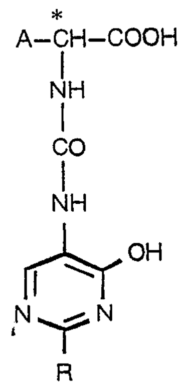

(IV)

dans laquelle A et R possèdent les significations indiquées plus haut, ou leurs sels ou dérivés réactifs avec des dérivés d'acide 7-amino-céphalosporanique de formule générale V

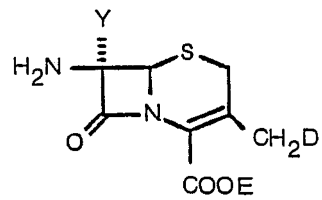

(V)

dans laquelle E et Y sont définis comme plus haut, et D possède les significations indiquées plus haut à l'exception de celle d'un groupe pyridinium ou aminocarbonylpyridinium, dans un solvant à des températures entre —40 et +40°C et ensuite, si on le désire

c) pour la préparation de composés de formule générale I dans laquelle D représente soit le groupe —SHet avec les significations indiquées plus haut pour Het ou le groupe pyridinium ou aminocarbonylpyridinium, et E remplace l'hydrogène, on fait réagir un composé de formule générale VI

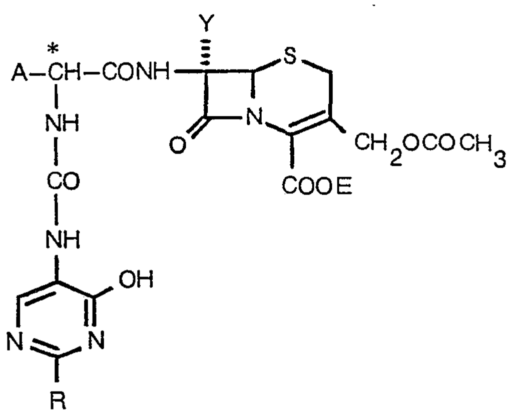

(VI)

dans laquelle A, R et Y ont les significations indiquées plus haut et E représente l'hydrogène, soit avec un composé de formule générale VII

Het—S—M (VII)

dans laquelle Het a les significations indiquées plus haut et M remplace un atome d'hydrogène ou un atome de métal alcalin ou un atome de métal alcalino-terreux soit avec la pyridine ou respectivement la 4-aminocarbonylpyridine dans un solvant à des températures entre 0 et 100°C dans un domaine de pH entre 2 et 10 pour donner un composé de formule générale I dans laquelle D possède les significations d'un groupe —SHet, pyridinium ou 4-aminocarbonylpyridinium et E celle d'un atome d'hydrogène, ou

d) pour la préparation d'un composé de formule générale I dans laquelle Y représente le groupe méthoxy, on fait réagir un composé de formule générale I dans laquelle Y représente un atome d'hydrogène, en présence de méthanol avec un méthylate de métal alcalin de formule générale $M^{\oplus}OCH_3^{\ominus}$, dans laquelle $M^{\oplus}$ représente un ion de métal alcalin, puis avec un agent d'halogénation dans un solvant inerte à des températures entre —120 et —10°C, éventuellement on transforme ensuite un composé ainsi obtenu de formules générales I ou respectivement I' dans lesquelles E représente un groupe protecteur facilement clivable in vitro ou in vivo, en l'acide carboxylique libre de formules générales I ou respectivement I' dans lesquelles E est un atome d'hydrogène et/ou on transforme un composé de formules générales I ou respectivement I' dans lesquelles E représente un

atome d'hydrogène en leurs esters ou, au moyen de bases minérales ou organiques, en les sels correspondants.

2. Procédé selon la revendication 1a, caractérisé en ce que pour la préparation de composés de formules générales I ou respectivement I' dans lesquelles E représente un atome d'hydrogène, les produits de départ de formule générale II sont mis en oeuvre sous forme de leurs sels minéraux ou organiques et la réaction avec des dérivés de pyrimidine de formule générale II est effectuée soit dans l'eau soit dans des mélanges d'eau avec des solvants organiques miscibles à l'eau en présence de bases ou dans un mélange d'eau et de solvants non miscibles à l'eau dans un domaine de pH entre 6,5 et 8,0.

3. Procédé selon la revendication 1a, caractérisé en ce que l'on fait réagir des dérivés silylés et des dérivés avec des groupes protecteurs facilement clivables des composés de formule générale II avec des pyrimidines ou respectivement leurs dérivés de formule générale III dans des solvants anhydres et ne contenant pas de groupes hydroxyle ou respectivement aprotiques, éventuellement en présence de bases.

4. Procédé selon la revendication 1b, caractérisé en ce que l'on utilise comme dérivés réactifs des acides uréidocarboxyliques de formule générale IV leurs anhydrides d'acides, leurs esters réactifs, leurs amides réactifs, leurs halogénures d'acides ou azides d'acides et comme dérivés d'acide 7-aminocéphalosporinique de formule générale V des composés dans lesquels E possède les significations mentionnées au début, à l'exception de celle d'un hydrogène, et en ce que la réaction a lieu dans un solvant, éventuellement en présence d'une base et/ou d'un agent de condensation.

5. Procédé selon la revendication 1c, caractérisé en ce que la réaction est effectuée dans un solvant fortement polaire à une valeur de pH de 4 à 8.

6. Procédé selon la revendication 1d, caractérisé en ce qu'on composé de formules générales I, I' ou Ia dans lesquelles Y représente un atome d'hydrogène, est dissous ou mis en suspension dans un agent inerte, mis ensemble avec 2 à 6 équivalents d'un méthylate de métal alcalin dans du méthanol en excès puis réagit avec un agent d'halogénation à des températures entre —100 et —50°C et la réaction est arrêtée par addition d'acide lorsque l'halogénation a eu lieu.

7. Procédé selon la revendication 1, caractérisé en ce que l'on fait réagir un sel alcalin de l'acide céphalosporanique de formules générales I ou respectivement I' avec un halogénure de pivaloyloxyméthyle ou un acétate d'halogénométhyle, propionate d'halogénométhyle ou acétate d'halogénoéthyle pour donner ses esters correspondants.

8. Procédé selon la revendication 1 pour la préparation d'un ester d'indanyle ou de phtalidyle de formules générales I ou respectivement I', caractérisé en ce que l'on fait réagir un acide de formules générales I ou respectivement I' dans lesquelles E représente un atome d'hydrogène, en présence d'un agent de condensation avec du 5-indanol dans un solvant inerte à 20 à 35°C ou un sel d'un acide de formules générales I ou respectivement I', avec le bromophtalide dans un solvant organique polaire.